# EUROPEAN PATENT SPECIFICATION

(11) **EP 1 660 491 B1**
(45) Date of publication and mention of the grant of the patent: **06.08.2008**
(21) Application number: 04779219.7
(22) Date of filing: 27.07.2004
(51) Int. Cl.: C07D 417/12, C07D 417/14, C07D 277/68, C07D 263/58, C07D 471/10, C07D 491/10, C07D 235/26, C07D 401/12, A61K 31/423, A61K 31/428, A61K 31/4184, A61K 31/4535, A61P 29/00

(54) **BENZIMIDAZOLE, BENZTHIAZOLE AND BENZOXAZOLE DERIVATIVES AND THEIR USE AS LTA4H MODULATORS**
BENZIMIDAZOL-, BENZOTHIAZOL- UND BENZOXAZOLDERIVATE UND DEREN VERWENDUNG ALS LTA4H-MODULATOREN
DERIVES DE BENZIMIDAZOLE, DE BENZTHIAZOLE ET DE BENZOXAZOLE ET UTILISATION DE CEUX-CI COMME MODULATEURS LTA4H

(30) Priority: 28.07.2003 US 490710 P
(43) Date of publication of application: 31.05.2006
(73) Proprietor: Janssen Pharmaceutica NV, 2340 Beerse (BE)
(72) Inventor: AXE, Frank, U., Escondido, CA 92027 (US); BEMBENEK, Scott, D., San Diego, CA 92130 (US); BUTLER, Christopher, R., Urbana, IL 61801 (US); EDWARDS, James, P., San Diego, CA 92129 (US); FOURIE, Anne, M., San Diego, CA 92130 (US); GRICE, Cheryl, A., Carlsbad, CA 92009 (US); SAVALL, Brad, M., San Diego, CA 92123 (US); TAYS, Kevin, L., Cardiff-By-The-Sea, CA 92007 (US); WEI, Jianmei, San Diego, CA 92129 (US)
(74) Representative: Fisher, Adrian John
(86) International application number: PCT/US2004/024050
(87) International publication number: WO 2005/012296

(56) References cited:
- EP-A- 0 268 148
- EP-A- 1 221 441
- WO-A-98/40364
- US-A- 4 873 346
- PENNING THOMAS D ET AL: "Structure-Activity Relationship Studies on 1-[2-(4- Phenylphenoxy)ethyl]pyrrolidine (SC-22716), a Potent Inhibitor of Leukotriene A4 (LTA4) Hydrolase" JOURNAL OF MEDICINAL CHEMISTRY, AMERICAN CHEMICAL SOCIETY. WASHINGTON, US, vol. 43, 2000, pages 721-725, XP002197495 ISSN: 0022-2623
- PALMER P J ET AL: "ANTIMICROBIALS. 2. SUBSTITUTED BENZOTHIAZOLYLBENZYLAMINES AND RELATED COMPOUNDS" JOURNAL OF MEDICINAL CHEMISTRY, AMERICAN CHEMICAL SOCIETY. WASHINGTON, US, vol. 14, no. 12, 1971, pages 1226-1227, XP000877145 ISSN: 0022-2623

## Description

### Field of the Invention

This invention relates to leukotriene A4 hydrolase (LTA4H) inhibitors for the treatment of inflammation. More particularly, this invention relates to certain benzooxazol-2-yl, benzothiazol-2-yl and 1*H*-benzoimidazol-2-yl compounds useful as selective inhibitors of the LTA4H enzyme for the manufacture of a medicament for the treatment of inflammatory conditions.

### Background of the Invention

Inflammation is normally an acute response by the immune system to invasion by microbial pathogens, chemicals or physical injury. In some cases, however, the inflammatory response can progress to a chronic state, and be the cause of inflammatory disease. Therapeutic control of this chronic inflammation in diverse diseases is a major medical need.

Leukotrienes (LT) are biologically active metabolites of arachidonic acid (B. Samuelsson, Science 1983, 220(4597):568-575) that have been implicated in inflammatory diseases, including asthma (D.A. Munafo et al., J. Clin. Invest. 1994, 93(3):1042-1050), inflammatory bowel disease (IBD) (P. Sharon and W.F. Stenson, Gastroenterology 1984, 86(3):453-460), chronic obstructive pulmonary disease (COPD) (P.J. Barnes, Respiration 2001, 68(5):441-448), arthritis (R.J. Griffiths et al., Proc. Natl. Acad. Sci. U.S.A. 1995, 92(2):517-521; F. Tsuji et al., Life Sci. 1998, 64(3):L51-L56), psoriasis (K. lkai, J. Dermatol. Sci. 1999, 21(3):135-146; Y.I. Zhu and M.J. Stiller, Skin Pharmacol. Appl. Skin Physiol. 2000, 13(5):235-245) and atherosclerosis (Friedrich, E. B. et al. Arterioscler Thromb Vasc Biol 23, 1761-7 (2003); Subbarao, K. et al. Arterioscler Thromb Vasc Biol 24, 369-75 (2004); Helgadottir, A. et al. Nat Genet 36, 233-9 (2004); Jala, V.R. et al Trends in lmmun. 25, 315-322 (2004)). The synthesis of leukotrienes is initiated by the conversion of arachidonic acid to an unstable epoxide intermediate, leukotriene A4 (LTA4), by 5-lipoxygenase (5-LO) (A.W. Ford-Hutchinson et al., Annu. Rev. Biochem. 1994, 63:383-347).

This enzyme is expressed predominantly by cells of myeloid origin, particularly neutrophils, eosinophils, monocytes/macrophages and mast cells (G.K. Reid et al., J. Biol. Chem. 1990, 265(32):19818-19823). LTA4 can either be conjugated with glutathione by leukotriene C4 (LTC4) synthase to produce the cysteinyl leukotriene, LTC4, or hydrolyzed to the diol, leukotriene B4 (LTB4) (B. Samuelsson, Science 1983, 220(4597):568-575). LTC4 and its metabolites, LTD4 and LTE4, induce smooth muscle contraction, broncho-constriction and vascular permeability, while LTB4 is a potent chemo-attractant and activator of neutrophils.

The stereospecific hydrolysis of LTA4 to LTB4 is catalyzed by leukotriene A4 hydrolase (LTA4H), a zinc-containing, cytosolic enzyme. This enzyme is ubiquitously expressed, with high levels in small intestinal epithelial cells, lung, and aorta (B. Samuelsson and C.D. Funk, J. Biol. Chem. 1989, 264(33):19469-19472). Moderate expression of LTA4H is observed in leukocytes, particularly neutrophils (T. Yokomizo et al., J. Lipid Mediators Cell Signaling 1995, 12(2,3):321-332).

Leukotriene B4 is a key pro-inflammatory mediator, able to recruit inflammatory cells, such as neutrophils and eosinophils, as well as activate neutrophils (F.A. Fitzpatrick et al., Ann. N. Y. Acad. Sci. 1994, 714:64-74; S.W. Crooks and R.A. Stockley, lnt. J. Biochem. Cell Biol. 1998, 30(2):173-178; A. Klein et al., J. Immunol. 2000, 164:4271-4276). LTB4 mediates its pro-inflammatory effects by binding to G protein-coupled receptors, leukotriene B4 receptor 1 (BLT1) and leukotriene B4 receptor 2 (BLT2) (T. Yokomizo et al., Arch. Biochem. Biophys. 2001, 385(2):231-241). The receptor first identified, BLT1, binds LTB₄ with high affinity, leading to intracellular signaling and chemotaxis. BLT1 is expressed mainly in peripheral leukocytes, particularly neutrophils, eosinophils, macrophages (Huang, W. W. et al. J Exp Med 188, 1063-74 (1998)) and monocytes (Yokomizo, T., Izumi, T. & Shimizu, T. Life Sci 68, 2207-12 (2001)). The murine receptor is also expressed on effector T cells and was recently shown to mediate LTB₄-dependent migration of effector CD8⁺ T cells (Goodarzi, K., Goodarzi, M., Tager, A. M., Luster, A. D. & von Andrian, U. H. Nat Immunol 4, 965-73 (2003).Ott, V. L., Cambier, J. C., Kappler, J., Marrack, P. & Swanson, B. J. Nat Immunol 4, 974-81 (2003)), early effector CD4⁺ T helper type 1 (T_{H}1 ) and T_{H}2 chemotaxis and adhesion to endothelial cells, as well as early effector CD4⁺ and CD8⁺ T cell recruitment in an asthma animal model (Tager, A. M. et al.. Nat Immunol 4, 982-90 (2003)).LTB4 receptor BLT2 (S. Wang et al., J. Biol. Chem. 2000, 275(52):40686-40694; T. Yokomizo et al., J. Exp. Med. 2000, 192(3):421-431) shares 42% amino acid homology with BLT1, but is more broadly expressed, including in peripheral tissues such as the spleen, ovary and liver, as well as in leukocytes. BLT2 binds LTB4 with lower affinity than BLT1 does, mediates chemotaxis at higher concentrations of LTB4, and differs from BLT1 in its affinity for certain antagonists. While LTB4 receptor antagonists may differ in their affinity for BLT1 versus BLT2, blocking the production of LTB4 using LTA4H inhibitors would be expected to inhibit the downstream events mediated through both BLT1 and BLT2.

Studies have shown that introduction of exogenous LTB4 into normal tissues can induce inflammatory symptoms (R.D.R. Camp et al., Br. J. Pharmacol. 1983, 80(3):497-502; R. Camp et al., J. Invest. Dermatol. 1984, 82(2):202-204). Elevated levels of LTB4 have been observed in a number of inflammatory diseases including IBD, COPD, psoriasis, rheumatoid arthritis (RA), cystic fibrosis and asthma (S.W. Crooks and R.A. Stockley, Int. J. Biochem. Cell Biol. 1998, 30(2):173-178). Therefore, reduction of LTB4 production by an inhibitor of LTA4H activity would be predicted to have therapeutic potential in a wide range of diseases.

This idea is supported by a study of LTA4H-deficient mice that, while otherwise healthy, exhibited markedly decreased neutrophil influx in arachidonic acid-induced ear inflammation and zymosan-induced peritonitis models (R.S. Byrum et al., J. Immunol. 1999, 163(12): 6810-6819). LTA4H inhibitors have been shown to be effective anti-inflammatory agents in pre-clinical studies. For example, oral administration of LTA4H inhibitor SC57461 caused inhibition of ionophore-induced LTB4 production in mouse blood ex vivo, and in rat peritoneum in vivo (J.K. Kachur et al., J. Pharm. Exp. Ther. 2002, 300(2), 583-587). Eight weeks of treatment with the same inhibitor compound significantly improved colitis symptoms in cotton top tamarins (T.D. Penning, Curr. Pharm. Des. 2001, 7(3):163-179). The spontaneous colitis that develops in these animals is very similar to human IBD. The results therefore indicate that LTA4H inhibitors would have therapeutic utility in this and other human inflammatory diseases.

Events that elicit the inflammatory response include the formation of the pro-inflammatory mediator leukotriene B4. Hydrolase LTA4H catalyzes the formation of this mediator, and LTA4H inhibitors block the production of the pro-inflammatory mediator LTB4, thus providing the ability to prevent and/or treat leukotriene-mediated conditions, such as inflammation. The inflammatory response is characterized by pain, increased temperature, redness, swelling, or reduced function, or by a combination of two or more of these symptoms. Regarding the onset and evolution of inflammation, inflammatory diseases or inflammation-mediated diseases or conditions include, but are not limited to, acute inflammation, allergic inflammation, and chronic inflammation.

Examples of textbooks on the subject of inflammation include J. I. Gallin and R. Snyderman, Inflammation: Basic Principles and Clinical Correlates, 3rd Edition, (Lippincott Williams & Wilkins, Philadelphia, 1999); V. Stvrtinova, J. Jakubovsky and I. Hulin, "Inflammation and Fever", Pathophysiology Principles of Diseases (Textbook for Medical Students, Academic Press, 1995); Cecil et al., Textbook Of Medicine, 18th Edition (W.B. Saunders Company, 1988); and Steadmans Medical Dictionary.

Background and review material on inflammation and conditions related with inflammation can be found in articles such as the following: C. Nathan, Points of control in inflammation, Nature 2002, 420:846-852; K.J. Tracey, The inflammatory reflex, Nature 2002, 420:853-859; L.M. Coussens and Z. Werb, Inflammation and cancer, Nature 2002, 420:860-867; P. Libby, Inflammation in atherosclerosis, Nature 2002, 420:868-874; C. Benoist and D. Mathis, Mast cells in autoimmune disease, Nature 2002, 420:875-878; H.L. Weiner and D.J. Selkoe, Inflammation and therapeutic vaccination in CNS diseases, Nature 2002, 420:879-884; J. Cohen, The immunopathogenesis of sepsis, Nature 2002, 420:885-891; D. Steinberg, Atherogenesis in perspective: Hypercholesterolemia and inflammation as partners in crime, Nature Medicine 2002, 8(11):1211-1217. Cited references are incorporated herein by reference.

Inflammation is due to any one of a plurality of conditions, such as asthma, chronic obstructed pulmonary disease (COPD), atherosclerosis, rheumatoid arthritis, multiple sclerosis, inflammatory bowel diseases (including Crohn's disease and ulcerative colitis), or psoriasis, which are each characterized by excessive or prolonged inflammation at some stage of the disease.

Applicants have discovered benzooxazol-2-yl, benzothiazol-2-yl and 1*H-*benzoimidazol-2-yl compounds and derivatives thereof; their use as inhibitors of enzymes, such as the LTA4H enzyme in the formation of pro-inflammatory mediators, such as the LTB4 mediator; also their use for the manufacture of a medicament for the treatment of inflammatory conditions; and the preparation of pharmaceutical compositions for the treatment of inflammation.

### Summary of the Invention

There are provided by the present invention LTA4H enzyme inhibitors, which have the following general formula (I): 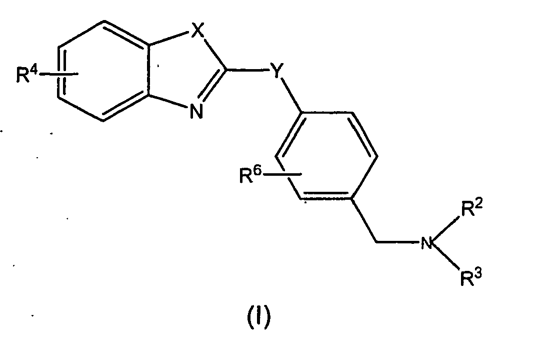 or an enantiomer, diasteromer, racemic, tautomer, hydrate, solvate, or a pharmaceutically acceptable salt, ester, or amide thereof,
wherein
X is selected from the group consisting of NR⁵, O, and S, with R⁵ being one of H and CH₃;
Y is selected from the group consisting of CH₂, and O;
R⁴ is selected from the group consisting of H, OCH₃, Cl, F, Br, I, OH, NH₂, CN, CF₃ and CH₃;
R⁶ is H or F; and
R² and R³ are each independently selected from the group consisting of
A) H, C₁₋₇alkyl, C₃₋₇alkenyl, wherein the carbon in said alkenyl that is attached to the nitrogen member has only single bonds, C₃₋₇alkynyl, wherein the carbon in said alkynyl that is/attached to the nitrogen member has only single bonds, C₃₋₇cycloalkyl optionally benzofused, C₅₋₇cycloalkenyl, -C₃₋₇cycloalkylC₁₋₇alkyl,-C₁₋₇alkylC₃₋₇cycloalkyl and phenyl, wherein each of the substituents A) is independently substituted with 0, 1, or 2 R^{Q}, and each of said R^{Q} is a substituent at a carbon member that is at least one carbon member removed from the nitrogen member;
B) a substituent HetR^{a};
C) -C₁₋₇alkylC(O)R^{x}, optionally substituted with CH₂R^{Ar} or CH₂R^{Ar'};
D) -C₂₋₅alkylC(O)R^{x}, wherein two valence allowed carbon members in the C₂₋₅alkyl of said -C₂₋₅alkylC(O)R^{x} are part of a saturated C₃₋₆carbocycle;
E) -C₂₋₅alkylOH wherein two valence allowed carbon members in the C₂₋₅alkyl of said -C₂₋₅alkylOH are part of a saturated C₃₋₆carbocycle;
F) -C₀₋₄alkylphenyl, wherein the phenyl in said -C₀₋₄alkylphenyl is fused at two adjacent carbon members in said phenyl to R^{f}, or is benzofused;
G) -C₀₋₄alkylAr⁶, where Ar⁶ is a 6-membered heteroaryl having a carbon member point of attachment and having one or two -N= heteroatom members, and benzofused;
H) -C₀₋₄alkylAr⁵, where Ar⁵ is a 5-membered heteroaryl, having one heteroatom member selected from the group consisting of O, S, and >NR^{Y}, and having 0 or 1 -N= additional heteroatom member, optionally containing two carbonyl groups, and optionally benzofused;
I) -C₁₋₄alkylAr^{5'}, where Ar^{5'} is a 5-membered heteroaryl containing 3 or 4 nitrogen members, optionally substituted with R^{Y}, and having a valence allowed site as a point of attachment;
J) -C₀₋₄alkylAr⁶⁻⁶, where Ar⁶⁻⁶ is a C₀₋₄alkyl-attached phenyl fused at valence allowed sites to a 6-membered heteroaryl, wherein said 6-membered heteroaryl has one or two -N= heteroatom members;
K) -C₀₋₄alkylAr⁶⁻⁵, where Ar⁶⁻⁵ is a C₀₋₄alkyl-attached phenyl fused at valence allowed sites to a 5-membered heteroaryl, said 5-membered heteroaryl having one heteroatom member selected from the group consisting of O, S, and >NR^{Y}, and said 5-membered heteroaryl having 0 or 1 additional heteroatom member which is -N=;
L) one of 2-(4-ethyl-phenoxy)-benzothiazole, 2-(4-ethyl-phenoxy)-benzooxazole, and 2-(4-ethyl-phenoxy)-1*H*-benzoimidazole; and
M) SO₂C₁₋₄alkyl;
   alternatively R² and R³ are taken together with the nitrogen to which they are attached to form a heterocyclic ring that contains at least one heteroatom member that is said attachment nitrogen, said heterocyclic ring being selected from the group consisting of
   i) a 4-7 membered heterocyclic ring HetR^{b}, said 4-7 membered heterocyclic ring HetR^{b} having one heteroatom member that is said attachment nitrogen, and being substituted with 0, 1, or 2 substituents at the same or at different substitution members, said substituents being selected from the group consisting of -R^{Y}, -CN, -C(O)R^{Y}, -C₀₋₄alkylCO₂R^{Y}, -C₀₋₄alkylC(O)CO₂R^{Y}, -C₀₋₄ alkylOR^{Y}, -C₀₋₄alkylC(O)NR^{Y}R^{Z}, -C₀₋₄alkylNR^{Y}C(O)R^{Z}, -C(O)NR^{Z}OR^{Y}, -C₀₋₄ alkylNR^{Y}C(O)CH₂OR^{Y}, -C₀₋₄alkylNR^{Y}C(O)CH₂C(O)R^{Y}, -C₀₋₄alkylNR^{Y}CO₂R^{Y}, - C₀₋₄alkylNR^{Y}C(O)NR^{Y}R^{Z}, -C₀₋₄alkylNR^{Y}C(S)NR^{Y}R^{Z}, -NR^{Y}C(O)CO₂R^{Y},-NR^{Y}R^{Z}, - C₀₋₄alkylNR^{W}SO₂R^{Y},1,3-dihydro-indol-2-one-1-yl, 1,3-dihydro-benzoimidazol-2-one-1-yl, tetrazol-5-yl, 1-R^{Y}-1*H*-tetrazol-5-yl, R^{Y}-triazolyl, 2-R^{Y}-2*H*-tetrazol-5-yl, pyrrolidine-2-thion-1-yl, piperidine-2-thion-1-yl, -C₀₋₄alkylC(O)N(R^{Y})(SO₂R^{Y}), -C₀₋₄alkylN(R^{Y})(SO₂)NR^{Y}R^{Y}, -C₀₋₄alkylN(R^{Y})(SO₂)NR^{Y}CO₂R^{Y}, halo, 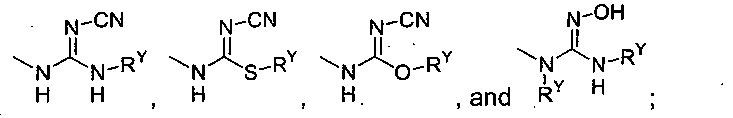
   ii) a 5-7 membered heterocyclic ring HetR^{c}, said 5-7 heterocyclic ring HetR^{c} having one additional heteroatom member separated from said attachment nitrogen by at least one carbon members, said additional heteroatom member being selected from the group consisting of O, S(=O)₀₋₂, and >NR^{M}, said 5-7 membered heterocyclic ring HetR^{c} having 0 or 1 carbonyl members, and being substituted with 0, 1, or 2 substituents at the same or at different carbon substitution members, said substituents being selected from the group consisting of -C(O)R^{Y}, -CO₂R^{Y} -C₃₋₄alkylCO₂R^{Y} and R^{Z};
   iii) one of imidazolidin-1-yl, 2-imidazolin-1-yl, pyrazol-1-yl, imidazol-1-yl, 2*H-*tetrazol-2-yl, 1*H*-tetrazol-1-yl, pyrrol-1-yl, 2-pyrrolin-1-yl, and 3-pyrrolin-1-yl, wherein each of said 2*H*-tetrazol-2-yl and 1*H*-tetrazol-1-yl is substituted at the carbon member with 0 or 1 of -C₀₋₄alkylR^{z}, -C₀₋₄alkylSR^{Y}, -C₀₋₄alkylCO₂R^{Y}, and substituent HetR^{a}; and
   iv) one of 1,2,3,4-tetrahydro-quinolin-1-yl, 1,2,3,4-tetrahydro-isoquinolin-2-yl, indol-1-yl, isoindol-2-yl, indolin-1-yl, benzimidazol-1-yl, 2,8-diaza-spiro[4.5]decan-1-one-8-yl, 4-{[(2-tert-butoxycarbonylamino-cyclobutanecarbonyl)-amino]-methyl}-piperidin-1-yl, 4-{[(2-amino-cyclobutanecarbonyl)-amino]-methyl}-piperidin-1-yl, 3,9-diaza-spiro[5.5]undecane-3-carboxylic acid-9-yl tert-butyl ester, 4-oxo-1-phenyl-1,3,8-triaza-spiro[4.5]dec-8-yl, and 4-oxo-1,3,8-triaza-spiro[4.5]dec-8-yl;
wherein
substituent HetR^{a} is a 4-7 membered heterocyclic ring having a carbon member point of attachment and containing a member >NR^{M} as a heteroatom member, and said heteroatom member being separated from said carbon member point of attachment by at least 1 additional carbon member;
R^{K} is selected from the group consisting of H, -C₁₋₄alkyl, -C₀₋₄alkylR^{Ar} each optionally substituted with 1, 2, or 3 substituents R^{N} R^{L} is selected from the group consisting of -CO₂R^{S} and -C(O)NR^{S}R^{S'}; R^{M} is selected from the group consisting of R^{z}, indol-7-yl, -SO₂R^{Y}, -C₃₋₄alkylCO₂R^{Y}, -CO₂R^{Y}, -C(O)NR^{Z}OR^{Y}, -C(O)R^{Y}, -C(O)C₁₋₄alkylOR^{Y}, - C₀₋₄alkylC(O)NR^{S}R^{S'}, C₀₋₄alkylC(O)CO₂R^{Y}, 1,3-dihydro-indol-2-one-1-yl, 1,3-dihydro-benzoimidazol-2-one-1-yl, tetrazol-5-yl, 1-R^{Y}-1*H*-tetrazol-5-yl, R^{Y}-triazolyl, 2-R^{Y}-2*H*-tetrazol-5-yl and -C₀₋₄alkylC(O)N(R^{Y})(SO₂R^{Y}), each optionally substituted with 1, 2 or 3 substituents R^{N};
R^{N} is selected from the group consisting of OCH₃, Cl, F, Br, I, OH, NH₂, CN, CF₃, CH₃, OC(O)CH₃, and NO₂;
R^{P} is selected from the group consisting of R^{Y}, -C₂₋₄alkylOR^{Y}, R^{Ar}, -C₁₋₂alkylCO₂R^{Y}, -C₁₋₂alkylCONR^{S}R^{S'}, indol-7-yl, and -SO₂C₁₋₄alkyl; R^{Q} is selected from the group consisting of fluoro, chloro, bromo, iodo, trifluoromethyl, trichloromethyl, -CN, -C₁₋₄alkyl, -C₀₋₄alkylR^{Ar}, -C₀₋₄alkylR^{Ar'}, -C₀₋₄alkylOR^{Y}, -C₀₋₄alkylCO₂R^{Y}, -C₀₋₄alkylNR^{Y}R^{Z}, -C₀₋₄alkylNR^{Y}COR^{Y}, -C₀₋₄alkylNR^{Y}CONR^{Y}R^{Z}, -C₀₋₄alkylNR^{Y}SO₂R^{Y}, and -C₀₋₄alkylSR^{Y}; R^{S} and R^{S'} are independently selected from the group consisting of H, -C₁₋₄alkyl, and -C₀₋₄alkylphenyl; alternatively, R^{S} and R^{S'} are taken together with the nitrogen member to which said R^{S} and R^{S'} are attached to form a 4-7 membered heterocyclic ring having 0 or 1 additional heteroatom member selected from the group consisting of O, S, and >NR^{Y}, provided that said additional heteroatom member is separated by at least two carbon members from said nitrogen member to which said R^{S} and R^{S'} are attached, and provided that where R^{Y} is C₀₋₄alkylR^{Ar}, then R^{Ar} is not substituted with R^{L}; R^{W} is selected from the group consisting of R^{Y}, and -C₃₋₇cycloalkyl;
R^{X} is selected from the group consisting of -OR^{Y}, -NR^{Y}R^{Z}, -C₁₋₄alkyl, and -C₀₋₄alkylR^{Ar};
R^{Y} is selected from the group consisting of H, -C₁₋₄alkyl, -C₀₋₄alkylR^{Ar} and -C₀₋₄alkylR^{Ar'}, each optionally substituted with 1, 2, or 3 substituents R^{N};
R^{Z} is selected from the group consisting of R^{Y}, -C₂₋₄alkylOR^{Y}, -C₁₋₂alkylCO₂R^{Y},-C₁₋₂alkylC(O)NR^{S}R^{S'}, and -C₂₋₄alkylNR^{S}R^{S'};
when R^{Y} and R^{Z} are attached to a nitrogen member, R^{Y} and R^{Z} are selected as defined above, or R^{Y} and R^{Z} are taken together with the R^{Y}- and R^{Z}- attached nitrogen member to form a 4-7 membered heterocyclic ring HetR^{d} having 0 or 1 additional heteroatom members selected from the group consisting of O, S, and >NR^{M}, said 4-7 membered heterocyclic ring HetR^{d} having 0 or 1 carbonyl members, and said 4-7 membered heterocyclic ring HetR^{d} having 0 or 1 valence allowed carbon members substituted with at least one of R^{M}, -CO₂H, and -C₀₋₁alkylOR^{Y};
R^{Ar} is a moiety with a carbon member attachment point and said moiety is selected from the group consisting of phenyl, pyridyl, pyrimidyl, and pyrazinyl, wherein each valence allowed carbon member in each of said moieties is independently substituted with at least one of 0, 1, 2 or 3 R^{N}, and 0 or 1 R^{L};
R^{Ar'} is a 3-8 membered ring, having 0, 1 or 2 heteroatom members selected from the group consisting of O, S, N, and >NR^{Y}, having 0, 1, or 2 unsaturated bonds, having 0 or 1 carbonyl members, wherein each valence allowed member in each of said rings is independently substituted with 0, 1, or 2 R^{K}; and
R^{f} is a linear 3- to 5-membered hydrocarbon moiety having 0 or 1 unsaturated carbon-carbon bonds and having 0 or 1 carbonyl members.

Embodiments of the present invention comprise new compounds that are LTA4H enzyme inhibitors and have the general formula (II): 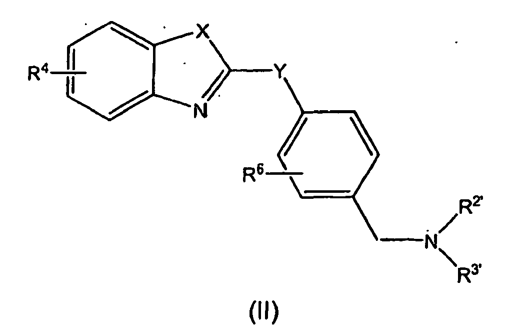 or an enantiomer, diasteromer, racemic, tautomer, hydrate, solvate, or a pharmaceutically acceptable salt, ester, or amide thereof,
wherein
R⁴, R⁶, X and Y are defined as in compound of formula (I), R^{2'} is defined as R² in compound of formula (I), and R^{3'} is defined as R³ in compound of formula (I), provided that
(a) said R^{2'} and R^{3'} further satisfy the following conditions:
   (e1): said R^{2'} and R^{3'} are not both H, when Y is O, and X is S;
   (e2): when Y is CH₂, X is N, and said R^{2'} and R^{3'} are parts of a primary or secondary amino group, then said R^{2'} and R^{3'} are not selected from the group consisting of H and methyl, (for example, the selection R^{2'} being H and R^{3'} being methyl is hereby disallowed; the selection R^{2'} being methyl and R^{3'} being H is hereby disallowed; the selection R^{2'} being H and R^{3'} being H is hereby disallowed; but the selection R^{2'} being methyl and R^{3'} being methyl is hereby allowed);
   (e3): said R^{2'} and R^{3'} taken together with the nitrogen member to which they are attached do not form a piperazine group, when X is O, and Y is one of O and CH₂;
   (e4): said R^{2'} and R^{3'} taken together with the nitrogen member to which they are attached do not form a piperidine group that is monosubstituted with a saturated 6-membered cyclic group, when X is O, and Y is one of O and CH₂; and
   (e5): said R^{2'} and R^{3'} taken together with the nitrogen member to which they are attached do not form either a substituted piperidine group or a substituted piperazine group, wherein said substituted piperidine group or said substituted piperazine group is substituted in the 4-position with a substituent XG, said XG having the structure 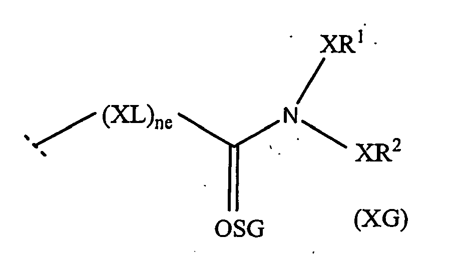
   wherein n = 0, 1, and when ne = 1 then XL is a C₁₋₆alkyl, OSG is O or S, and XR¹ and XR² taken together with the nitrogen member to which they are attached form one of a piperidine group, a piperazine group, a morpholine group, a thiomorpholine group, and a pyrrolidine group, or each of XR¹ and XR² taken independently are one of H, C₁₋₆alkyl, aryl, aralkyl, C₃₋₈cycloalkyl, C₃₋₈cycloalkyl-C₁₋₆alkyl, heteroalkyl, heteroaryl-C₁₋₆alkyl, heterocycloalkyl and heterocycloalkyl-C₁₋₆alkyl; wherein the aryl , aralkyl, cycloalkyl, heteroaryl or heterocycloalkyl may be optionally substituted with one or more substituents independently selected from halogen, hydroxy, C₁₋₆alkyl, C₁₋₆alkoxy, halogenatedC₁₋₆alkyl, halogenatedC₁₋₆alkoxy, nitro, cyano, amino, C₁₋₄alkylamino, di(C₁₋₄alkyl)amino, heteroaryl or heterocycloalkyl; and
(b) further provided that when X is S, and Y is O, then one of R^{2'} and R^{3'} is not XCG when the other is C₁₋₆alkyl, wherein XCG is the group 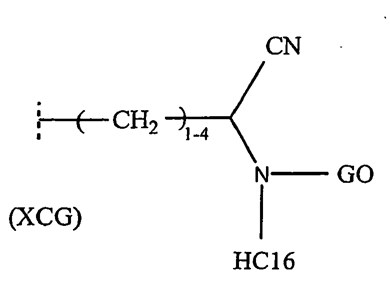
wherein HC16 is one of H, C₁₋₆alkyl, haloC₁₋₆alkyl, allyl, and C₁₋₆alkoxymethyl, and GO is a group attached by a carbon member that has a =O substituent forming an amido group (>N-C(O)-) with the nitrogen member to wich said GO group is attached.

Isomeric forms of the compounds of formulae (I) and (II), and of their pharmaceutically acceptable salts, amides and esters, are encompassed within the present invention, and reference herein to one of such isomeric forms is meant to refer to at least one of such isomeric forms. One of ordinary skill in the art will recognize that compounds according to this invention may exist, for example in a single isomeric form whereas other compounds may exist in the form of a regioisomeric mixture.

Whether stated explicitly or not in any part of the written description and claims, it is understood that each substituent and member assignment in the context of this invention is made independently of any other member and substituent assignment, unless stated otherwise. By way of a first example on substituent terminology, if substituent S¹ₑₓₐₘₚₗₑ is one of S₁ and S₂, and substituent S²ₑₓₐₘₚₗₑ is one of S₃ and S₄, then these assignments refer to embodiments of this invention given according to the choices S¹ₑₓₐₘₚₗₑ is S₁ and

S²ₑₓₐₘₚₗₑ is S₃; S¹ₑₓₐₘₚₗₑ is S₁ and S²ₑₓₐₘₚₗₑ is S₄; S¹ₑₓₐₘₚₗₑ is S₂ and S²ₑₓₐₘₚₗₑ is S₃; S¹ₑₓₐₘₚₗₑ is S₂ and S²ₑₓₐₘₚₗₑ is S₄; and equivalents of each one of such choices. The shorter terminology "S¹ₑₓₐₘₚₗₑ is one of S₁ and S₂, and S²ₑₓₐₘₚₗₑ is one of S₃ and S₄" is accordingly used herein for the sake of brevity, but not by way of limitation. The foregoing first example on substituent terminology, which is stated in generic terms, is meant to illustrate the various substituent R assignments described herein. The foregoing convention given herein for substituents extends, when applicable, to members such as X, Y, Z, and W, and the index n.

Furthermore, when more than one assignment is given for any member or substituent, embodiments of this invention comprise the various groupings that can be made from the listed assignments, taken independently, and equivalents thereof. By way of a second example on substituent terminology, if it is herein described that substituent Sₑₓₐₘₚₗₑ is one of S₁, S₂, and S₃, this listing refers to embodiments of this invention for which Sₑₓₐₘₚₗₑ is S₁; Sₑₓₐₘₚₗₑ is S₂; Sₑₓₐₘₚₗₑ is S₃; Sₑₓₐₘₚₗₑ is one of S₁ and S₂; Sₑₓₐₘₚₗₑ is one of S₁ and S₃; Sₑₓₐₘₚₗₑ is one of S₂ and S₃; Sₑₓₐₘₚₗₑ is one of S₁, S₂ and S₃; and Sₑₓₐₘₚₗₑ is any equivalent of each one of these choices. The shorter terminology "Sₑₓₐₘₚₗₑ is one of S₁, S₂, and S₃" is accordingly used herein for the sake of brevity, but not by way of limitation. The foregoing second example on substituent terminology, which is stated in generic terms, is meant to illustrate the various substituent R assignments described herein. The foregoing convention given herein for substituents extends, when applicable, to members such as X, Y, Z, and W, and the index n.

The nomenclature "Cᵢ₋ⱼ" with j > i, when applied herein to a class of substituents, is meant to refer to embodiments of this invention for which each and every one of the number of carbon members, from i to j including i and j, is independently realized. By way of example, the term C₁₋₃ refers independently to embodiments that have one carbon member (C₁), embodiments that have two carbon members (C₂), and embodiments that have three carbon members (C₃).

The term Cₙ₋ₘalkyl refers to an aliphatic chain, whether straight or branched, with a total number N of carbon members in the chain that satisfies n ≤N ≤m, with m > n.

When any variable referring to a substituent, compound member or index, occurs more than once, the full range of assignments is meant to apply to each occurrence, independently of the specific assignment(s) to any other occurrence of such variable.

According to the foregoing interpretive considerations on assignments and nomenclature, it is understood that explicit reference herein to a set implies, where chemically meaningful and unless indicated otherwise, independent reference to embodiments of such set, and reference to each and every one of the possible embodiments of subsets of the set referred to explicitly.

The present invention also features methods for inhibiting LTA4H enzyme activity with such compounds, and pharmaceutical compositions containing such compounds and methods of using such compositions in the manufacture of a medicament for the

treatment or prevention of conditions that are mediated by LTA4H enzyme activity.

Pharmaceutical compositions according to the present invention include at least one of the compounds of the present invention. If more than one of such compounds is included in a composition, the therapeutically effective amount may be a jointly effective amount. As such inhibitors of the LTA4H enzyme, compounds and compositions according to the present invention are useful in the prevention, inhibition, or treatment of inflammation.

The invention also features a pharmaceutical composition for treating or preventing an LTA4H-mediated condition in a subject, comprising a therapeutically effective amount of at least one LTA4H modulator selected from compounds of formulae enantiomers, diastereomers, racemates thereof, pharmaceutically acceptable salts, amides and esters thereof. In addition, the invention features a pharmaceutical composition for inhibiting inflammatory response in a subject, comprising a therapeutically effective amount of at least LTA4H inhibitor selected from compounds of formulae (II), enantiomers, diastereomers, racemates thereof, pharmaceutically: acceptable salts, amides and esters thereof. The invention additionally features an anti-inflammatory composition, comprising a therapeutically effective amount of at least one anti-inflammatory compound selected from compounds of formulae (II), enantiomers, diastereomers, racemates thereof, pharmaceutically acceptable salts, amides and esters thereof.

The invention features the use of a therapeutically effective amount of at least one anti-inflammatory compound selected from compounds of formulae (II), enantiomers, diastereomers, racemates thereof, pharmaceutically acceptable salts, amides and esters thereof in the manufacture of a medicament for treating or preventing inflammation in a subject. The invention also features the use of a therapeutically effective amount of at least one LTA4H modulator selected from compounds of formulae (II), enantiomers, diastereomers, racemates thereof, pharmaceutically acceptable salts, amides and esters thereof in the manufacture of a medicament for treating or preventing an LTA4H-mediated condition in a subject. Furthermore, the invention, features the use of a therapeutically effective amount of at least one LTA4H inhibitor selected from compounds of formulae (II), enantiomers, diastereomers, racemates thereof, pharmaceutically acceptable salts, amides and esters thereof in the manufacture of a medicament for inhibiting inflammation in a subject.

This invention features uses as described above for the treatment, prevention and/or inhibition of conditions that are associated with and/or cause inflammation, such as any one or a plurality of the followoing conditions: Asthma, chronic obstructed pulmonary disease (COPD), atherosclerosis, rheumatoid arthritis, multiple sclerosis, inflammatory bowel diseases (including Crohn's disease and ulcerative colitis), or psoriasis, which are each characterized by excessive or prolonged inflammation at some stage of the disease.

Additional features and advantages of the invention will become apparent from the detailed description below, including examples, and the appended claims.

### Detailed Description of the Invention

The present invention is directed to compounds of formula (II) as herein defined, enantiomers, diastereomers, racemates thereof, pharmaceutically acceptable salts, amides and esters thereof, pharmaceutical compositions that contain at least one of such compounds, use of such compounds for manufacture of medicament for treatment and/or prevention of conditions such as those that are mediated by LTA4H, and methods of making such pharmaceutical compositions.

The following terms are defined below, and by their usage throughout the disclosure.

"Alkyl" includes straight chain and branched hydrocarbons with at least one hydrogen removed to form a radical group. Alkyl groups include methyl, ethyl, propyl, isopropyl, butyl, isobutyl, t-butyl, 1-methylpropyl, pentyl, isopentyl, sec-pentyl, hexyl, heptyl, octyl, and so on. Alkyl does not include cycloalkyl.

"Alkenyl" includes straight chain and branched hydrocarbon radicals as above with at least one carbon-carbon double bond (sp²). Unless indicated otherwise by the prefix that indicates the number of carbon members, alkenyls include ethenyl (or vinyl), prop-1-enyl, prop-2-enyl (or allyl), isopropenyl (or 1-methylvinyl), but-1-enyl, but-2-enyl, butadienyls, pentenyls, hexa-2,4-dienyl, and so on.

"Alkynyl" includes straight chain and branched hydrocarbon radicals as above with at least one carbon-carbon triple bond (sp). Unless indicated otherwise by the prefix that indicates the number of carbon members, alkynyls include ethynyl, propynyls, butynyls, and pentynyls. Hydrocarbon radicals having a mixture of double bonds and triple bonds, such as 2-penten-4-ynyl, are grouped as alkynyls herein.

"Alkoxy" includes a straight chain or branched alkyl group with a terminal oxygen linking the alkyl group to the rest of the molecule. Alkoxy includes methoxy, ethoxy, propoxy, isopropoxy, butoxy, t-butoxy, pentoxy and so on. "Aminoalkyl", "thioalkyl", and "sulfonylalkyl" are analogous to alkoxy, replacing the terminal oxygen atom of alkoxy with, respectively, NH (or NR), S, and SO₂.

Unless indicated otherwise by the prefix that indicates the number of carbon members, "cycloalkyl" includes cyclopropyl, cyclobutyl, cyclopentyl, cyclohexyl, cycloheptyl, cyclooctyl, and so on.

Unless indicated otherwise by the prefix that indicates the number of members in the cyclic structure, "heterocyclyl", "heterocyclic" or "heterocycle" is a 3- to 8-member aromatic, saturated, or partially saturated single or fused ring system that comprises carbon atoms wherein the heteroatoms are selected from N, O, and S. Examples of heterocyclyls include thiazoylyl, furyl, pyranyl, isobenzofuranyl, pyrrolyl, imidazolyl, pyrazolyl, isothiazolyl, isoxazolyl, pyridyl, pyrazinyl, pyrimidinyl, pyridazinyl, indolizinyl, isoindolyl, indolyl, indazolyl, purinyl, quinolyl, furazanyl, pyrrolidinyl, pyrrolinyl, imidazolidinyl, imidazolinyl, pyrazolidinyl, pyrazolinyl, piperidyl, piperazinyl, indolinyl, and morpholinyl. For example, preferred heterocyclyls or heterocyclic radicals include morpholinyl, piperazinyl, pyrrolidinyl, pyridyl, cyclohexylimino, cycloheptylimino, and more preferably, piperidyl.

Substitution positions are referred to in conventional terms. For example, piperidine and piperazine group substitution positions are numberd as follows:

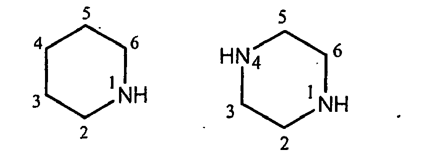

"Carbocycle" is a cycloalkyl or a partially saturated cycloalkyl that is not benzo ( 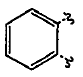 ).

"Aryl" includes phenyl, naphthyl, biphenylyl, tetrahydronaphthyl, and so on, any of which may be optionally substituted. Aryl also includes arylalkyl groups such as benzyl, phenethyl, and phenylpropyl. Aryl includes a ring system containing an optionally substituted 6-membered carbocyclic aromatic ring, said system may be bicyclic, bridge, and/or fused. The system may include rings that are aromatic, or partially or completely saturated. Examples of ring systems include indenyl, pentalenyl, 1-4-dihydronaphthyl, indanyl, benzimidazolyl, benzothiophenyl, indolyl, benzofuranyl, isoquinolinyl, and so on. Examples illustrating heteroaryl are thienyl, furanyl, pyrrolyl, imidazolyl, oxazolyl, thiazolyl, benzothienyl, benzofuranyl, benzimidazolyl, benzoxazolyl, benzothiazolyl.

"Halo" includes fluoro, chloro, bromo, and iodo, and is preferably fluoro or chloro.

The term "carbonyl" refers to a >C=O moiety, such that when this term is characterized as being part of a chain or cyclic structure, the carbon member in the carbonyl group is taken as being one of the carbon members of such chain or cyclic structure.

As in standard chemical nomenclature, the group phenyl is herein referred to as "phenyl" or as "Ph".

It is understood that substitutions and combinations of substitutions recited herein, whether stated explicitly or not, refer to substitutions that are consistent with the valency of the member being substituted. Terms such as "valence allowed site", "valence allowed member" and morphological variations thereof are used herein in this sense. For example, "valence allowed" when applied to a carbon member refers to the tetravalency of C; it refers to the trivalency of N when applied to a nitrogen member; and it refers to the four bonds of a nitrogen member that is conventionally characterized with a positive electric charge. Valence allowed options are part of the ordinary skill in the art.

"Patient" or "subject" includes mammals such as human beings and animals (e.g., dogs, cats, horses, rats, rabbits, mice, non-human primates) in need of observation, experiment, treatment or prevention in connection with the relevant disease or condition. Preferably, the patient is a human being.

"Composition" includes a product comprising the specified ingredients in the specified amounts, including in the effective amounts, as well as any product that results directly or indirectly from combinations of the specified ingredients in the specified amounts.

"Therapeutically effective amount" or "effective amount" and grammatically related terms mean that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

**Table of Acronyms**

| Term | Acronym |
|---|---|
| Tetrahydrofuran | THF |
| N,N-Dimethylformamide | DMF |
| N,N-Dimethylacetamide | DMA |
| Dimethyl sulfoxide | DMSO |
| tert-Butylcarbamoyl | BOC |
| Bovine serum albumin | BSA |
| High-pressure liquid chromatography | HPLC |
| Thin layer chromatography | TLC |

Compounds of formula (II), comprise compounds that satisfy any one of the combinations of definitions given herein and equivalents thereof.

It is understood that some compounds refered to herein are chiral and/or have geometric isomeric centers, for example E- and Z- isomers. The present invention encompasses all such optical isomers, including diasteroisomers and racemic mixtures, and geometric isomers that possess the activity that characterizes the compounds of this invention. In addition, certain compounds referred to herein can exist in solvated as well as unsolvated forms. It is understood that this invention encompasses all such solvated and unsolvated forms that possess the activity that characterizes the compounds of this invention. Compounds according to the present invention that have been modified to be detectable by some analytic technique are also within the scope of this invention. An example of such compounds is an isotopically labeled compound, such as an ¹⁸F isotopically labeled compound that may be used as a probe in detection and/or imaging techniques, such as positron emission tomography (PET) and single-photon emission computed tomography (SPECT). Another example of such compounds is an isotopically labeled compound, such as a deuterium and/or tritium labeled compound that may be used in reaction kinetic studies.

It is understood that substitutions and combinations of substitutions recited herein, whether stated explicitly or not, refer to substitutions that are consistent with the valency of the member being substituted. For example, a substitution applied to a carbon member refers to the tetravalency of C; it refers to the trivalency of N when applied to a nitrogen member; and it refers to the four bonds of a nitrogen member that is conventionally characterized with a positive electric charge. Valence allowed options are part of the ordinary skill in the art.

The "pharmaceutically acceptable salts, amides or and esters thereof" refer to those salts, amides and ester forms of the compounds of the present invention that would be apparent to the pharmaceutical chemist, *i*.*e*., those that are non-toxic and that would favorably affect the pharmacological properties of said compounds of the present invention. Those compounds having favorable pharmacological properties would be apparent to the pharmaceutical chemist, *i.e.*, those that are non-toxic and that possess such pharmacological properties to provide sufficient palatability, absorption, distribution, metabolism and excretion. Other factors, more practical in nature, that are also important in the selection are cost of raw materials, ease of crystallization, yield, stability, hygroscopicity, and flowability of the resulting bulk drug.

Representative acids and bases that may be used in the preparation of pharmaceutically acceptable salts include the following:
acids including acetic acid, 2,2-dichlorolactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, phosphoric acid, L-pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

*See*, for example, S.M. Berge, et al., "Pharmaceutical Salts", J. Pharm. Sci. 1977, 66:1-19, which is incorporated herein by reference. Examples of suitable esters include C₁₋₇alkyl, C₅₋₇cycloalkyl, phenyl, substituted phenyl, and phenylC₁₋₆alkyl- esters. Preferred esters include methyl esters.

The present invention includes within its scope prodrugs of the compounds of this invention. In general, such prodrugs will be functional derivatives of the compounds that are readily convertible *in vivo* into the required compound. Thus, in the methods of treatment of the present invention, the term "administering" shall encompass the treatment of the various disorders described with the compound specifically disclosed or with a compound that may not be specifically disclosed, but that converts to the specified compound *in vivo* after administration to the patient. Conventional procedures for the selection and preparation of suitable prodrug derivatives are described, for example, in "Design of Prodrugs", ed. H. Bundgaard, Elsevier, 1985.

Embodiments of this invention, where X is O, are made according to the synthetic methods outlined in Schemes A-D and F-L, have demonstrated LTA4H inhibitory activity, and are selected from the group consisting of:

Other embodiments of this invention, where X is S, are made according to the synthetic methods outlined in Schemes A-G, and I-L, have demonstrated LTA4H inhibitory activity, and are selected from the group consisting of:

| Example | Compound |
|---|---|
| 250 | 1-[4-(Benzothiazol-2-yloxy)-benzyl]-piperidine-4-carboxylic acid; |
| 251 | 1-{1-[4-(Benzothiazol-2-yloxy)-benzyl]-piperidin-4-yl}-pyrrolidin-2-one; |
| 252 | 2-(2-Fluoro-4-piperidin-1-ylmethyl-phenoxy)-benzothiazole; |
| 253 | N-{1-[4-(Benzothiazol-2-yloxy)-benzyl]-piperidin-4-yl}-2-hydroxy-acetamide; |
| 254 | 1-(2-{[4-(Benzothiazol-2-yloxy)-benzyl]-cyclopropyl-amino}-ethyl)-4-hydroxy-pyrrolidin-2-one; |
| 255 | N-{1-[4-(Benzothiazol-2-yloxy)-benzyl]-piperidin-4-yl}-N-methyl-methanesulfonamide; |
| 256 | 2-{4-[4-(1H-Tetrazol-5-yl)-piperidin-1-ylmethyl]-phenoxy}-benzothiazole; |
| 257 | 1-{4-[4-(Benzothiazol-2-yloxy)-benzyl]-piperazin-1-yl}-2-hydroxy-ethanone; |
| 258 | N-{1-[4-(Benzothiazol-2-yloxy)-benzyl]-piperidin-4-ylmethyl}-methanesulfonamide; |
| 259 | 3-{1-[4-(Benzothiazol-2-yloxy)-benzyl]-piperidin-4-yl}-oxazolidin-2-one; |
| 260 | 4-{1-[4-(Benzothiazol-2-yloxy)-benzyl]-piperidin-4-yl}-morpholin-3-one; |
| 271 | 2-(4-Piperidin-1-ylmethyl-phenoxy)-benzooxazole; |
| 272 | [4-(Benzothiazol-2-yloxy)-benzyl]-cyclohexyl-ethyl-amine; |
| 273 | [4-(Benzothiazol-2-yloxy)-benzyl]-cyclopropylmethyl-propyl-amine; |
| 274 | 1-[4-(Benzothiazol-2-yloxy)-benzyl]-piperidine-4-carboxylic acid amide; |
| 275 | 1'-[4-(Benzothiazol-2-yloxy)-benzyl]-[1,4']bipiperidinyl-2-one; |
| 276 | {4-[4-(Benzothiazol-2-yloxy)-benzyl]-piperazin-1-yl}-pyridin-3-yl-methanone; |
| 277 | {1-[4-(Benzothiazol-2-yloxy)-benzyl]-piperidin-4-ylmethyl}-carbamic acid tert-butyl ester; |
| 278 | {1-[4-(Benzothiazol-2-yloxy)-benzyl]-piperidin-4-ylmethyl}-carbamic acid methyl ester; |
| 279 | N-{C-[[4-(benzothiazol-2-yloxy)-benzyl]-piperidin-4-yl]-methylamino sulfonyl}-carbamic acid tert-butyl ester, |
| 280 | N-{1-[4-(Benzothiazol-2-yloxy)-benzyl]-piperidin-4-ylmethyl}-sulfamide hydrochloride, |
| 281 | N-{1-[4-(Benzothiazol-2-yloxy)-benzyl]-piperidin-4-ylmethyl}-acetamide; |
| 282 | {1-[4-(Benzothiazol-2-yloxy)-benzyl]-piperidin-4-yl}-acetic acid; |
| 283 | Acetic acid ({1-[4-(benzothiazol-2-yloxy)-benzyl]-piperidin-4-ylmethyl}-carbamoyl)-methyl ester; |
| 284 | [2-({1-[4-(Benzothiazol-2-yloxy)-benzyl]-piperidin-4-ylmethyl}-carbamoyl)-cyclobutyl]-carbamic acid tert-butyl ester; |
| 285 | 2-Amino-cyclobutanecarboxylic acid {1-[4-(benzothiazol-2-yloxy)-benzyl]-piperidin-4-ylmethyl}-amide dihydrochloride; |
| 286 | 2-(4-Pyrrolidin-1-ylmethyl-phenoxy)-benzothiazole; |
| 287 | 2-{[4-(Benzothiazol-2-yloxy)-benzyl]-ethyl-amino}-ethanol; |
| 288 | 2-{1-[4-(Benzothiazol-2-yloxy)-benzyl]-piperidin-2-yl}-ethanol; |
| 289 | 1-{4-[4-(Benzothiazol-2-yloxy)-benzyl]-piperazin-1-yl}-ethanone; |
| 290 | 8-[4-(Benzothiazol-2-yloxy)-benzyl]-2,8-diaza-spiro[4.5]decan-1-one; |
| 291 | Spiro[isobenzofuran-1(3H), 4-piperidin]-3-one, 1'-[4-(Benzothiazol-2-yloxy)-benzyl] |
| 292 | (*R*)-1-[4-(Benzothiazol-2-yloxy)-benzyl]-pyrrolidin-3-ol; |
| 293 | 2-[4-(2-Methyl-piperidin-1-ylmethyl)-phenoxy]-benzothiazole; |
| 294 | [4-(Benzothiazol-2-yloxy)-benzyl]-diethyl-amine; |
| 295 | [4-(Benzothiazol-2-yloxy)-benzyl]-butyl-methyl-amine; |
| 296 | 2-{1-[4-(Benzothiazol-2-yloxy)-benzyl]-piperidin-4-yl}-ethanol; |
| 297 | 1-[4-(Benzothiazol-2-yloxy)-benzyl]-piperidin-4-ol; |
| 298 | {1-[4-(Benzothiazol-2-yloxy)-benzyl]-piperidin-2-yl}-methanol; |
| 299 | (*R*)-{1-[4-(Benzothiazol-2-yloxy)-benzyl]-pyrrolidin-2-yl}-methanol; |
| 300 | 2-(4-Azetidin-1-ylmethyl-phenoxy)-benzothiazole; |
| 301 | 1-[4-(Benzothiazol-2-yloxy)-benzy)]-[1,4]diazepan-5-one; |
| 302 | {1-[4-(Benzothiazol-2-yloxy)-benzyl]-piperidin-3-yl}-methanol; |
| 303 | 1-[4-(Benzothiazol-2-yloxy)-benzyl]-pipeidine-3-carboxylic acid amide; |
| 304 | 9-[4-(Benzothiazol-2-yloxy)-benzyl]-3,9-diaza-spiro[5.5]undecane-3-carboxylic acid tert-butyl ester; |
| 305 | 2-{1-[4-(Benzothiazol-2-yloxy)-benzyl]-piperidin-3-yl}-ethanol; |
| 357 | 2-(4-Piperidin-1-ylmethyl-phenoxy)-benzothiazole; |
| 358 | 1-[4-(Benzothiazol-2-yloxy)-benzyl]-4-phenyl-piperidin-4-ol; |
| 359 | 1-[4-(Benzothiazol-2-yloxy)-benzyl]-piperldin-4-ol; |
| 360 | {1-[4-(Benzothiazol-2-yloxy)-benzyl]-piperidin-4-yl}-methanol; |
| 361 | N-{1-[4-(Benzothiazol-2-yloxy)-benzyl]-piperidin-4-yl}-methanesulfonamide; |
| 362 | N-{1-[4-(Benzothiazol-2-yloxy)-benzyl]-piperidin-4-ylmethyl}-2-hydroxy-acetamide; |
| 363 | {1-[4-(Benzothiazol-2-yloxy)-benzyl]-piperidin-4-yl}-carbamic acid methyl ester; |
| 364 | {1-[4-(Benzothiazol-2-yloxy)-benzyl]-piperidin-4-ylmethyl}-urea; |
| 365 | N-{1-[4-(Benzothiazol-2-yloxy)-benzyl]-piperidin-4-ylmethyl}-2,2,2-trifluoro-acetamide; |
| 366 | {4-[4-(Benzothiazol-2-yloxy)-benzyl]-piperazin-1-yl}-acetic acid; |
| 367 | 2-[4-(4-Methanesulfonyl-piperazin-1-ylmethyl)-phenoxy]-benzothiazole; |
| 368 | 1-{4-[4-(Benzothiazol-2-yloxy)-benzyl]-piperazin-1-yl}-2,2,2-trifluoro-ethanone; |
| 369 | 2-(4-Morpholin-4-ylmethyl-phenoxy)-benzothiazole; |
| 370 | {1-[4-(Benzothiazol-2-yloxy)-benzyl]-piperidin-4-yl}-carbamic acid phenyl ester; |
| 371 | N-{1-[4-(Benzothiazol-2-yloxy)-benzyl]-piperidin-4-yl}-benzenesulfonamide; |
| 372 | 3-[4-(Benzothiazol-2-yloxy)-benzylamino]-propionic acid ethyl ester; |
| 373 | 3-[4-(Benzothiazol-2-yloxy)-benzylamino]-propionic acid; |
| 434 | 1-{3-[4-(Benzothiazol-2-yloxy)-benzylamino]-propyl}-pyrrolidin-2-one; |
| 435 | 1-(3-{[4-(Benzothiazol-2-yloxy)-benzyl]-methyl-amino}-propyl)-pyrrolidin-2-one; |
| 436 | 1-(3-{[4-(Benzothiazol-2-yloxy)-benzyl]-isopropyl-amino}-propyl)-pyrrolidin-2-one; |
| 437 | 1-(3-{[4-(Benzothiazol-2-yloxy)-benzyl]-ethyl-amino}-propyl)-pyrrolidin-2-one; |
| 438 | [4-(Benzothiazol-2-yloxy)-benzyl]-cyclopropyl-amine; |
| 439 | N-1-[4-(Benzothiazol-2-yloxy)-benzyl]-N1-cyclopropyl-propane-1,3-diamine; |
| 440 | N-(3-{[4-(Benzothiazol-2-yloxy)-benzyl]-cyclopropyl-amino}-propyl)-isobutyramide; |
| 441 | 1-(3-{[4-(Benzothiazol-2-yloxy)-benzyl]-cyclopropyl-amino}-propyl)-3-isopropyl-urea; |
| 442 | 1-{1-[4-(Benzothiazol-2-yloxy)-benzyl]-piperidin-4-yl}-3-isopropyl-urea; |
| 443 | N-{1-[4-(Benzothiazol-2-yloxy)-benzyl]-piperidin-4-yl}-oxalamic acid methyl ester; |
| 444 | N-{1-[4-(Benzothiazol-2-yloxy)-benzyl]-piperidin-4-yl}-isobutyramide; |
| 445 | Tetrahydro-furan-2-carboxylic acid {1-[4-(benzothiazol-2-yloxy)-benzyl]-piperidin-4-yl}-amide; |
| 446 | 1-{1-[4-(Benzothiazol-2-yloxy)-benzyl]-piperidin-4-yl}-4-hydroxy-pyrrolidin-2-one; |
| 447 | 1-{1-[4-(Benzothiazol-2-yloxy)-benzyl]-piperidin-4-yl}-4-hydroxy-pyrrolidin-2-one; |
| 448 | {1-[4-(Benzothiazol-2-yloxy)-benzyl]-piperidin-4-yl}-urea; |
| 449 | N-{1-[4-(Benzothiazol-2-yloxy)-benzyl]-piperidin-4-yl}-oxalamic acid; |
| 450 | N-{1-[4-(Benzothiazol-2-yloxy)-benzyl]-piperidin-4-yl}-2-hydroxy-acetamide; |
| 451 | N-{1-[4-(Benzothiazol-2-yloxy)-benzyl]-piperidin-4-yl}-2,2,2-trifluoro-acetamide; |
| 452 | 2-[4-(1,1-Dioxo-1l6-thiomorpholin-4-ylmethyl)-phenoxy]-benzothiazole; |
| 453 | N-{1-[4-(benzothiazol-2-yloxy)-benzyl]-piperidin-4-amino sulfonyl}-carbamic acid tert-butyl ester; |
| 454 | N-{1-[4-(Benzothiazol-2-yloxy)-benzyl]-piperidin-4-yl}-acetamide; |
| 455 | N-{1-[4-(Benzothiazol-2-yloxy)-benzyl]-piperidin-4-yl}-N,N-dimethylsulfamide; |
| 456 | 1-{1-[4-(Benzothiazol-2-yloxy)-benzyl]-piperidin-4-yl}-3-ethyl-urea; |
| 457 | 1-{1-[4-(Benzothiazol-2-yloxy)-benzyl]-piperidin-4-yl}-3-ethyl-thiourea; |
| 458 | Propane-1-sulfonic acid {1-[4-(benzothiazol-2-yloxy)-benzyl]-piperidin-4-yl}-amide; |
| 459 | Propane-2-sulfonic acid {1-[4-(benzothiazol-2-yloxy)-benzyl]-piperidin-4-yl}-amide; |
| 460 | N-{1-[4-(Benzothiazol-2-yloxy)-benzyl]-piperidin-4-yl}-sulfamide; |
| 461 | N-{1-[4-(Benzothiazol-2-yloxy)-benzyl]-piperidin-4-yl}-formamide; |
| 462 | {1-[4-(Benzothiazol-2-yloxy)-benzyl]-piperidin-4-yl}-carbamic acid ethyl ester; |
| 463 | N-{1-[4-(Benzothiazol-2-yloxy)-benzyl]-piperidin-4-yl}-propionamide; |
| 464 | N-{1-[4-(Benzothiazol-2-yloxy)-benzyl]-piperidin-4-yl}-butyramide; |
| 465 | 1-{1-[4-(Benzothiazol-2-yloxy)-benzyl]-piperidin-4-yl}-3-propyl-urea; |
| 466 | {1-[4-(Benzothiazol-2-yloxy)-benzyl]-piperidin-4-yl}-carbamic acid propyl ester; |
| 467 | 1-{1-[4-(Benzothiazol-2-yloxy)-benzyl]-piperidin-4-yl}-3-methyl-urea; |
| 469 | 1-{1-[4-(Benzothiazol-2-yloxy)-benzyl]-piperidin-4-yl}-1,3-dimethyl-urea; |
| 470 | 1-{1-[4-(Benzothiazol-2-yloxy)-benzyl]-piperidin-4-yl}-1-methyl-urea; |
| 471 | N-{1-[4-(Benzothiazol-2-yloxy)-benzyl]-piperidin-4-yl}-N-methyl-acetamide; |
| 472 | {1-[4-(Benzothiazol-2-yloxy)-benzyl]-piperidin-4-yl}-methyl-carbamic acid methyl ester; |
| 473 | N-{1-[4-(Benzothiazol-2-yloxy)-benzyl]-piperidin-4-yl}-N-methyl-oxalamic acid methyl ester; |
| 474 | N-{1-[4-(Benzothiazol-2-yloxy)-benzyl]-piperidin-4-yl}-N-methyl-oxalamic acid; |
| 475 | Guanidine, N-{1-[4-(Benzothiazol-2-yloxy)-benzyl]-piperidin-4-yl}-N'-hydroxy; |
| 476 | {1-[4-(Benzothiazol-2-yloxy)-benzyl]-piperidin-4-yl}carbamic acid isopropyl ester; |
| 477 | 3-{1-[4-(Benzothiazol-2-yloxy)-benzyl]-piperidin-4-yl}-1,1-dimethyl-urea; |
| 478 | Acetic acid {1-[4-(benzothiazol-2-yloxy)-benzyl]-piperidin-4-ylcarbamoyl}-methyl ester; |
| 479 | {1-[4-(Benzothiazol-2-yloxy)-benzyl]-piperidin-4-yl}-thiourea; |

Additional embodiments of this invention, where X is NR⁵ with R⁵ being one of H and CH₃, are made according to the synthetic methods outlined in Schemes A-C, F, G and J-L, have demonstrated LTA4H inhibitory activity, and are selected from the group consisting of:

Some preferred compounds found in the context of this invention include the following:

| | |
|---|---|
| 271 | 2-(4-Piperidin-1-ylmethyl-phenoxy)-benzooxazole; |
| 250 | 1-[4-(Benzothiazol-2-yloxy)-benzyl]-piperidine-4-carboxylic acid; |
| 251 | 1-{1-[4-(Benzothiazol-2-yloxy)-benzyl]-piperidin-4-yl}-pyrrolidin-2-one; |
| 252 | 2-(2-Fluoro-4-piperidin-1-ylmethyl-phenoxy)-benzothiazole; |
| 253 | N-{1-[4-(Benzothiazol-2-yloxy)-benzyl]-piperidin-4-yl}-2-hydroxy-acetamide; |
| 254 | 1-(2-{[4-(Benzothiazol-2-yloxy)-benzyl]-cyclopropyl-amino}-ethyl)-4-hydroxy-pyrrolidin-2-one; |
| 255 | N-{1-[4-(Benzothiazol-2-yloxy)-benzyl]-piperidin-4-yl}-N-methyl-methanesulfonamide; |
| 256 | 2-{4-[4-(1H-Tetrazol-5-yl)-piperidin-1-ylmethyl]-phenoxy}-benzothiazole; |
| 257 | 1-{4-[4-(Benzothiazol-2-yloxy)-benzyl]-piperazin-1-yl}-2-hydroxy-ethanone; |
| 258 | N-{1-[4-(Benzothiazol-2-yloxy)-benzyl]-piperidin-4-ylmethyl}-methanesulfonamide; |
| 259 | 3-{1-[4-(Benzothiazol-2-yloxy)-benzyl]-piperidin-4-yl}-oxazolidin-2-one; |
| 260 | 4-{1-[4-(Benzothiazol-2-yloxy)-benzyl]-piperidin-4-yl}-morpholin-3-one; |
| 357 | 2-(4-Piperidin-1-ylmethyl-phenoxy)-benzothiazole; |
| 358 | 1-[4-(Benzothiazol-2-yloxy)-benzyl]-4-phenyl-piperidin-4-ol; |
| 359 | 1-[4-(Benzothiazol-2-yloxy)-benzyl]-piperidin-4-ol; |
| 360 | {1-[4-(Benzothiazol-2-yloxy)-benzyl]-piperidin-4-yl}-methanol; |
| 361 | N-{1-[4-(Benzothiazol-2-yloxy)-benzyl]-piperidin-4-yl}-methanesulfonamide; |
| 362 | N-{1-[4-(Benzothiazol-2-yloxy)-benzyl]-piperidin-4-ylmethyl}-2-hydroxy-acetamide; |
| 363 | {1-[4-(Benzothiazol-2-yloxy)-benzyl]-piperidin-4-yl}-carbamic acid methyl ester; |
| 364 | {1-[4-(Benzothiazol-2-yloxy)-benzyl]-piperidin-4-ylmethyl}-urea; |
| 365 | N-{1-[4-(Benzothiazol-2-yloxy)-benzyl]-piperidin-4-ylmethyl}-2,2,2-trifluoro-acetamide; |
| 366 | {4-[4-(Benzothiazol-2-yloxy)-benzyl]-piperazin-1-yl}-acetic acid; |
| 367 | 2-[4-(4-Methanesulfonyl-piperazin-1-ylmethyl)-phenoxy]-benzothiazole; |
| 368 | 1-{4-[4-(Benzothiazol-2-yloxy)-benzyl]-piperazin-1-yl}-2,2,2-trifluoro-ethanone; |
| 369 | 2-(4-Morpholin-4-ylmethyl-phenoxy)-benzothiazole; |
| 370 | {1-[4-(Benzothiazol-2-yloxy)-benzyl]-piperidin-4-yl}-carbamic acid phenyl ester; |
| 371 | N-{1-[4-(Benzothiazol-2-yloxy)-benzyl]-piperidin-4-yl}-benzenesulfonamide; |
| 372 | 3-[4-(Benzothiazol-2-yloxy)-benzylamino]-propionic acid ethyl ester; |
| 373 | 3-[4-(Benzothiazol-2-yloxy)-benzylamino]-propionic acid; |
| 434 | 1-{3-[4-(Benzothiazol-2-yloxy)-benzylamino]-propyl}-pyrrolidin-2-one; |
| 435 | 1-(3-{[4-(Benzothiazol-2-yloxy)-benzyl]-methyl-amino}-propyl)-pyrrolidin-2-one; |
| 436 | 1-(3-{[4-(Benzothiazol-2-yloxy)-benzyl]-isopropyl-amino}-propyl)-pyrrolidin-2-one; |
| 437 | 1-(3-{[4-(Benzothiazol-2-yloxy)-benzyl]-ethyl-amino}-propyl)-pyrrolidin-2-one; |
| 438 | [4-(Benzothiazol-2-yloxy)-benzyl]-cyclopropyl-amine; |
| 439 | N-1-[4-(Benzothiazol-2-yloxy)-benzyl]-N1-cyclopropyl-propane-1,3-diamine; |
| 440 | N-(3-{[4-(Benzothiazol-2-yloxy)-benzyl]-cyclopropyl-amino}-propyl)-isobutyramide; |
| 441 | 1-(3-{[4-(Benzothiazol-2-yloxy)-benzyl]-cyclopropyl-amino}-propyl)-3-isopropyl-urea; |
| 442 | 1-{1-[4-(Benzothiazol-2-yloxy)-benzyl]-piperidin-4-yl}-3-isopropyl-urea; |
| 443 | N-{1-[4-(Benzothiazol-2-yloxy)-benzyl]-piperidin-4-yl}-oxalamic acid methyl ester; |
| 444 | N-{1-[4-(Benzothiazol-2-yloxy)-benzyl]-piperidin-4-yl}-isobutyramide; |
| 445 | Tetrahydro-furan-2-carboxylic acid {1-[4-(benzothiazol-2-yloxy)-benzyl]-piperidin-4-yl}-amide; |
| 446 | 1-{1-[4-(Benzothiazol-2-yloxy)-benzyl]-piperidin-4-yl}-4-hydroxy-pyrrolidin-2-one; |
| 447 | 1-{1-[4-(Benzothiazol-2-yloxy)-benzyl]-piperidin-4-yl}-4-hydroxy-pyrrolidin-2-one; |
| 448 | {1-[4-(Benzothiazol-2-yloxy)-benzyl]-piperidin-4-yl}-urea; |
| 449 | N-{1-[4-(Benzothiazol-2-yloxy)-benzyl]-piperidin-4-yl}-oxalamic acid; |
| 450 | N-{1-[4-(Benzothiazol-2-yloxy)-benzyl]-piperidin-4-yl}-2-hydroxy-acetamide; |
| 451 | N-{1-[4-(Benzothiazol-2-yloxy)-benzyl]-piperidin-4-yl}-2,2,2-trifluoro-acetamide; |
| 452 | 2-[4-(1,1-Dioxo-116-thiomorpholin-4-ylmethyl)-phenoxy]-benzothiazole; |
| 453 | N-{1-[4-(benzothiazol-2-yloxy)-benzyl]-piperidin-4-amino sulfonyl}-carbamic acid tert-butyl ester; |
| 454 | N-{1-[4-(Benzothiazol-2-yloxy)-benzyl]-piperidin-4-yl}-acetamide; |
| 455 | N-{1-[4-(Benzothiazol-2-yloxy)-benzyl]-piperidin-4-yl)-N,N-dimethylsulfamide; |
| 456 | 1-{1-[4-(Benzothiazol-2-yloxy)-benzyl]-piperidin-4-yl}-3-ettiyl-urea; |
| 457 | 1-{1-[4-(Benzothiazol-2-yloxy)-benzyl]-piperidin-4-yl}-3-ethyl-thiourea; |
| 458 | Propane-1-sulfonic acid {1-[4-(benzothiazol-2-yloxy)-benzyl]-piperidin-4-yl}-amide; |
| 459 | Propane-2-sulfonic acid {1-[4-(benzothiazol-2-yloxy)-benzyl]-piperidin-4-yl}-amide; |
| 460 | N-{1-[4-(Benzothiazol-2-yloxy)-benzyl]-piperidin-4-yl}-sulfamide; |
| 461 | N-{1-[4-(Benzothiazol-2-yloxy)-benzyl]-piperidin-4-yl}-formamide; |
| 462 | {1-[4-(Benzothiazol-2-yloxy)-benzyl]-piperidin-4-yl}-carbamic acid ethyl ester; |
| 463 | N-{1-[4-(Benzothiazol-2-yloxy)-benzyl]-piperidin-4-yl}-propionamide; |
| 464 | N-{1-[4-(Benzothiazol-2-yloxy)-benzyl]-piperidin-4-yl}-butyramide; |
| 465 | 1-{1-[4-(Benzothiazol-2-yloxy)-benzyl]-piperidin-4-yl}-3-propyl-urea; |
| 466 | {1-[4-(Benzothiazol-2-yloxy)-benzyl]-piperidin-4-yl}-carbamic acid propyl ester; |
| 467 | 1-{1-[4-(Benzothiazol-2-yloxy)-benzyl]-piperidin-4-yl}-3-methyl-urea; |
| 469 | 1-{1-[4-(Benzothiazol-2-yloxy)-benzyl]-piperidin-4-yl}-1,3-dimethyl-urea; |
| 470 | 1-{1-[4-(Benzothiazol-2-yloxy)-benzyl]-piperidin-4-yl}-1-methyl-urea; |
| 471 | N-{1-[4-(Benzothiazol-2-yloxy)-benzyl]-piperidin-4-yl}-N-methyl-acetamide; |
| 472 | {1-[4-(Benzothiazol-2-yloxy)-benzyl]-piperidin-4-yl}-methyl-carbamic acid methyl ester; |
| 473 | N-{1-[4-(Benzothiazol-2-yloxy)-benzyl]-piperidin-4-yl}-N-methyl-oxalamic acid methyl ester; |
| 474 | N-{1-[4-(Benzothiazol-2-yloxy)-benzyl]-piperidin-4-yl}-N-methyl-oxalamic acid; |
| 475 | Guanidine, N-{1-[4-(Benzothiazol-2-yloxy)-benzyl]-piperidin-4-yl}-N'-hydroxy; |
| 476 | {1-[4-(Benzothiazol-2-yloxy)-benzyl]-piperidin-4-yl}-carbamic acid isopropyl ester; |
| 477 | 3-{1-[4-(Benzothiazol-2-yloxy)-benzyl]-piperidin-4-yl}-1,1-dimethyl-urea; |
| 478 | Acetic acid {1-[4-(benzothiazol-2-yloxy)-benzyl]-piperidin-4-ylcarbamoyl}-methyl ester; |
| 479 | {1-[4-(Benzothiazol-2-yloxy)-benzyl]-piperidin-4-yl}-thiourea; |
| 488 | 2-(4-Piperidin-1-ylmethyl-phenoxy)-1H-benzoimidazole |
| 535 | 1-[4-(1H-Benzoimidazol-2-yloxy)-benzyl]-piperidine-4-carboxylic acid |

The foregoing compounds either were prepared as explicitly described herein, or for those that are not accompanied by an explicit description of how they were made, their preparation followed variations of the illustrative preparations described herein that can be implemented in light of the present description together with the ordinary skill in the art.

Compounds of the present invention may be prepared according to the reaction schemes described below. The schemes represent two basic approaches to target compound synthesis, both in a linear fashion, commencing from either end of the molecule. Persons skilled in the art will recognize that certain compounds are more advantageously produced by one scheme over another.

To obtain the various compounds herein, starting materials may be employed which carry the ultimately desired substituents though the reaction scheme with or without protection as appropriate. Starting materials may be obtained from commercial sources or synthesized by methods known to one skilled in the art. Alternatively, it may be necessary to employ, in the place of the ultimately desired substituent, a suitable group, which may be carried through the reaction scheme and replaced as appropriate with the desired substituent. Any product containing a chiral center may be separated into its enantiomers by conventional techniques.

Embodiments of processes illustrated herein include, when chemically meaningful, one or more steps such as hydrolysis, halogenation, protection, and deprotection: These steps can be implemented in light of the teachings provided herein and the ordinary skill in the art.

During any of the processes for preparation of the compounds of the present invention, it may be necessary and/or desirable to protect sensitive or reactive groups on any of the molecules concerned. In addition, compounds of the invention may be modified by using protecting groups; such compounds, precursors, or prodrugs are also within the scope of the invention. This may be achieved by means of conventional protecting groups, such as those described in "Protective Groups in Organic Chemistry", ed. J.F.W. McOmie, Plenum Press, 1973; and T.W. Greene & P.G.M. Wuts, "Protective Groups in Organic Synthesis", 3rd ed., John Wiley & Sons, 1999. The protecting groups may be removed at a convenient subsequent stage using methods known from the art.

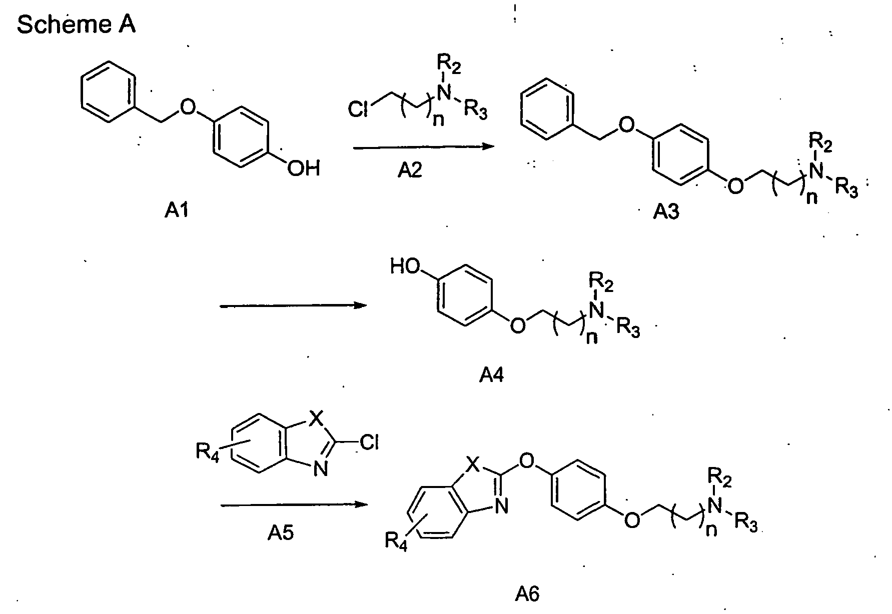

Referring to Scheme A, n is 1 or 2 commercially available 4-benzyloxyphenol, A1, is alkylated with amino alkyl halides, A2; several amino alkyl chlorides are commercially available. The reactions can be run under a wide range of temperatures, including room temperature and more elevated temperatures, in the presence of an inorganic base known to facilitate O-alkylation, such as, but not limited to, K₂CO₃, Cs₂CO₃ and mixtures thereof (J. Med. Chem, 1997, 40, 1407-1416). Suitable solvents include but are not limited to DMF. Removal of the benzyl group on A3 may be accomplished using catalytic hydrogenation conditions well known to those skilled in the art (Greene, T.W.; Wuts, P.G.M. Protective Groups in Organic Synthesis, 3rd ed.; John Wiley & Sons: New York, 1999.). Suitable catalysts include but are not limited to Pd on carbon (Pd/C), in solvents such as ethyl acetate, alcohols and mixtures thereof. Examples of alcohols include but are not limited to CH₃OH, ethanol, *i*-PrOH. These reactions are typically run at room temperature. Removal of the benzyl group on A3 may be accomplished in some embodiments by using dissolving metal reductions or transfer hydrogenation conditions at suitable temperatures. For example, dissolving metal reductions are typically performed at temperatures below room temperature (-33 °C). Reaction of A4 with the aromatic bicyclic ring system, A5, suitably protected if appropriate, may be accomplished within a wide range of temperatures including room temperature and more elevated temperatures in the presence of a suitable base including but not limited to amine or inorganic base as defined above. Suitable amine bases include but are not limited to triethylamine (TEA), *N,N*-diisopropylethylamine, 1,8-diazabicyclo[5.4.0]undec-7-ene (DBU), resin-bound amine bases and mixtures thereof. When X is, oxygen or sulfur, protecting groups are not applicable. Suitable solvents include but are not limited to DMF, CH₃CN, acetone and mixtures thereof. When X is NR⁵, and R⁵ is a suitable silicon based protecting group, such as SEM (trimethylsilylethoxymethyl), removal of the silicon-based protecting group on NR⁵ can be accomplished using conditions well known to those skilled in the art (Greene et. al. as cited above). Typical reaction conditions include but are not limited to the use of tetrabutylammonium fluoride (TBAF), in suitable solvents such as THF at elevated temperatures.

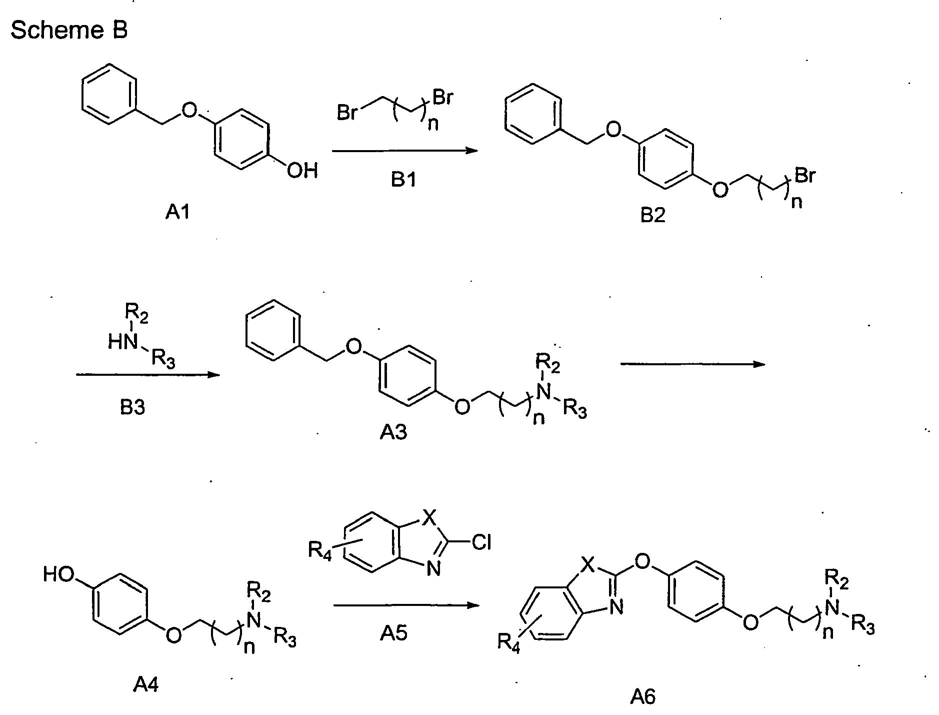

Referring to Scheme B, commercially available 4-benzyloxyphenol, A1, is alkylated with dihaloalkanes, preferably dibromoalkanes such as 1,2-dibromoethane and 1,3-dibromopropane, B1, both of which are commercially available, under a wide range of temperatures with elevated temperatures preferred (Zhou, Z. -L. et al., J. Med. Chem. 1999, 42:2993-3000). The reactions are conducted in the presence of an inorganic base known to facilitate O-alkylation such as, but not limited to, K₂CO₃, Cs₂CO₃ and mixtures thereof. Suitable solvents include but are not limited to CH₃CN and DMF. Compounds of structure B2 are treated with amines, B3, either in the presence or absence of a suitable amine base as described above under a wide range of temperatures with elevated temperatures preferred. Suitable solvents include CH₃CN, CH₂Cl₂ and DMF. Further conversion of the resulting products, of structure A3, to compounds of structure A6, is as detailed above for Scheme A.

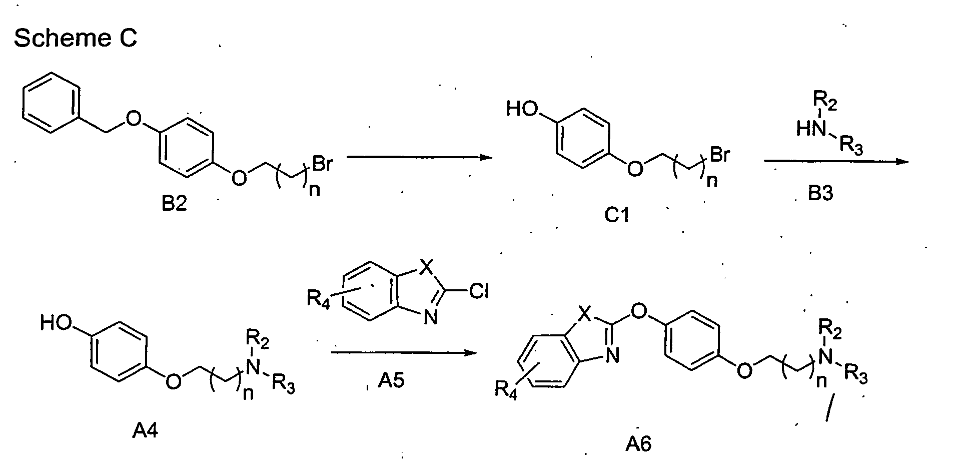

Referring to Scheme C, the benzyl group of compounds of structure B2 can be removed using catalytic hydrogenation conditions well known to those skilled in the art (Greene et. al. as cited above). Suitable catalysts include but are not limited to Pd/C, in solvents such as THF and THF/ethanol mixtures. These reactions are typically run at room temperature. Removal of the benzyl group on B2 can be accomplished in some embodiments using transfer-hydrogenation conditions using suitable solvents and temperatures. Compounds of general structure C1 are treated with amines of structure B3 either in the presence or absence of a suitable amine base as described above under a wide range of temperatures with elevated temperatures preferred. Suitable solvents include but are not limited to CH₃CN, CH₂Cl₂ and DMF. Further conversion of the resulting products, A4, to compounds A6, is as detailed above for Scheme A.

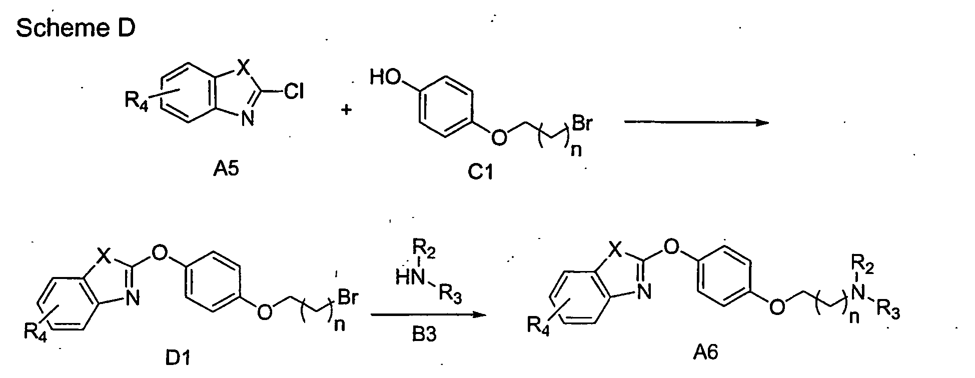

Referring to Scheme D, the compounds of structure A6 can also be prepared by treatment of compounds of structure C1 with aromatic bicyclic compounds, A5, where X = S and O, in the presence of a suitable inorganic base, as defined above, under a wide range of temperatures with elevated temperatures preferred. Suitable solvents include but are not limited to DMF, CH₃CN and mixtures thereof. Conversion of compounds of structure D1 to compounds of structure A6 can be accomplished by treatment with compounds of structure B3. These reactions can be performed either in the presence or absence of a suitable amine base as defined above or an inorganic base such as, but not limited to, K₂CO₃, Cs₂CO₃ and mixtures thereof as described above, under a wide range of temperatures with elevated temperatures preferred. Suitable solvents include but are not limited to CH₃CN and DMF.

It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as (Greene et. al. as cited above).

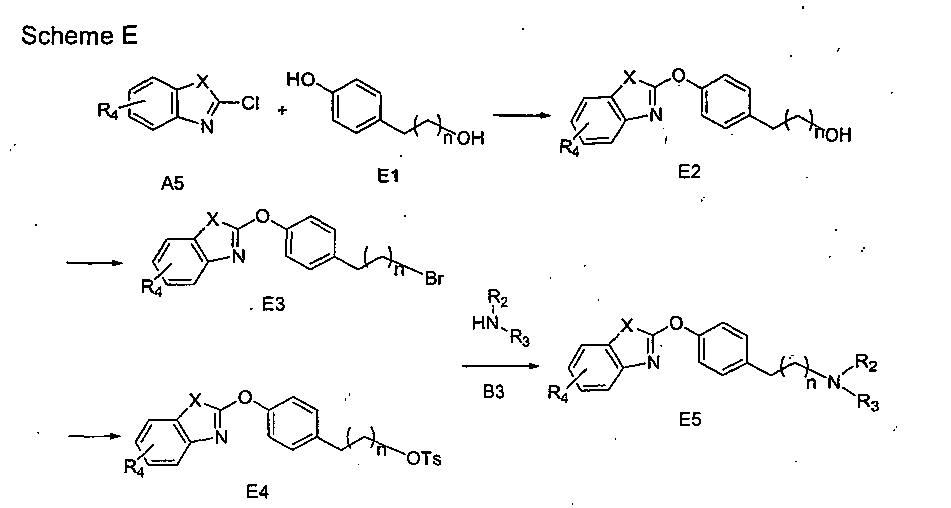

Referring to Scheme E, treatment of compounds of structure E1 with aromatic bicyclic compounds, A5, where X is Swhy not O also?, in the presence of a suitable inorganic base, as defined above, under a wide range of temperatures with elevated temperatures are preferred. Suitable solvents include but are not limited to DMF, CH₃CN and mixtures thereof. Compounds of structure E2 can be converted to compounds of structure E3 using typical brominating conditions including but not limited to the use of PBr₃ at elevated temperatures. Suitable solvents include but are not limited to benzene. Compounds of structure E2 can also be converted to compounds of structure E4 using standard conditions for sulphonylation well known to those skilled in the art. These include but are not limited to the use of TsCl to prepare tosylates, as denoted in the scheme, in the presence of an amine base at room temperature in CH₂Cl₂. Conversion of compounds of structure E3 to compounds of structure E5 can be accomplished by treatment with compounds of structure B3. These reactions can be performed either in the presence or absence of a suitable amine base as described above or an inorganic base such as, but not limited to, K₂CO₃, Cs₂CO₃ and mixtures thereof under a wide range of temperatures with elevated temperatures preferred. Suitable solvents include but are not limited to CH₃CN and DMF. Compounds of structure E4 can be converted to the compounds of the structure E5 by treatment with compounds of structure B3. These reactions can be performed either in the presence or absence of a suitable amine base as described above under a wide range of temperatures. Suitable solvents include but are not limited to CH₃CN and DMF.

It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as Greene et. al. (as cited above).

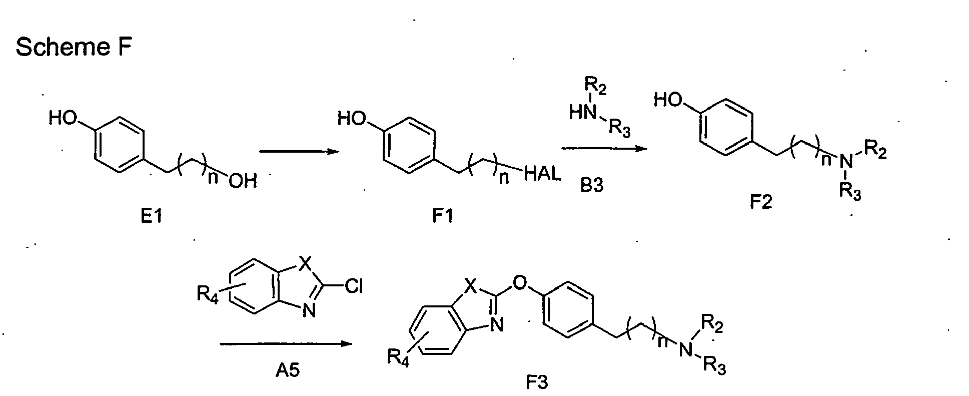

Referring to Scheme F, commercially available 4-(2-hydroxy-ethyl)-phenol and 4-(2-hydroxy-propyl)-phenol are converted to the corresponding alkyl halides F1, where HAL is chloride or bromide, using typical brominating or chlorinating conditions. These conditions include but are not limited to treatment with 48% HBr solutions at elevated temperatures. The resulting bromophenols of structure F1 can then be treated with amines of the structure of B3 either in the presence or absence of a suitable amine base as described above under a wide range of temperatures. Suitable solvents include but are not limited to CH₃CN and DMF. Reaction of F2 with the aromatic bicyclic ring system, A5, suitably protected if appropriate, may be accomplished within a wide range of temperatures including room temperature and more elevated temperatures, in the presence of a suitable amine or inorganic base as defined above. Suitable solvents include but are not limited to DMF, CH₃CN, acetone and mixtures thereof. When X is O or S, protecting groups are not applicable. When X is NR⁵, and R⁵ is a suitable silicon-based protecting group, such as SEM (trimethylsilylethoxymethyl), removal of the silicon-based protecting group on NR⁵ can be accomplished using conditions well known to those skilled in the art (Greene et. al. as cited above). Typical reaction conditions include but are not limited to the use of TBAF, in suitable solvents such as THF at elevated temperatures.

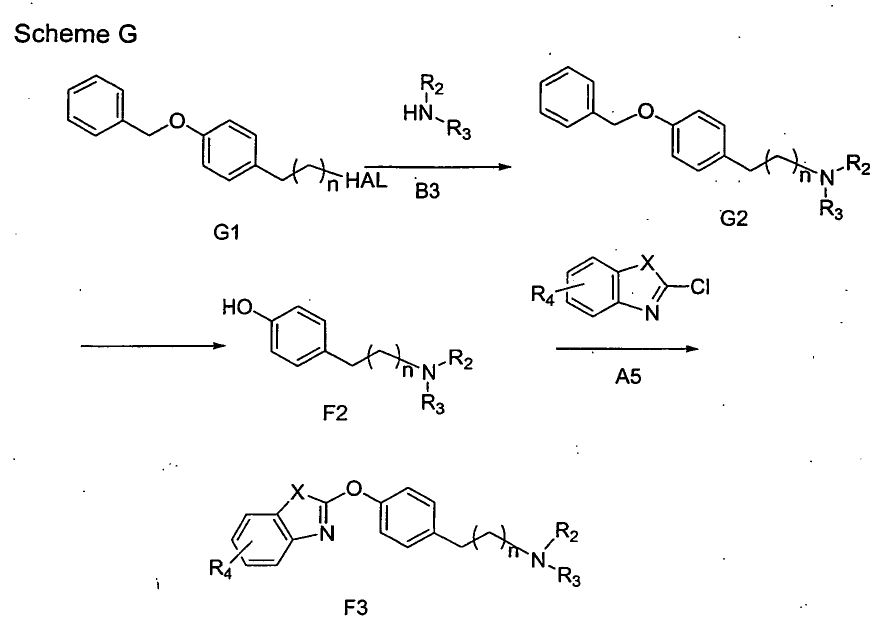

Referring to Scheme G, G1, where n is 0 or 2 and HAL is bromide or chloride, are commercially available materials or may be obtained from **, and G1, where n is 1, is envisaged to be available using standard alkylation conditions starting from 4-(2-hydroxy-ethyl)-phenol and benzyl bromide. The benzyl group in G1 serves as a protecting group. Other compatible protecting groups known to one skilled in the art may be employed in this sequence. Compounds with the general structure G2 can be obtained by treatment with amines of the general structure B3, either in the presence or absence of a suitable amine base as described above under a wide range of temperatures. Suitable solvents include but are not limited to CH₃CN and DMF. Removal of the benzyl may be accomplished using catalytic hydrogenation conditions well known to those skilled in the art (Greene et. al. as cited above). Suitable catalysts include but are not limited to Pd/C, in solvents such as ethyl acetate, alcohols and mixtures thereof. Examples of alcohols include but are not limited to CH₃OH, ethanol, *i*-PrOH. These reactions are typically run at room temperature. Removal of the benzyl group on G2 may be accomplished in some embodiments using transfer-hydrogenation conditions at suitable temperatures. Further conversion of the resulting products, F2, to the final target compounds F3 is as detailed above for Scheme F.

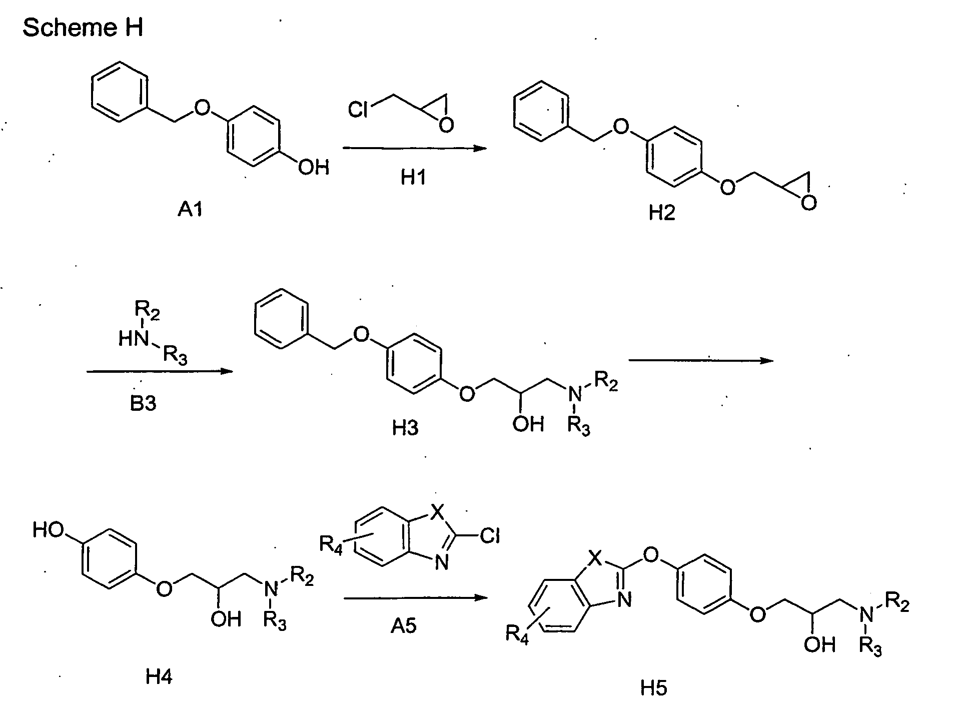

Referring to Scheme H, commercially available 4-benzyloxyphenol, A1, is treated with epichlorohydrin, H1, both of which are commercially available. The reaction can be run under a wide range of temperatures, with elevated temperatures preferred, in the presence of an inorganic base such as, but not limited to, K₂CO₃, Cs₂CO₃ and mixtures thereof. Suitable solvents include but are not limited to DMF. Conversion of compounds of structure H2 to compounds of structure H3 can be accomplished by treatment with amines of general structure B3 either in the presence or absence of a suitable amine base as described above or an inorganic base such as, but not limited to, K₂CO₃, Cs₂CO₃ and mixtures thereof under a wide range of temperatures with elevated temperatures preferred. Suitable solvents include but are not limited to CH₃CN and DMF. Removal of the benzyl group on H3 may be accomplished using catalytic-hydrogenation conditions well known to those skilled in the art (Greene et. al. as cited above). Suitable catalysts include but are not limited to Pd/C, in solvents such as ethyl acetate, alcohols and mixtures thereof. Examples of alcohols include but are not limited to ethanol, CH₃OH, i-PrOH. These reactions are typically run at room temperature. Removal of the benzyl group on B2 can be accomplished in some embodiments using transfer-hydrogenation conditions using suitable solvents and temperatures.

Conversion of compounds of structure H4 to final target compounds H5 can be accomplished by treatment with the aromatic bicyclic ring system, A5, where X is O, in the presence of a suitable inorganic base, as defined above, under a wide range of temperatures with lower temperatures preferred. Suitable solvents include but are not limited to acetone.

It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as Greene et. al. (as cited above).

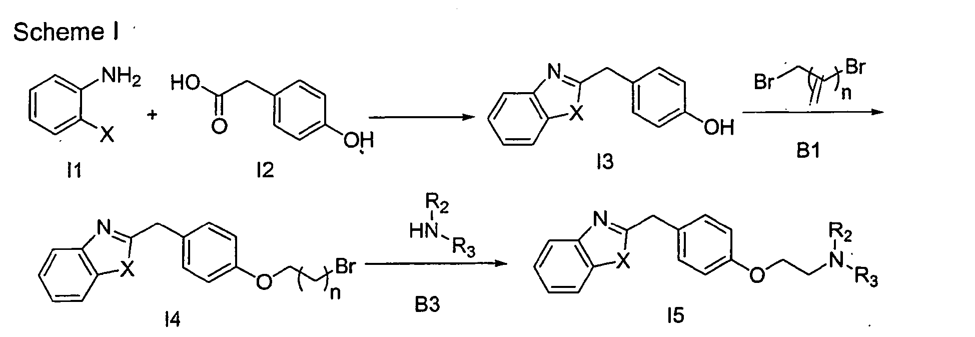

Referring to Scheme I, compounds of type 15 are prepared by heating commercially available 4-hydroxyphenyl acetic acid with, in the case of X is S, 2-aminothiophenol. In the case of X is O, 2-aminophenol is used. The two starting materials are heated in the absence of solvent, and the resulting phenols, 13, are treated with dihaloalkanes, preferably dibromoalkanes such as 1,2-dibromoethane and 1,3-dibromopropane, B1, both of which are commercially available, under a wide range of temperatures with elevated temperatures preferred *(Zhou, Z. -L. et al*. as cited above). The reactions are conducted in the presence of an inorganic base known to facilitate O-alkylation such as, but not limited to, K₂CO₃, Cs₂CO₃ and mixtures thereof. Suitable solvents include but are not limited to CH₃CN and DMF. Compounds of structure I4 are treated with amines, B3, either in the presence or absence of a suitable amine base as described above under a wide range of temperatures with elevated temperatures preferred. Suitable solvents include CH₃CN, CH₂Cl₂ and DMF.

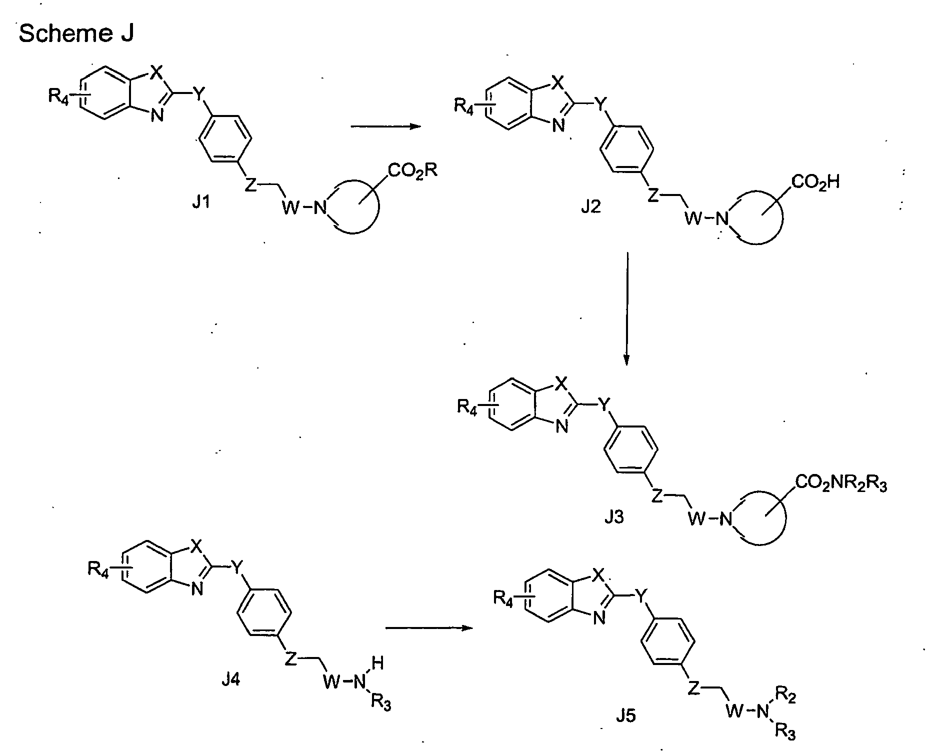

Referring to Scheme J, compounds of the structure J1 can be further elaborated within the limits of the claims to give more highly functionalized target compounds. For example, hydrolysis using methods well known to those skilled in the art such as but not limited to the use of aqueous solutions of LiOH, KOH or NaOH, or aqueous solutions of HCl or CH₃CO₂H, or the use of (CH₃)₃SiOK. Furthermore, persons skilled in the art will recognize that certain compounds are more advantageously produced by one method as compared to another and that salts of the desired compounds may initially result. Compounds of the structure J2 can be further modified to give amides using methods well known to those skilled in the art including but not limited to using (COCl₂)₂ to convert to the intermediary acid chloride followed by exposure to amines of the structure B3. Alternatively, standard amide bond-forming conditions may be utilized, including but not limited to the use of 1,(3-dimethylaminopropyl)-3-ethylcarbodiimide (EDCI), with or without additives such as HOBT, and amines of the structure B3. Compounds of the structure J4 can be further modified by reductive amination using standard conditions well known to those skilled in the art, including but not limited to the use of an amine of the structure B3 and NaBH(OAc)₃ in an appropriate solvent such as CH₂Cl₂, ClCH₂CH₂Cl or CF₃CH₂OH.

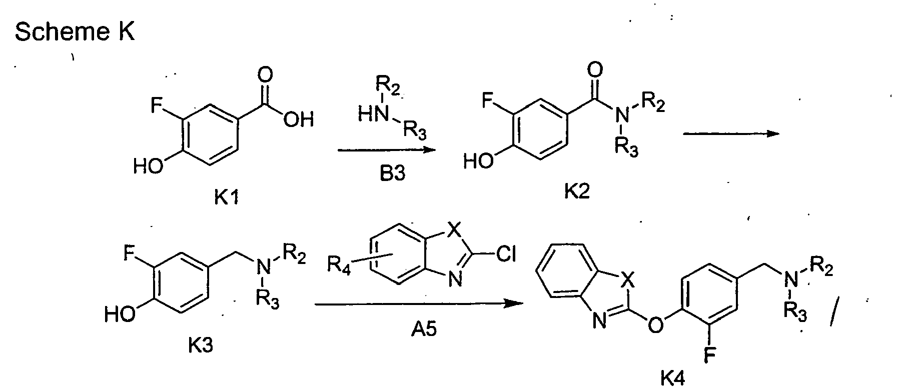

Referring to Scheme K, commercially available 3-fluoro-4-hydroxybenzoic acid, K11, is converted to amides K2, with amines of structure B3, using standard peptide coupling conditions well known to those skilled in the art such as, but not limited to, 1-(3-dimethylaminopropyl)-3-ethylcarbodiimide hydrochloride (EDCI), 1,3-dicyclohexylcarbodiimide (DCC), *O*-(7-azabenzotriazol-1-yl)-*N,N,N',N*'-tetramethyluronium hexafluorophoshate (HATU), *O*-benzotriazol-1-*N*,*N,N',N'*-tetramethyluronium hexafluorophosphate (HBTU) and mixtures thereof. Suitable solvents include, but are not limited to, CH₂Cl₂ and THF. The resulting amides of structure K2 are reduced to amines of formula K3 under reducing conditions well known to those skilled in the art, including but not limited to, lithium aluminum hydride in an appropriate solvent such as, but not limited to, THF. Conversion of benzyl amines K3 to the final target compounds, K4, can be accomplished by treatment with the aromatic bicyclic ring system, A5, where X is S or O, in the presence of a suitable inorganic base under a wide range of temperatures with elevated temperatures preferred. Suitable inorganic bases include, but are not limited to, K₂CO₃, Cs₂CO₃ and mixtures thereof. Suitable solvents include, but are not limited to, acetone and CH₃CN.

It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as Greene et. al. (as cited above).

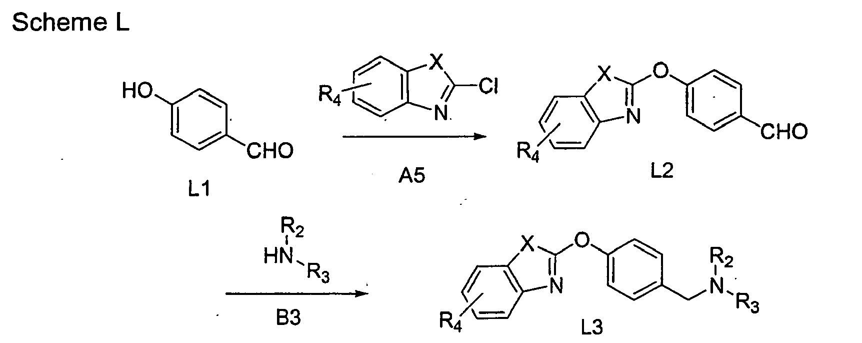

Referring to Scheme L, an alternate embodiment toward the preparation of compounds of formula (I) where n is 0 is described. The starting material L1, 4-hydroxybenzaldehyde, is converted to ethers of formula L2 by treatment with the aromatic bicyclic ring system, A5, where X is S or O, in the presence of a suitable inorganic base under a wide range of temperatures with elevated temperatures preferred. Suitable inorganic bases include, but are not limited to, K₂CO₃, Cs₂CO₃ and mixtures thereof. Suitable solvents include, but are not limited to, acetone and CH₃CN. It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as Greene et. al. (as cited above). Aldehydes of formula L2 are converted to amines of formula L3 under reductive amination conditions with amines of formula B3. Suitable reducing agents include Na(OAc)₃BH and NaCNBH₃, with or without the addition of activating agents such as acetic acid or ZnCl₂. Suitable solvents include THF and methanol, and reaction temperatures may range from 0 °C to 70 °C. Preferred reaction conditions are Na(OAc)₃BH in THF at room temperature.

Pharmaceutically acceptable salts, esters, and amides of compounds according to the present invention refer to those salt, ester, and amide forms of the compounds of the present invention which would be apparent to the pharmaceutical chemist, *i.e.,* those which are non-toxic and which would favorably affect the pharmacokinetic properties of said compounds of the present invention. Those compounds having favorable pharmacokinetic properties would be apparent to the pharmaceutical chemist, *i.e.,* those which are non-toxic and which possess such pharmacokinetic properties to provide sufficient palatability, absorption, distribution, metabolism and excretion. Other factors, more practical in nature, which are also important in the selection, are cost of raw materials, ease of crystallization, yield, stability, hygroscopicity and flowability of the resulting bulk drug.

In addition, acceptable salts of carboxylates include sodium, potassium, calcium and magnesium. Examples of suitable cationic salts include hydrobromic, hydroiodic, hydrochloric, perchloric, sulfuric, maleic, fumaric, malic, tartaric, citric, benzoic, mandelic, methanesulfonic, hydroethanesulfonic, benzenesulfonic, oxalic, palmitic, 2-naphthalenesulfonic, p-toluenesulfonic, cyclohexanesulfamic and saccharic.

More extensive sets of examples of acids and bases that may be used in the preparation of pharmaceutically acceptable salts include the following: Acids such as acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitric acid, pamoic acid, phosphoric acid, L-pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid and undecylenic acid; and bases such as ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amines, sodium hydroxide, triethanolamine, tromethamine and zinc hydroxide. See; *e*.*g*., S.M. Berge, et al., "Pharmacetuical Salts", J. Pharm. Sci., 1977, 66:1-19, which is incorporated herein by reference.

Examples of suitable esters include such esters where one or more carboxyl substituents is replaced with p-methoxybenzyloxycarbonyl, 2,4,6-trimethylbenzyloxycarbonyl, 9-anthryloxycarbonyl, CH₃SCH₂COO-, tetrahydrofur-2-yloxycarbonyl, tetrahydropyran-2-yloxycarbonyl, fur-2-yloxycarbonyl, benzoylmethoxycarbonyl, p-nitrobenzyloxycarbonyl, 4-pyridylmethoxycarbonyl, 2;2,2-trichloroethoxycarbonyl, 2,2,2-tribromoethoxycarbonyl, t-butyloxycarbonyl, t-amyloxycarbonyl, diphenylmethoxycarbonyl, triphenylmethoxycarbonyl, adamantyloxycarbonyl, 2-benzyloxyphenyloxycarbonyl, 4-methylthiophenyloxycarbonyl, or tetrahydropyran-2-yloxycarbonyl.

Compounds of the present invention may be used in the manufacture of medicaments to treat patients (humans and other mammals) with disorders involving the action of the LTA4H enzyme. In particular, compounds of the present invention may be used in the manufacture of medicaments to treat inflammation. More particularly compounds of the present invention may be used in the manufacture of medicaments to treat inflammatory conditions such as inflammatory bowel disease (IBD) (such as Crohn's disease and ulcerative colitis), chronic obstructive pulmonary disease (COPD), arthritis, psoriasis, asthma, cystic fibrosis, atherosclerosis, rheumatoid arthritis, and multiple sclerosis.

The present invention features pharmaceutical compositions containing such compounds and methods of using such compositions in the treatment or prevention of conditions that are mediated by LTA4H enzyme activity. Accordingly, the present invention also contemplates a pharmaceutical composition that comprises at least one compound according to this invention, preferably dispersed in a pharmaceutically acceptable carrier. The at least one compound according to this invention is present in such composition in an amount sufficient to inhibit LTA4H enzyme activity. More particularly, the at least one compound according to this invention is present in such composition in an anti-inflammatory amount.

Accordingly, a pharmaceutical composition that comprises an anti-inflammatory amount of at least one compound according to the present invention in a pharmaceutically acceptable carrier is also contemplated herein. The composition comprises a unit dosage of the at least one compound according to this invention. In preferred practice, the at least one compound according to the present invention that is comprised in the pharmaceutical composition is capable of inhibiting LTA4H enzyme activity in the amount at which that compound is present in the pharmaceutical composition, when that pharmaceutical composition is introduced as a unit dose into an appropriate patient or subject.

The terms "unit dose" and their grammatical equivalent forms are used herein to refer to physically discrete units suitable as unitary dosages for human patients and other animals, each unit containing a predetermined effective, pharmacologic amount of the active ingredient calculated to produce the desired pharmacological effect. The specifications for the novel unit dosage forms of this invention are determined by, and are directly dependent on, the characteristics of the active ingredient, and on the limitations inherent in the art of compounding such an active ingredient for therapeutic use in humans and other animals.

The pharmaceutical compositions can be prepared using conventional pharmaceutical excipients and compounding techniques. Examples of suitable unit dosage forms are tablets, capsules, pills, powder packets, granules, wafers, and the like, segregated multiples of any unit dosage form, as well as liquid solutions, and suspensions. Oral dosage forms may be elixirs, syrups, capsules tablets and the like. Examples of solid carriers include those materials usually employed in the manufacture of pills or tablets, such as lactose, starch, glucose, methylcellulose, magnesium stearate, dicalcium phosphate, mannitol and the like, thickeners such as tragacanth and methylcellulose USP, finely divided SiO₂, polyvinylpyrrolidone, magnesium stearate, and the like. Typical liquid oral excipients include ethanol, glycerol, water and the like. All excipients may be mixed as needed with inert diluents (for example, sodium and calcium carbonates, sodium and calcium phosphates, and lactose), disintegrants (for example, cornstarch and alginic acid), diluents, granulating agents, lubricants (for example, magnesium stearate, stearic acid, and talc), binders (for example, starch and gelatin), thickeners (for example, paraffin, waxes, and petrolatum), flavoring agents, coloring agents, preservatives, and the like by conventional techniques known to those of ordinary skill in the art of preparing dosage forms. Coatings can be present and include, for example, glyceryl monostearate and/or glyceryl distearate. Capsules for oral use include hard gelatin capsules in which the active ingredient is mixed with a solid diluent, and soft gelatin capsules, in which the active ingredient is mixed with water or oil, such as peanut oil, liquid paraffin, or olive oil.

Parenteral dosage forms may be prepared using water or another sterile carrier. For intramuscular, intraperitoneal, subcutaneous, and intravenous use, the compounds of the invention will generally be provided in sterile aqueous solutions or suspensions, buffered to an appropriate pH and isotonicity.
Suitable aqueous vehicles include Ringer's solution and isotonic sodium chloride. Aqueous suspensions may include suspending agents such as cellulose derivatives, sodium alginate, polyvinyl-pyrrolidone, and gum tragacanth, and a wetting agent, such as lecithin. Suitable preservatives for aqueous suspensions include ethyl and n-propyl p-hydroxybenzoate.

Physiologically acceptable carriers are well known in the art. Examples of liquid carriers are solutions in which compounds according to the present invention form solutions, emulsions, and dispersions. Compatible antioxidants, such as methlyparaben and propylparaben, can be present in solid and liquid compositions, as can sweeteners.

Pharmacetuical compositions according to the present invention may include suitable emulsifiers typically used in emulsion compositions. Such emulsifiers are described in standard publications such as H.P. Fiedler, 1989, Lexikon der Hilfsstoffe für Pharmazie, Kosmetic und agrenzende Gebiete, Cantor ed., Aulendorf, Germany, and in Handbook of Pharmacetutical Excipients, 1986, American Pharmaceutical Association, Washington, DC, and the Pharmaceutical Society of Great Britain, London, UK, which are incorporated herein by reference. Gelling agents may also be added to compositions according to this invention. Polyacrylic acid derivatives, such as carbomers, are examples of gelling agents, and more particularly, various types of carbopol, which are typically used in amounts from about 0.2% to about 2%. Suspensions may be prepared as a cream, an ointment, including a water-free ointment, a water-in-oil emulsion, an oil-in-water emulsion, an emulsion gel, or a gel.

It is anticipated that the compounds of the invention can be administered by oral or parenteral routes, including intravenous, intramuscular, intraperitoneal, subcutaneous, rectal, and topical administration, and inhalation. For oral administration, the compounds of the invention will generally be provided in the form of tablets, capsules, or as a solution or suspension.

"Therapeutically effective amount" or "effective amount" and grammatically related terms mean that amount of active compound or pharmaceutical agent that elicits the biological or medicinal response in a subject that is being sought by a researcher, veterinarian, medical doctor, or other clinician, which includes alleviation of the symptoms of the disease or disorder being treated. "Subject" or "patient" includes mammals such as human beings and animals (e.g., dogs, cats, horses, rats, rabbits, mice, non-human primates) in need of observation, experiment, treatment or prevention in connection with the relevant disease or condition. Preferably, the patient or subject is a human being.

Effective doses of the compounds of the present invention may be ascertained by conventional methods. The specific dosage level required for any particular patient will depend on a number of factors, including severity of the condition, the route of administration, and the weight of the patient.

In general, it is anticipated that the daily dose (whether administered as a single dose or as divided doses) will be in the range from about 0.01 mg to about 1000 mg per day, more usually from about 1 mg to about 500 mg per day, and most usually form about 10 mg to about 200 mg per day. Expressed as dosage per unit body weight, a typical dose will be expected to be between about 0.0001 mg/kg and about 15 mg/kg, especially between about 0.01 mg/kg and about 7 mg/kg, and most especially between about 0.15 mg/kg and 2.5 mg/kg.

Anticipated oral dose ranges include from about 0.01 to 500 mg/kg, daily, more preferably from about 0.05 to about 100 mg/kg, taken in 1-4 separate doses. Some compounds of the invention may be orally dosed in the range of about 0.05 to about 50 mg/kg daily, while others may be dosed at 0.05 to about 20 mg/kg daily. Infusion doses can range from about 1.0 to about 1.0 x 10⁴ µg/(kg.min) of inhibitor, admixed with a pharmaceutical carrier over a period ranging from several minutes to several days. For topical administration, compounds of the present invention may be mixed with a pharmaceutical carrier at a concentration from about 0.1 to about 10% of drug to vehicle.

Use of a compound of the invention in the manufacture of a medicament for treating inflammation in a patient exhibiting or susceptible to an inflammatory condition is also contemplated. Also a use for treating an LTA4H-mediated condition is also contemplated.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value. Whenever a yield is given as a percentage, such yield refers to a mass of the entity for which the yield is given with respect to the maximum amount of the same entity that could be obtained under the particular stoichiometric conditions. Concentrations that are given as percentages refer to mass ratios, unless indicated differently.

### EXAMPLES

In order to illustrate the invention, the following examples are included. These examples do not limit the invention. They are only meant to suggest a method of practicing the invention. Those skilled in the art may find other methods of practicing the invention that are obvious to them. However, those methods are deemed to be within the scope of this invention.

### General Experimental Procedures:

NMR spectra were obtained on either a Bruker model DPX400 (400 MHz) or DPX500 (500 MHz) spectrometer. The format of the ¹H NMR data below is: chemical shift in ppm down field of the tetramethylsilane reference (multiplicity, coupling constant *J* in Hz, integration).

Mass spectra were obtained on an Agilent series 1100 MSD using electrospray ionization (ESI) in either positive or negative mode as indicated. The "mass calculated" for a molecular formula is the monoisotopic mass of the compound.

Reversed-Phase HPLC retention times are reported in minutes, using the methods and conditions reported below.

| | |
|---|---|
| Instrument: | Gilson 215 |
| Solvent: | CH₃CN (0.05% trifluoroacetic acid, TFA)/H₂O (0.05% TFA) |
| Flow rate: | 25 mL/min |
| Gradient: | 0 min at 10% CH₃CN; 20 min linear ramp to 99% CH₃CN; |
| Column: | YMC-Pack ODS-A AA 12505-1.530WT SH-362-5 (S-5 um, 12 nM, 150x30 mm) |
| Temperature: | 25 °C |
| Wavelength: | Dual detection at 220 and 254 nM |

Flash column chromatography was accomplished using ISCO Foxy 200 or ISCO OPTIX 10X systems employing one of the following commercially available prepacked columns: Biotage 40S (SiO₂ 40 g), Biotage 40M (SiO₂ 90 g), Biotage 40L (SiO₂ 120 g), Biotage 65M (SiO₂ 300 g) or ISCO Redisep (SiO₂, 10 g, 12 g, 35 g, 40 g, or 120 g).

As indicated in the context of this written description, compounds of this invention that are referred to herein for which no explicit preparation description is provided can be prepared according to procedures analogous to those described herein in light of the knowledge of one of ordinary skill in the art and the teachings provided herein.

### EXAMPLE 250

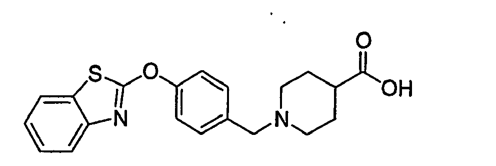

1-[4-(Benzothiazol-2-yloxy)-benzyl]-piperidine-4-carboxylic acid.
A. 1-(4-Benzyloxy-benzyl)-piperidine-4-carboxylic acid ethyl ester. A mixture of 4-benzyloxybenzyl chloride (15.2 g, 65.3 mmol), isonipecotic acid ethyl ester (15 mL, 97 mmol), and K₂CO₃ (13.5 g, 97.6 mmol) in CH₃CN (300 mL) was stirred at reflux for 20 h. The reaction mixture was cooled to room temperature and filtered. The solvent was removed under reduced pressure to yield a clear golden oil. This material was diluted with iPrOH (100 mL), and the mixture was filtered. The solid was air dried to yield a white solid (19.7 g, 85% yield). TLC (SiO₂, 15% acetone/CH₂Cl₂): R_{f} = 0.32. MS (ESI): mass calculated for C₂₂H₂₇NO₃, 353.2; m/z found, 354.3 [M+H]⁺. ¹H NMR (400 MHz, DMSO-*d₆*): 7.44 (d, *J* = 7.1, 2H), 7.39 (t, *J* = 7.1, 2H), 7.33 (d, *J* = 7.2, 1H), 7.18 (d, *J* = 8.2, 2H), 6.94 (2H, *J* = 8.6, 2H), 5.08 (s, 2H), 4.04 (q, *J* = 7.09, 2H), 2.72 (d, *J* = 11.5, 2H), 2.32-2.18 (m, 1H), 1.94 (t, *J* = 11.6, 2H), 1.76 (d, *J* = 10.2, 2H), 1.59-1.48 (m, 2H), 1.17 (t, *J* = 7.1, 3H).
B. 1-(4-Hydroxy-benzyl)-piperidine-4-carboxylic acid ethyl ester. 1-(4-Benzyloxy-benzyl)-piperidine-4-carboxylic acid ethyl ester (10.0 g, 28.3 mmol) was dissolved in 1:1 ethanol/ethyl acetate (150 mL). To this solution was added Pd on carbon (10 wt %, 503 mg) as a suspension in ethanol (5.0 mL). The resulting suspension was placed on a Parr hydrogenator at 40 psi of H₂ and shaken overnight. The reaction mixture was filtered through a pad of diatomaceous earth, and the filtrate was concentrated under reduced pressure to give a clear golden oil. The oil was purified on SiO₂ (90 g; 50% acetone/CH₂Cl₂) to give a white solid (2.0 g, 27% yield). TLC (SiO₂, 50% acetone/CH₂Cl₂): R_{f} = 0.32. MS (ESI): mass calculated for C₁₅H₂₁NO₃, 263.2; m/z found, 264.2 [M+H]⁺. ¹H NMR (400 MHz, DMSO-*d*₆): 9.25 (s, 1H), 7.05 (d, *J* = 8.4, 2H), 6.68 (d, *J* = 8.4, 2H), 4.04 (q, *J* = 7.1, 2H), 3.34 (s, 2H), 2.71 (d, *J* = 11.5, 2H), 2.32-2.18 (m, 1H), 1.92 (t, *J* = 11.6, 2H), 1.76 (d, *J* = 10.2, 2H), 1.59- 1.48 (m, 2H), 1.17 (t, *J*= 7.1, 3H).
C. 1-[4-(Benzothiazol-2-yloxy)-benzyl]-piperidine-4-carboxylic acid ethyl ester. To a stirring solution of 1-(4-hydroxy-benzyl)-piperidine-4-carboxylic acid ethyl ester (508 mg, 1.93 mmol) in CH₃CN (15 mL), was added K₂CO₃ (564 mg, 4.1 mmol) and 2-chlorobenzothiazole (0.50 mL, 4.0 mmol). The suspension was heated to 80 °C and stirred overnight. The reaction mixture was allowed to cool to room temperature and then filtered through diatomaceous earth. The filtrate was concentrated under reduced pressure, and the residue was purified on SiO₂ (12 g; 0-15% acetone/CH₂Cl₂) to give a clear and colorless tacky oil (717 mg, 94% yield). TLC (SiO₂, 15% acetone/CH₂Cl₂): R_{f} = 0.5. MS (ESI): mass calculated for C₂₂H₂₄N₂O₃S, 396.2; m/z found, 397.3 [M+H]⁺. ¹H NMR (400 MHz, DMSO-*d₆*): 7.92 (d, *J* = 8.0, 1H), 7.68 (d, *J* = 8.0, 1H), 7.48-7.33 (m, 5H), 7.33 (t, *J* = 7.1, 1H), 4.06 (q, *J* = 7.1, 2H), 3.49 (s, 2H), 2.76 (d, *J* = 11.5, 2H), 2.34-2.22 (m, 1H), 2.02 (t, *J* = 11.6, 2H), 1.80 (d, *J* = 10.2, 2H), 1.64- 1.54 (m, 2H), 1.18 (t, *J*= 7.1, 3H).
D. 1-[4-(Benzothiazol-2-yloxy)-benzyl]-piperidine-4-carboxylic acid. To a stirring solution of 1-[4-(benzothiazol-2-yloxy)-benzyl]-piperidine-4-carboxylic acid ethyl ester (663 mg, 1.7 mmol) in 25% *i*PrOH/H₂O (20 mL) was added potassium hydroxide (206 mg, 3.1 mmol). The reaction mixture was stirred at room temperature for 20 h, and the solution was treated to pH 5.5 with 1 M HCl. The resulting solution was extracted with 10% *i*PrOH/CHCl₃ (3 x 50 mL). The combined extracts were dried (MgSO₄), filtered, and concentrated under reduced pressure to yield a white solid (561 mg, 91 % yield). MS (ESI): mass calculated for C₂₀H₂₀N₂O₃S, 368.1; m/z found, 369.3 [M+H]⁺. ¹H NMR (400 MHz, DMSO-*d*₆): 7.92 (d, *J* = 7.6, 1H), 7.69 (d, *J* = 7.6, 1H), 7.48-7.34 (m, 5H), 7.33 (t, *J* = 7.1, 1H), 3.49 (s, 2H), 2.76 (d, *J* = 11.4, 2H), 2.22-2.11 (m, 1H), 2.02 (t, *J* = 11.2, 2H); 1.80 (d, *J* = 13.2, 2H), 1.62- 1.48 (m, 2H), 1.18 (t, *J* = 7.1, 3H).

### EXAMPLE 251

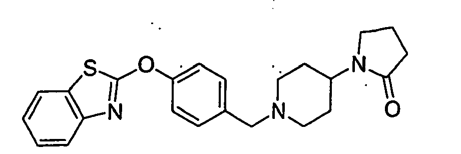

1-{1-[4-(Benzothiazol-2-yloxy)-benzyl]-piperidin-4-yl}-pyrrolidin-2-one.
A. 4-(Benzothiazol-2-yloxy)-benzaldehyde. To a mixture of 4-hydroxybenzaldehyde (1 g, 8.2 mmol) and 2-chlorobenzothiazole (2.03 mL, 16.4 mmol) in CH₃CN (100 mL) was added Cs₂CO₃ (5.5 g, 17.2 mmol). The reaction mixture was stirred at 60 °C for 24 h. The resulting mixture was cooled to room temperature, filtered through diatomaceous earth and concentrated under reduced pressure to yield the crude product as an orange oil. The oil was triturated with hexanes/CH₂Cl₂ (100 mL) and the solvent layer decanted and concentrated under reduced pressure to yield an orange oil which was further purified on SiO₂ (120 g; 0-50% ethyl acetate/hexanes) to give a white solid (853 mg, 41% yield). ¹H NMR (400 MHz, CDCl₃): 10.1 (s, 1H), 7.97-7.78 (m, 2H), 7.78-7.70 (m, 2H), 7.60-7.50 (m, 2H), 7.48-7.38 (m, 1H), 7.36-7.30 (m, 1H).
B. 1-{1-[4-(Benzothiazol-2-yloxy)-benzyl]-piperidin-4-yl}-pyrrolidin-2-one. A mixture of 4-(benzothiazol-2-yloxy)-benzaldehyde (500 mg, 1.9 mmol), 1-piperidin-4-yl-pyrrolidin-2-one hydrochloride (440 mg, 2.2 mmol), Et₃N (300 µL, 2.2 mmol) and molecular sieves (500 mg, crushed, 4Å) in ClCH₂CH₂Cl (10 mL) was stirred at room temperature for 1 h. To the resulting mixture was added NaBH(OAc)₃ (830 mg, 3.92 mmol). The mixture was stirred at room temperature for 24 h, filtered through diatomaceous earth, rinsed with CH₂Cl₂ (50 mL) and concentrated under reduced pressure to yield the crude product as a yellow oil. The crude product was purified on SiO₂ (40 g; 0-100% acetone/CH₂Cl₂) to give a clear oil which crystallized on standing (287 mg, 36% yield). MS (ESI): mass calculated for C₂₃H₂₅N₃O₂S, 407.5; m/z found, 408.3 [M+H]⁺. ¹H NMR (400 MHz, CDCl₃): 7.76 (d, *J* = 8.0, 1H), 7.69 (d, *J* = 8.0, 1H), 7.41-7.30 (m, 3H), 7.35-7.27 (m, 3H), 4.46-3.98 (m, 1H), 3.71 (s, 2H), 3.36 (t, *J* = 6.9, 2H), 2.97 (d, *J* = 11.7, 2H), 2.40 (t, *J* = 8.1, 2H), 2.17-1.97 (m, 4H), 1.81-1.62 (m, 4H).

### EXAMPLE 252

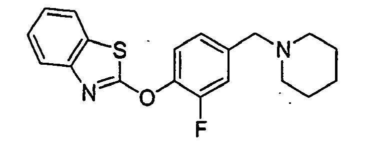

2-(2-Fluoro-4-piperidin-1-ylmethyl-phenoxy)-benzothiazole.
A. (3-Fluoro-4-hydroxy-phenyl)-piperidin-1-yl-methanone. A solution of 3-fluoro-4-hydroxybenzoic acid (5.0 g, 32 mmol), piperidine (5 mL, 51 mmol), and EDCI (9.3 g, 49 mmol) in CH₂Cl₂ (100 mL) was stirred at room temperature for 20 h. The reaction mixture was added to CH₂Cl₂ (200 mL) and was washed with 1 M HCl (2 x 100 mL). The organic layers were combined, dried (MgSO₄), and concentrated under reduced pressure to yield a clear golden oil. The oil was purified on SiO₂ (120 g; 0-10% acetone/CH₂Cl₂) to give a white solid (2.4 g, 34% yield). TLC (SiO₂, 15% acetone/CH₂Cl₂): R_{f} = 0.35. MS (ESI): mass calculated for C₁₂H₁₄FNO₂, 223.1; m/z found, 224.3 [M+H]⁺. ¹H NMR (400 MHz, DMSO-*d₆*): 10.26 (s, 1H), 7.18 (d, *J* = 11.6, 1H), 7.00 (d, *J* = 8.3, 1H), 6.98 (t, *J* = 8.5, 1H), 3.42 (br s, 4H), 1.65-1.45 (m, 6H).
B. 2-Fluoro-4-piperidin-1-ylmethyl-phenol. A solution of lithium aluminum hydride (1.9 g, 50 mmol) in THF (40 mL) was stirred at 5 °C. To the mixture was added (3-fluoro-4-hydroxy-phenyl)-piperidin-1-yl-methanone (2.3 g, 10.4 mmol) in THF (10 mL) over 15 min and then the mixture was heated to 60 °C. After 20 h the mixture was cooled to 5 °C and saturated NH₄Cl (200 mL) was added followed by CH₂Cl₂ (200 mL). The organic layer was separated, dried (MgSO₄), and concentrated under reduced pressure to yield a white solid (812 mg, 37% yield). TLC (SiO₂, acetone): R_{f} = 0.22. MS (ESI): mass calculated for C₁₂H₁₆FNO, 209.1; m/z found, 210.3 [M+H]⁺. ¹H NMR (400 MHz, DMSO-*d*₆): 6.58 (d, *J* = 13.4, 1H), 6.48 (d, *J* = 8.2, 1H), 6.40 (t, *J* = 9.9, 1H), 3.14 (s, 2H), 2.24 (br s, 4H), 1.54-1.30 (m, 6H).
C. 2-(2-Fluoro-4-piperidin-1-ylmethyl-phenoxy)-benzothiazole. To a stirring solution of 2-fluoro-4-piperidin-1-ylmethyl-phenol (152 mg, 0.73 mmol) in CH₃CN (10 mL), was added K₂CO₃ (201 mg, 1.5 mmol) and 2-chlorobenzothiazole (0.14 mL, 1.1 mmol). The suspension was heated to 80 °C and stirred overnight. The reaction mixture was allowed to cool to room temperature and then filtered through diatomaceous earth. The filtrate was concentrated under reduced pressure, and the residue was purified on SiO₂ (12 g; 0-50% acetone/CH₂Cl₂) to give a clear golden oil (142 mg, 57% yield). TLC (SiO₂, 50% acetone/CH₂Cl₂): R_{f} = 0.44. MS (ESI): mass calculated for C₁₉H₁₉FN₂OS, 342.1; m/z found, 343.3 [M+H]⁺. ¹H NMR (400 MHz, DMSO-*d*₆): 7.95 (d, *J* = 7.9, 1H), 7.69 (d, *J* = 7.5, 1H), 7.56 (t, *J* = 8.2, 1H), 7.47-7.32 (m, 3H), 7.44 (d, *J* = 15.4, 1H), 3.48 (s, 2H), 2.36 (s, 4H), 1.63-1.37 (m, 6H).

### EXAMPLE 253

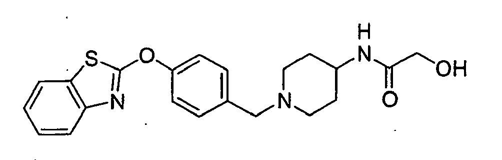

N-{1-[4-(Benzothiazol-2-yloxy)-benzyl]-piperidin-4-yl}-2-hydroxy-acetamide.
A. {1-[4-(Benzothiazol-2-yloxy)-benzyl]-piperidin-4-yl}-carbamic acid tert-butyl ester. A mixture of 4-(benzothiazol-2-yloxy)-benzaldehyde (EXAMPLE 251, step A, 500 mg, 1.9 mmol), piperidin-4-yl-carbamic acid tert-butyl ester (785 mg, 3.9 mmol) and molecular sieves (500 mg, crushed, 4Å) in ClCH₂CH₂Cl (10 mL) was stirred at room temperature for 40 min. To the resulting reaction mixture was added NaBH(OAc)₃ portion wise over 1.5 h (4 x 207 mg, 3.9 mmol). The resulting mixture was stirred at room temperature for 24 h, filtered through diatomaceous earth and rinsed with CH₂Cl₂ (50 mL). The filtrate was washed with sat. aq. NaHCO₃ (1 x 25 mL), dried (Na₂SO₄) and concentrated under reduced pressure to yield the crude product as a light yellow oil. The crude product was purified on SiO₂ (40 g; 0-5% 2 M NH₃ in CH₃OH/CH₂Cl₂) to give a white foam (504 mg, 59% yield). MS (ESI): mass calculated for C₂₄H₂₉N₃O₃S, 439.6; m/z found, 440.4 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.32-7.24 (m, 3H), 4.44 (br s, 1H), 3.50 (s, 2H), 2.82-2.76 (m, 2H), 2.16-2.06 (m, 2H), 1.96-1.88 (m, 2H), 1.45 (s, 9H), 1.48-1.38 (m, 2H).
B. 1-[4-(Benzothiazol-2-yloxy)-benzyl]-piperidin-4-ylamine. To a solution of {1-[4-(benzothiazol-2-yloxy)-benzyl]-piperidin-4-yl}-carbamic acid tert-butyl ester (200 mg, 0.45 mmol) in CH₂Cl₂ (2 mL) at 0 °C was added 4 N HCl in dioxane (1.8 mL, 7.2 mmol) dropwise. The resulting reaction mixture was stirred at room temperature for 2 h. The desired product was isolated by filtration and was rinsed with Et₂O (50 mL) to yield a white powder (187 mg, 100% yield). MS (ESI): mass calculated for C₁₉H₂₁N₃OS, 339.5; m/z found, 340.4 [M+H]⁺. ¹H NMR (400 MHz, CDCl₃): 7.68-7.64 (m, 1H), 7.58-7.52 (m, 2H), 7.48-7.44 (m, 1H), 7.40-7.35 (m, 2H), 7.30-7.24 (m, 1H), 7.20-7.14 (m, 1H), 4.25 (s, 2H), 3.52-3.46 (m, 2H), 3.36-3.28 (m, 1H), 3.08-2.99 (m, 2H), 2.16-2.08 (m, 2H), 1.92-1.80 (m, 2H).
C. Acetic acid {1-[4-(benzothiazol-2-yloxy)-benzyl]-piperidin-4-ylcarbamoyl}-methyl ester. To a solution of {1-[4-(benzothiazol-2-yloxy)-benzyl]-piperidin-4-ylamine dihydrochloride (413 mg, 1.0 mmol) in CH₂Cl₂ (20 mL) at room temperature was added TEA (0.70 mL, 5.0 mmol), followed by acetoxyacetyl chloride (0.16 mL, 1.5 mmol). The resulting mixture was stirred at room temperature overnight. The reaction mixture was dissolved in CH₂Cl₂ (100 mL), washed with sat. aq. NaHCO₃ (1 x 25 mL), dried (Na₂SO₄) and concentrated under reduced pressure to yield the crude product as an off-white solid. The crude product was purified on SiO₂ (40 g; 0-10% CH₃OH/CH₂Cl₂) to give a white solid (410 mg, 93% yield). MS (ESI): mass calculated for C₂₃H₂₅N₃O₄S, 439.2; m/z found, 440.4 [M+H]⁺. ¹H NMR (400 MHz, CDCl₃): 7.72 (d, *J* = 7.8, 1H), 7.65 (d, *J* = 8.1, 1H), 7.40-7.35 (m, 3H), 7.32-7.23 (m, 3H), 6.11 (d, *J* = 8.3, 1H), 4.53, (s, 2H), 3.93-3.82 (m, 1H), 3.50 (s, 2H), 2.83 (d, *J*= 11.9, 2H), 2.15 (s, 3H), 2.14 (t, *J* = 11.9, 2H), 1.93 (d, *J* = 12.1, 2H), 1.56-1.45 (m, 2H).
D. N-{1-[4-(Benzothiazol-2-yloxy)-benzyl]-piperidin-4-yl}-2-hydroxy-acetamide. To a solution of acetic acid {1-[4-(benzothiazol-2-yloxy)-benzyl]-piperidin-4-ylcarbamoyl}-methyl ester (368 mg, 0.84 mmol) in THF (30 mL), CH₃OH (10 mL) and H₂O (10 mL) was added lithium hydroxide (80.2 mg, 3.34 mmol). The resulting mixture was stirred at room temperature overnight. The mixture was extracted with CH₂Cl₂ (30 mL x 3). The combined organic phases were concentrated under reduced pressure to yield the crude product as an off-white solid. The crude product was purified on SiO₂ (40 g; 0-10% CH₃OH/CH₂Cl₂) to give a white solid (297 mg, 82% yield). MS (ESI): mass calculated for C₂₁H₂₃N₃O₃S, 397.2; m/z found, 398.3 [M+H]⁺. ¹H NMR (400 MHz, CDCl₃): 7.72 (d, *J* = 7.8, 1H), 7.65 (d, *J* = 8.1, 1H), 7.40-7.35 (m, 3H), 7.31-7.23 (m, 3H), 6.83 (d, *J* = 8.1, 1H), 5.33 (br s, 1H), 3.99 (s, 2H), 3.87-3.75 (m, 1H), 3.50 (s, 2H), 2.85 (d, *J* = 11.4, 2H), 2.14 (t, *J* = 10.9, 2H), 1.92 (d, *J* = 12.6, 2H), 1.56-1.43 (m, 2H).

### EXAMPLE 254

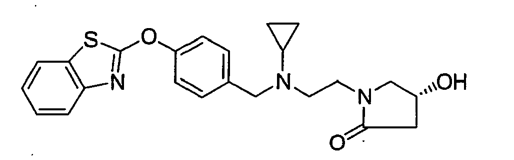

1-(2-{[4-(Benzothiazol-2-yloxy)-benzyl]-cyclopropyl-amino}-ethyl)-4-hydroxy-pyrrolidin-2-one.
A. [4-(Benzothiazol-2-yloxy)-benzyl]-cyclopropyl-amine. A mixture of 4-(benzothiazol-2-yloxy)-benzaldehyde (5.0 g, 19.6 mmol), cyclopropylamine (3.35 g, 58.7 mmol) in ClCH₂CH₂Cl (80 mL) was stirred at room temperature for 40 min. To the resulting reaction mixture was added NaBH(OAc)₃ portion wise over 1.5 h (4 x 2.1 g, 39.2 mmol). The resulting mixture was stirred at room temperature for 24 h, filtered through diatomaceous earth and rinsed with CH₂Cl₂ (500 mL). The filtrate was washed with sat. aq. NaHCO₃ (1 x 250 mL), dried (Na₂SO₄) and concentrated under reduced pressure to yield the crude product as a pale yellow oil. The crude product was purified on SiO₂ (330 g; 0-5% CH₃OH/CH₂Cl₂) to give a white solid (3.95 g, 68% yield). MS (ESI): mass calculated for C₁₇H₁₆N₂OS, 296.1; m/z found, 297.3 [M+H]⁺. ¹H NMR (400 MHz, CDCl₃): 7.72 (d, *J* = 8.1, 1H), 7.63 (d, *J* = 8.1, 1H), 7.40-7.33 (m, 3H), 7.32-7.27 (m, 2H), 7.24 (t, *J* = 8.1, 1H), 3.85 (s, 2H), 2.19-2.12 (m, 1H), 1.86 (br s, 1H), 0.48-0.35 (m, 4H).
B. (2-{[4-(Benzothiazol-2-yloxy)-benzyl]-cyclopropyl-amino}-ethyl)-carbamic acid tert-butyl ester. To a solution of [4-(benzothiazol-2-yloxy)-benzyl]-cyclopropyl-amine (2.96 g, 10 mmol) in CH₃CN (40 mL) at room temperature was added *N,N-*diisopropylethylamine (3.48 mL, 20 mmol), followed by (2-bromo-ethyl)-carbamic acid tert-butyl ester (3.36 g, 15 mmol). The resulting mixture was heated at 60 °C overnight. The mixture was cooled and dissolved in CH₂Cl₂ (200 mL), washed with sat. aq. NaHCO₃ (1 x 25 mL) and H₂O (2 x 25 mL), dried (Na₂SO₄) and concentrated under reduced pressure to yield the crude product as a pale yellow solid. The crude product was purified on SiO₂ (120 g; 0-50% ethyl acetate/hexanes) to give a white solid (3.44 g, 78% yield). MS (ESI): mass calculated for C₂₄H₂₉N₃O₃S, 439.2; m/z found, 440.4 [M+H]⁺. ¹HNMR (400 MHz, CDCl₃): 7.73 (d, *J* = 8.1, 1H), 7.64 (d, *J* = 8.1, 1H), 7.39-7.22 (m, 6H), 4.75 (br s, 1H), 3.74 (s, 2H), 3.25 (dd, *J* = 5.8, 6.1, 2H), 2.65 (t, *J* = 6.3, 2H), 1.82-1.75 (m, 1H), 1.43 (s, 9H), 0.54-0.48 (m, 2H), 0.43-0.37 (m, 2H).
C. N1-[4-(Benzothiazol-2-yloxy)-benzyl]-N1-cyclopropyl-ethane-1,2-diamine. To a solution of (2-{[4-(benzothiazol-2-yloxy)-benzyl]-cyclopropyl-amino}-ethyl)-carbamic acid tert-butyl ester in CH₂Cl₂ (16 mL) at 0 °C was added trifluoroacetic acid in dioxane (4 mL) dropwise. The resulting reaction mixture was stirred at room temperature for 2 h and concentrated under reduced pressure to yield the crude product as a pale yellow oil. The oil was dissolved in CH₂Cl₂ (100 mL), washed with sat. aq. NaHCO₃ (1 x 25 mL), dried (Na₂SO₄) and concentrated under reduced pressure to yield the product as a clear oil. MS (ESI): mass calculated for C₁₉H₂₁N₃OS, 339.1; m/z found, 340.4 [M+H]⁺. ¹H NMR (400 MHz, CDCl₃): 7.71 (d, *J* = 8.1, 1H), 7.61 (d, *J* = 8.1, 1H), 7.37-7.18 (m, 6H), 3.75 (s, 2H), 2.76 (t, *J* = 6.3, 2H), 2.60 (t, *J* = 6.3, 2H), 1.98 (s, 2H), 1.79-1.73 (m, 1H), 0.51-0.45 (m, 2H), 0.42-0.37 (m, 2H).
D. 1-(2-{[4-(Benzothiazol-2-yloxy)-benzyl]-cyclopropyl-amino}-ethyl)-4-hydroxy-pyrrolidin-2-one. To a solution of N1-[4-(benzothiazol-2-yloxy)-benzyl]-N1-cyclopropyl-ethane-1,2-diamine (1.5 g, 4.4 mmol) in CH₃CN (18 mL) at room temperature was added *N,N*-diisopropylethylamine (1.15 mL, 6.63 mmol), followed by 4-(R)-bromo-3-hydroxy-butyric acid ethyl ester (1.11 g, 5.3 mmol). The resulting mixture was heated at 60 °C overnight. The mixture was cooled and dissolved in CH₂Cl₂ (100 mL), washed with sat. aq. NaHCO₃ (1 x 10 mL) and H₂O (2 x 10 mL), dried (Na₂SO₄) and concentrated under reduced pressure to yield the crude product as a light brown oil. The crude product was purified on reverse phase HPLC (0-99%, 0.05 % TFA in H₂O/CH₃CN) to give a clear oil (435 mg, 18.3% yield). MS (ESI): mass calculated for C₂₃H₂₅N₃O₃S, 423.2; m/z found, 424.3 [M+H]⁺. ¹H NMR (400 MHz, CD₃OD): 7.64 (d, *J* = 8.1, 1H), 7.58-7.54 (m, 2H), 7.49 (d, *J* = 8.1, 1H), 7.37-7.33 (m, 2H), 7.26 (t, *J* = 7.6, 1H), 7.16 (t, *J* = 7.6, 1H), 4.48 (dd, *J* = 8.8, 12.9, 2H), 4.32 (t, *J* = 5.8, 1H), 3.84-3.75 (m, 1H), 3.64-3.56 (m, 2H), 3.38 (t, *J* = 6.3, 2H), 3.24 (t, *J* = 10.9, 1H), 2.73-2.66 (m, 1H), 2.59 (dd, *J* = 6.3, 11.1, 1H), 2.15 (d, *J* = 17.2, 1H), 0.79 (d, *J* = 6.6, 4H).

### EXAMPLE 255

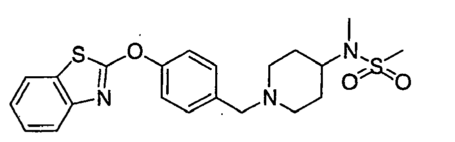

N-{1-[4-(Benzothiazol-2-yloxy)-benzyl]-piperidin-4-yl}-N-methyl-methanesulfonamide.
A. {1-[4-(Benzothiazol-2-yloxy)-benzyl]-piperidin-4-yl}-methyl-carbamic acid tert-butyl ester. A mixture of 4-(benzothiazol-2-yloxy)-benzaldehyde (4.4 g, 17.2 mmol), methyl-piperidin-4-yl-carbamic acid tert-butyl ester (4.06 g, 18.9 mmol) in ClCH₂CH₂Cl (172 mL) was stirred at room temperature for 40 min. To the resulting reaction mixture was added NaBH(OAc)₃ portion wise over 1.5 h (4 x 1.82 g, 34.4 mmol). The resulting mixture was stirred at room temperature for 24 h, filtered through diatomaceous earth and rinsed with CH₂Cl₂ (300 mL). The filtrate was washed with sat. aq. NaHCO₃ (1 x 50 mL), dried (Na₂SO₄) and concentrated under reduced pressure to yield the crude product as a pale yellow oil. The crude product was purified on SiO₂ (330 g; 0-100% ethyl acetate/hexanes) to give a light yellow foam (3.75 g, 48% yield). MS (ESI): mass calculated for C₂₅H₃₁N₃O₃S, 453.2; m/z found, 454.5 [M+H]⁺. ¹H NMR (400 MHz, CDCl₃): 7.73 (d, *J* = 8.1, 1H), 7.66 (d, *J* = 8.1, 1H), 7.41-7.35 (m, 3H), 7.33-7.23 (m, 3H), 4.13-3.94 (m, 1H), 3.53 (s, 2H), 2.93 (d, *J* = 11.6, 2H), 2.74 (s, 3H), 2.08 (t, *J* = 11.6, 2H), 1.81-1.69 (m, 2H), 1.65-1.57 (m, 2H), 1.46 (s, 9H).
B. {1-[4-(Benzothiazol-2-yloxy)-benzyl]-piperidin-4-yl}-methyl-amine. To a solution of {1-[4-(benzothiazol-2-yloxy)-benzyl]-piperidin-4-yl}-methyl-carbamic acid tert-butyl ester (3.7 g, 8.2 mmol) in CH₂Cl₂ (41 mL) at 0 °C was added 4 N HCl in dioxane (8.2 mL, 32.6 mmol) dropwise. The resulting mixture was stirred at room temperature for 2 h. The desired product was isolated by filtration and was washed with Et₂O (150 mL) to yield a white powder (3.38 g, 97% yield). MS (ESI): mass calculated for C₂₀H₂₃N₃OS, 353.2; m/z found, 354.4 [M+H]⁺. ¹H NMR (400 MHz, CDCl₃): 8.92 (br s, 1H), 7.72 (d, *J*= 8.1, 1H), 7.63 (d, *J* = 8.1, 1H), 7.39-7.33 (m, 3H), 7.30-7.21 (m, 3H), 3.49 (s, 2H), 2.98-2.86 (m, 3H), 2.63 (s, 3H), 2.08-1.98 (m, 4H), 1.84-1.71 (m, 2H).
C. N-{1-[4-(Benzothiazol-2-yloxy)-benzyl]-piperidin-4-yl}-N-methyl-methanesulfonamide. To a solution of {1-[4-(benzothiazol-2-yloxy)-benzyl]-piperidin-4-yl}-methyl-amine dihydrochloride (354 mg, 1.0 mmol) in CH₂Cl₂ (20 mL) at room temperature was added TEA (0.70 mL, 5.0 mmol), followed by methanesulfonyl chloride (0.12 mL, 1.5 mmol). The resulting mixture was stirred at room temperature overnight. The mixture was dissolved in CH₂Cl₂ (100 mL), washed with sat. aq. NaHCO₃ (1 x 25 mL), dried (Na₂SO₄) and concentrated under reduced pressure to yield the crude product as a pale yellow solid. The crude product was purified on SiO₂ (40 g; 0-10% CH₃OH/CH₂Cl₂) to give a white solid (378 mg, 88% yield). MS (ESI): mass calculated for C₂₁H₂₅N₃O₃S₂, 431.1; m/z found, 432.4 [M+H]⁺. ¹H NMR (400 MHz, CDCl₃): 7.73 (d, *J* = 8.1, 1H), 7.66 (d, *J* = 8.1, 1H), 7.41-7.35 (m, 3H), 7.33-7.23 (m, 3H), 3.80-3.70 (m, 1H), 3.50 (s, 2H), 2.96 (d, *J* = 11.6, 2H), 2.82 (s, 3H), 2.80 (s, 3H), 2.08 (t, *J* = 11.6, 2H), 1.89-1.77 (m, 2H), 1.70-1.60 (m, 2H).

### EXAMPLE 256

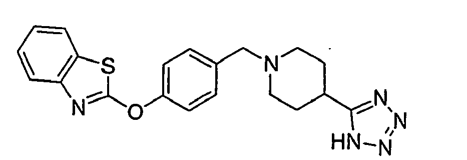

2-{4-[4-(1H-Tetrazol-5-yl)-piperidin-1-ylmethyl]-phenoxy}-benzothiazole.

A solution of 4-(benzothiazol-2-yloxy)-benzaldehyde (EXAMPLE 251 step A, 620 mg, 2.4 mmol), 4-(1*H*-tetrazol-5-yl)-piperidine hydrochloride (565 mg, 3.0 mmol), and Et₃N (0.43 mL, 3.1 mmol) in 30% THF/CH₂Cl₂ (35 mL) was stirred at room temperature for 30 min. To the stirring mixture was added Na(AcO)₃BH (787 mg, 3.7 mmol). After 20 h the reaction mixture was added to 10% *i*PrOH/H₂O (50 mL) and the organic layer was separated and dried (MgSO₄). The solvent was removed under reduced pressure to yield a clear yellow tacky oil. This material was diluted with ethanol (10 mL), heated to 80 °C, and filtered while hot. To the filtrate was added Et₂O (10 mL) and the flask was cooled on ice. A solid formed and was filtered to yield a white solid (48 mg, 5% yield). MS (ESI): mass calculated for C₂₀H₂₀N₆OS, 392.1; m/z found, 393.4 [M+H]⁺. ¹H NMR (400 MHz, DMSO-*d*₆): 7.95 (d, *J* = 7.9, 1H), 7.70 (d, *J* = 7.9, 1H), 7.53-7.35 (m, 5H), 7.33 (t, *J* = 7.9, 1H), 3.59 (s, 2H), 3.09-3.00 (m, 1H), 2.91 (d, *J* = 10.8, 2H), 2.22 (t, *J* = 9.8, 2H), 1.85-1.72 (m, 2H).

### EXAMPLE 257

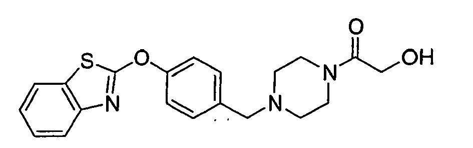

1-{4-[4-(Benzothiazol-2-yloxy)-benzyl]-piperazin-1-yl}-2-hydroxy-ethanone.
A. 4-[4-(Benzothiazol-2-yloxy)-benzyl]-piperazine-1-carboxylic acid tert-butyl ester. A mixture of 4-(benzothiazol-2-yloxy)-benzaldehyde (2.5 g, 9.8 mmol), piperazine-1-carboxylic acid tert-butyl ester-2-one (3.7 g, 19.6 mmol) and molecular sieves (2.5 g, crushed, 4Å) in ClCH₂CH₂Cl (25 mL) was stirred at room temperature for 40 min. To the resulting reaction mixture was added NaBH(OAc)₃ portion wise over 1.5 h (4 x 504 mg, 19.6 mmol). The resulting mixture was stirred at room temperature for 24 h, filtered through diatomaceous earth and rinsed with CH₂Cl₂ (200 mL). The filtrate was washed with sat. aq. NaHCO₃ (1 x 50 mL), dried (Na₂SO₄) and concentrated under reduced pressure to yield the crude product as a yellow semi-solid. The crude product was purified on SiO₂ (120 g; 0-100% acetone/CH₂Cl₂) to give an off-white solid (1.72 g, 42% yield). MS (ESI): mass calculated for C₂₃H₂₇N₃O₃S, 425.5; m/z found, 426.4 [M+H]⁺. ¹H NMR (400 MHz, CDCl₃): 7.74 (d, *J* = 8.2, 1H), 7.67 (d, *J* = 8.2, 1H), 7.42-7.37 (m, 3H), 7.4-7.25 (m, 3H), 3.53 (s, 2H), 3.45 (br t, *J* = 4.9, 4H), 2.41 (br t, *J* = 4.5, 4H), 1.46 (s, 9H).
B. 2-(4-Piperazin-1-ylmethyl-phenoxy)-benzothiazole. To a solution of 4-[4-(benzothiazol-2-yloxy)-benzyl]-piperazine-1-carboxylic acid tert-butyl ester (1.7 g, 4.0 mmol) in CH₂Cl₂ (20 mL) at 0 °C was added 4 N HCl in dioxane (5 mL, 20 mmol) dropwise. The resulting mixture was stirred at room temperature for 2 h and concentrated under reduced pressure to yield the crude product as a white solid. The crude product was triturated with Et₂O (50 mL) and isolated by filtration to yield the desired product as a white powder (1.37 g, 87% yield). MS (ESI): mass calculated for C₁₈H₁₉N₃OS, 325.4; m/z found, 326.3 [M+H]⁺. ¹H NMR (400 MHz, CD₃OD): 7.81 (d, *J* = 8.0, 1H), 7.77-7.72 (m, 2H), 7.62 (d, *J* = 8.2, 1H), 7.57-7.53 (m, 2H), 7.45-7.40 (m, 1H), 7.35-7.31 (m, 1H), 4.45 (s, 2H), 3.62 (br s, 8H).
C. 1-{4-[4-(Benzothiazol-2-yloxy)-benzyl]-piperazin-1-yl}-2-hydroxy-ethanone. To a mixture of glycolic acid (47 mg, 0.62 mmol) and HOBT (1.25 mL, 0.62 mmol, 0.5 M in DMF) in CH₂Cl₂ (25 mL) was added 2-(4-piperazin-1-ylmethyl-phenoxy)-benzothiazole (150 mg, 0.42 mmol) followed by EDCI (150 mg, 0.79 mmol). The resulting mixture was stirred at room temperature for 24 h, diluted with CH₂Cl₂ (50 mL) and washed with sat. aq. NaHCO₃ (1 x 20 mL). The organic layer was dried (Na₂SO₄) and concentrated under reduced pressure to yield the crude product as apale yellow oil. The crude product was purified on SiO₂ (40 g; 0-3% 2 M NH₃ in CH₃OH/CH₂Cl₂) to give a white foam (93 mg, 59% yield). MS (ESI): mass calculated for C₂₀H₂₁N₃O₃S, 383.5; m/z found, 384.4 [M+H]⁺. ¹H NMR (400 MHz, CDCl₃): 7.74 (d, *J* = 8.0, 1H), 7.68 (d, *J* = 8.0, 1H), 7.45-7.25 (m, 6H), 4.16 (d, *J* = 4.1, 2H), 3.71-3.68 (m, 2H), 3.64-3.61 (m, 1H), 3.55 (s, 2H), 3.32-3.25 (m, 2H), 2.53-2.44 (m, 4H).

### EXAMPLE 258

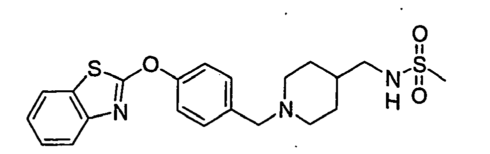

N-{1-[4-(Benzothiazol-2-yloxy)-benzyl]-piperidin-4-ylmethyl}-methanesulfonamide.
A. {1-[4-(Benzothiazol-2-yloxy)-benzyl]-piperidin-4-ylmethyl}-carbamic acid tert-butyl ester. A mixture of 4-(benzothiazol-2-yloxy)-benzaldehyde (1.0 g, 3.9 mmol), piperidin-4-ylmethyl-carbamic acid tert-butyl ester (1.3 g, 5.9 mmol) and molecular sieves (1.0 g, crushed, 4A) in ClCH₂CH₂Cl (15 mL) was stirred at room temperature for 40 min. To the resulting mixture was added NaBH(OAc)₃ portion wise over 1.5 h (4 x 412 mg, 7.8 mmol). The mixture was stirred at room temperature for 24 h, filtered through diatomaceous earth and rinsed with CH₂Cl₂ (100 mL). The filtrate was washed with sat. aq. NaHCO₃ (1 x 50 mL), dried (Na₂SO₄) and concentrated under reduced pressure to yield the crude product as a yellow semi-solid. The crude product was purified on SiO₂ (40 g; 0-100% acetone/CH₂Cl₂) to give a white foam (890 mg, 50% yield). MS (ESI): mass calculated for C₂₅H₃₁N₃O₃S, 453.6; m/z found, 454.4 [M+H]⁺. ¹H NMR (400 MHz, CDCl₃): 7.74 (d, *J* = 8.0, 1H), 7.67 (d, *J* = 8.0, 1H), 7.41-7.36 (m, 3H), 7.32-7.24 (m, 3H), 4.59 (br s, 1H), 3.50 (s, 2H), 3.06-3.00 (m, 2H), 2.94-2.88 (m, 2H), 1.97 (t, *J* = 11.4, 2H), 1.68 (d, *J* = 11.4, 2H), 1.44 (s, 9H), 1.22-1.33 (m, 2H).
B. C-{1-[4-(Benzothiazol-2-yloxy)-benzyl]-piperidin-4-yl}-methylamine. To a solution of {1-[4-(benzothiazol-2-yloxy)-benzyl]-piperidin-4-ylmethyl}-carbamic acid tert-butyl ester (866 mg, 1.9 mmol) in CH₂Cl₂ (5 mL) at 0 °C was added 4 N HCl in dioxane (2.4 mL, 9.5 mmol) dropwise. The resulting mixture was stirred at room temperature for 24 h and concentrated under reduced pressure to yield the crude product as a white solid. The crude product was triturated with Et₂O (50 mL) and isolated by filtration to yield the desired product as a white powder (813 mg, 100% yield). MS (ESI): mass calculated for C₂₀H₂₃N₃OS, 353.5; m/z found, 354.4 [M+H]⁺. ¹H NMR (400 MHz, CDCl₃): 7.80 (d, *J* = 8.0, 1H), 7.69 (d, *J* = 8.6, 2H), 7.60 (d, *J* = 8.0, 1H), 7.51 (d, *J* = 8.6, 1H), 7.42-7.38 (m, 1H), 7.33-7.28 (m, 1H), 4.37 (s, 2H), 3.78 (br d, *J* = 12.7, 1H), 2H), 3.13-3.04 (m, 2H), 2.89 (d, *J* = 6.6, 2H), 2.08-1.92 (m, 3H), 1.68-1.56 (m, 2H).
C. N-{1-[4-(Benzothiazol-2-yloxy)-benzyl]-piperidin-4-ylmethyl}-methanesulfonamide. To a mixture of C-{1-[4-(benzothiazol-2-yloxy)-benzyl]-piperidin-4-yl}-methylamine (150 mg, 0.39 mmol) in CH₂Cl₂ (5 mL) was added Et₃N (400 µL, 2.86 mmol). The resulting mixture was cooled to 0 °C and CH₃SO₂Cl was added via syringe (41 µL, 0.52 mmol). The mixture was stirred at room temperature for 24 h, diluted with CH₂Cl₂ (10 mL) and washed with sat. aq. NaHCO₃ (1 x 5 mL). The organic layer was dried (Na₂SO₄) and concentrated under reduced pressure to yield the crude product as a white solid. The crude product was purified on SiO₂ (10 g; 0-3% 2 M NH₃ in CH₃OH/CH₂Cl₂) to give a white solid (112 mg, 67% yield). MS (ESI): mass calculated for C₂₁H₂₅N₃O₃S₂, 431.6; m/z found, 432.4 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.33-7.25 (m, 3H), 4.25 (br t, *J* = 6.6, 1H), 3.51 (s, 2H), 3.04 (d, *J* = 6.6, 2H), 2.96 (s, 3H), 2.91-2.84 (m, 2H), 1.98 (t, *J* = 11.7, 2H), 1.74 (br d, *J* = 12.7, 2H), 1.60-1.48 (m, 2H), 1.36-1.24 (m, 2H).

### EXAMPLE 259

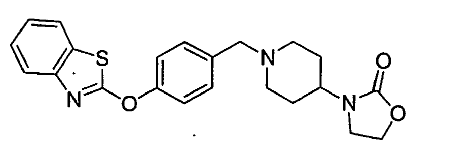

3-{1-[4-(Benzothiazol-2-yloxy)-benzyl]-piperidin-4-yl}-oxazolidin-2-one.
A. 3-Piperidin-4-yl-oxazolidin-2-one hydrochloride salt. To a solution of 1-benzyl-4-piperidinone (10.3 g, 54 mmol) and ethanolamine (13.2 mL, 218. mmol) in CH₃OH (20 mL) was added sodium cyanoborohydride (10.2 g, 163 mmol) and trifluoromethanesulfonic acid (5 mL) and the reaction was stirred at 23 °C for 3 days. The mixture was cooled to 0 °C and 12 N HCl was slowly added until gas evolution ceased and the resulting mixture was stirred for a further 3 h. The mixture was filtered and the filtrate concentrated under reduced pressure. The crude oil was redissolved in H₂O (50 mL), the solution was made basic by the addition of 10 N NaOH. The mixture was extracted with CH₂Cl₂ (8 x 70 mL). The combined CH₂Cl₂ extracts were dried and concentrated under reduced pressure to yield the crude product (12.0 g, 95%). A solution of 2-(1-benzyl-piperidin-4-ylamino)-ethanol (3.6 g, 15.3 mmol) in ClCH₂CH₂Cl (5 mL) was treated with carbonyl diimidazole (CDI) (2.6 g, 16 mmol) and the mixture stirred at 23 °C for 30 min. The mixture was diluted with CH₂Cl₂ (100 mL), washed with H₂O (1 x 50 mL) and sat. aq. NaHCO₃ (1 x 50 mL), dried, and concentrated under reduced pressure to yield 2.85 g (65%) of 3-(1-benzyl-piperidin-4-yl)-oxazolidin-2-one. To a solution of 3-(1-benzyl-piperidin-4-yl)-oxazolidin-2-one (2.3 g, 8.8 mmol) in ClCH₂CH₂Cl (40 mL) was added α-chloroethyl acetylchloride (1.5 g, 10.6 mmol) and the mixture was heated to 100 °C for 90 min. The mixture was cooled to 23 °C and concentrated under reduced pressure. The crude residue was dissolved in CH₃OH and heated to reflux for 1 h. The mixture was cooled to 0 °C and concentrated under reduced pressure to yield the title compound (1.89 g, 99%). MS (ESI): exact mass calculated for C₈H₁₄N₂O₂, 170.1; m/z found, 171.2 [M+H]⁺. ¹H NMR (400 MHz, DMSO-*d₆*): 8.97 (br s, 2H), 4.27 (dd, *J* = 9.1, 7.8, 2H), 3.80 (tt, *J* = 11.8, 4.2, 1H), 3.49 (dd, *J* = 8.0, 6.6, 1H), 3.30 (br d, *J* = 12.7, 2H), 2.97 (dt, *J* = 12.6, 2.3, 2H), 1.90 (ddd, *J* = 16.6, 13.0, 4.1, 2H), 1.86-1.75 (m, 2H).
B. [4-(Benzothiazol-2-yloxy)-phenyl]-methanol. To a mixture of 4-hydroxybenzyl alcohol (12 g, 97 mmol) in CH₃CN (200 mL) containing K₂CO₃ (22 g, 159 mmol) was added 2-chlorobenzothiazole (22 g, 130 mmol) and the mixture was heated to reflux for 72 h. The mixture was cooled to room temperature, filtered, and concentrated under reduced pressure to give the crude product as a golden oil. The crude oil was purified on SiO₂ (300 g; 5% acetone/CH₂Cl₂) to give a clear and colorless oil. (15 g, 60% yield). MS (ESI): exact mass calculated for C₁₄H₁₁NO₂S, 257.1; m/z found, 258.3 [M+H]⁺. ¹H NMR (400 MHz, DMSO-*d₆*): 7.92 (d, *J* = 7.4, 1H), 7.69 (d, *J* = 8.0, 1H), 7.50-7.31 (m, 5H), 7.32 (t, *J* = 7.5, 1H), 5.32 (t, *J* = 5.7, 1H), 4.55 (d, *J* = 5.7, 2H).
C. 2-(4-Chloromethyl-phenoxy)-benzothiazole. To a mixture of [4-(benzothiazol-2-yloxy)-phenyl]-methanol (11 g, 43 mmol) in CH₂Cl₂ (100 mL) containing triethytlamine (9 mL, 65 mmol) at 5°C was added dropwise over 15 minutes thionyl chloride (4 mL g, 55 mmol). The ice bath was removed and the mixture warmed to room temperature and stirred for 24 hr. The mixture was washed once with saturated K₂ CO₃ (100 mL), dried (MgSO₄), and concentrated under reduced pressure to give a black oil. The crude oil was purified on SiO₂ (300 g; 100% CH₂Cl₂) to give a clear orange oil. (10 g, 84% yield). MS (ESI): exact mass calculated for C₁₄H₁₀CINOS, 275.0; m/z found, 276.2 [M+H]⁺. ¹H NMR (400 MHz, DMSO-*d₆*): 7.95 (d, *J* = 7.3, 1H), 7.70 (d, *J* = 7.6, 1H), 7.59 (d, *J* = 8.6, 2H), 7.47 (d, *J* = 8.6, 2H), 7.37 (t, *J* = 7.4, 1H), 7.33 (t, *J* = 7.5, 1H), 4.84 (s, 2H).
D. 3-{1-[4-(Benzothiazol-2-yloxy)-benzyl]-piperidin-4-yl}-oxazolidin-2-one. To a mixture of 2-(4-chloromethyl-phenoxy)-benzothiazole (670 mg, 2.4 mmol) in CH₃CN (15 mL) containing K₂CO₃ (544 mg, 3.9 mmol) was added 3-piperidin-4-yl-oxazolidin-2-one hydrochloride salt (355 mg, 1.7 mmol) and the mixture was heated to 60 °C for 24 h. The mixture was cooled to room temperature, filtered, and concentrated under reduced pressure to give the crude product as a golden oil. The crude oil was purified on SiO₂ (12 g; acetone) to give a white solid (591 mg, 84% yield). MS (ESI): mass calculated for C₂₂H₂₃N₃O₃S, 409.2; m/z found, 410.3 [M+H]⁺. ¹H NMR (400 MHz, DMSO-*d*₆): 7.94 (d, *J* = 8.7, 1H), 7.70 (d, *J* = 7.5, 1H), 7.48-7.37 (m, 5H), 7.33 (d, *J* = 8.0, 1H), 4.25 (t, *J* = 7.7, 2H), 3.60-3.42 (m, 5H), 2.85 (d, *J* = 11.5, 2H), 2.04 (t, *J* =11.4, 2H), 1.78-1.58 (m, 4H).

### EXAMPLE 260

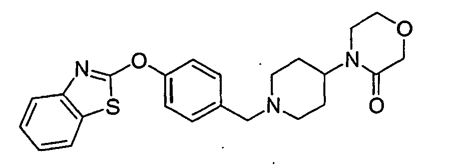

4-{1-[4-(Benzothiazol-2-yloxy)-benzyl]-piperidin-4-yl}-morpholin-3-one.
A. 4-Piperidin-4-yl-morpholin-3-one. To a solution of 1-benzyl-4-piperidinone (10.3 g, 54 mmol) and ethanolamine (13.2 mL, 218 mmol) in CH₃OH (20 mL) was added sodium cyanoborohydride (10.2 g, 163 mmol) and trifluoromethanesulfonic acid (5 mL) and the reaction mixture was stirred at 23 °C for 3 days. The mixture was cooled to 0 °C and 12 N HCl was slowly added until gas evolution ceased and the resulting mixture was stirred for a further 3 h. The mixture was filtered and the filtrate concentrated. The crude oil was redissolved in H₂O (50 mL), and the solution was made basic by the addition of 10 N NaOH. The mixture was extracted with CH₂Cl₂ (8 x 70 mL). The combined CH₂Cl₂ extracts were dried and concentrated under reduced pressure to yield 12.0 g (95% yield) crude product. A 0 °C solution of 2-(1-benzyl-piperidin-4-ylamino)-ethanol (2.13 g, 9.1 mmol) in ethanol (11 mL) and H₂O (5 mL) was treated simultaneously with chloroacetyl chloride (1.8 mL, 22.7 mmol) and 35% aq. NaOH solution and the mixture was stirred below 20 °C for 3 h. The reaction was diluted with H₂O (20 mL) and extracted with ethyl acetate (2 x 50 mL). The combined organic layers were washed with brine, dried, and concentrated to yield 1.83 g (66% yield) of 4-(1-benzyl-piperidin-4-yl)-morpholin-3-one. To a solution of 4-(1-benzyl-piperidin-4-yl)-morpholin-3-one (1.1 g, 3.9 mmol) in ClCH₂CH₂Cl (20 mL) was added α-chloroethyl acetylchloride (660 mg, 4.6 mmol) and the reaction mixture was heated to 100 °C for 16 h. The mixture was cooled to 23°C and concentrated under reduced pressure. The resulting crude material was purified on SiO₂ (12 g, 0-10% 2 M ammonia in CH₃OH/CH₂Cl₂) to yield 282 mg (53% yield) of 4-piperidin-4-yl-morpholin-3-one. MS (ESI): exact mass calculated for C₈H₁₄N₂O₂, 184.1; m/z found, 185.4 [M+H]⁺. ¹H NMR (400 MHz, CDCl₃): 3.72 (s, 2H), 2.94 (t, *J* = 6.6, 2H), 2.55 (t, *J* = 3.6, 2H), 2.46 (tt, *J* = 10.3, 3.7, 1H), 1.92 (dd, *J* = 12.3, 2.3, 2H), 1.82-1.70 (m, 2H), 1.70-1.60 (m, 1H), 1.40-1.02 (m, 4H).
B. 4-{1-[4-(Benzothiazol-2-yloxy)-benzyl]-piperidin-4-yl}-morpholin-3-one. The title compound was prepared according to EXAMPLE 259, step D. MS (ESI): exact mass calculated for C₂₄H₂₇N₃O₃S₁, 423.16; m/z found, 424.3 [M+H]⁺. ¹H NMR (400 MHz, CDCl₃): 7.77 (dd, *J* = 8.1, 0.6, 1H), 7.70 (dd, *J* = 7.9, 0.7, 1H), 7.45-7.40 (m, 2H), 7.36-7.28 (m, 4H), 4.57 (tt, *J* = 12.1, 4.2, 1H), 4.22 (s, 2H), 3.90 (t, *J* = 4.9, 2H), 3.56 (s, 2H), 3.34 (t, *J* = 5.1, 2H), 3.01 (br d, *J* = 11.6, 2H), 2.18 (ddd, *J* = 11.7, 11.7, 2.2, 2H), 1.79 (dddd, *J* = 12.1, 12.1, 12.0, 3.8, 2H), 1.72-1.65 (m, 2H).

### EXAMPLE 271

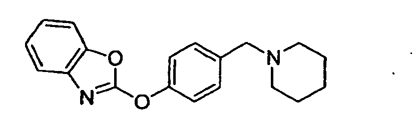

2-(4-Piperidin-1-ylmethyl-phenoxy)-benzooxazole.
A. 4-Piperidin-1-ylmethyl-phenol. A mixture of 4-hydroxybenzaldehyde (10 g, 82 mmol), piperidine (16 mL, 164 mmol), and molecular sieves (10 g, crushed, 4A) in ClCH₂CH₂Cl (150 mL) was stirred at room temperature for 40 min. To the resulting mixture was added NaBH(OAc)₃ portion wise over 1.5 h (7 x 5 g, 164 mmol). The mixture was stirred at room temperature for 24 h. The resulting mixture was diluted with CH₂Cl₂, (300 mL) filtered through diatomaceous earth and rinsed with additional CH₂Cl₂ (100 mL). The filtrate was washed with sat. aq. NaHCO₃ (3 x 150 mL), and extracted with 25% isopropanol/CHCl₃, dried (Na₂SO₄) and concentrated under reduced pressure to 1H yield the crude product as a orange semi-solid. The crude product was purified on SiO₂ (120 g; 0-100% acetone/CH₂Cl₂) to give a yellow solid (3.08 g, 48% yield). MS (ESI): mass calculated for C₁₂H₁₇NO, 191.1; m/z found, 192.4 [M+H]⁺. ¹H NMR (400 MHz, CDCl₃): 7.18 (d, *J* = 8.4, 2H), 6.75 (d, *J* = 8.4, 2H), 3.83 (s, 2H), 2.81 (br s, 4H), 1.77 (quint, *J* = 5.7, 4H), 1.54 (br s, 2H).
B. 2-(4-Piperidin-1-ylmethyl-phenoxy)-benzooxazole. The title compound was prepared according to the procedure for EXAMPLE 13, step B using 4-piperidin-1-ylmethyl-phenol. MS (ESI): mass calculated for C₁₉H₂₀N₂O₂S, 308.2; m/z found, 309.4 [M+H]⁺. ¹H NMR (400 MHz, DMSO-*d*₆): 7.64 (d, *J* = 3.1 Hz, 1H), 7.52 (d, *J* = 8.9 Hz, 1H), 7.3 (q, *J* = 8.3 Hz, 4H), 7.34-7.26 (m, 2H), 3.45 (s, 2H), 2.33 (br.s, 4H), 1.57-1.46 (m, 4H), 1.44-1.32 (m, 2H). 1H), 7.68-7.56 (m, 5H), 7.53 (t, *J* = 7.1, 1H), 3.70 (s, 2H), 3.51-3.45 (m, 4H), 2.60-2.50 (m, 4H), 1.66 (t, *J* = 5.3, 4H), 1.58 (s, 9H), 1.54 (t, *J* = 5.4, 4H).

### EXAMPLE 305

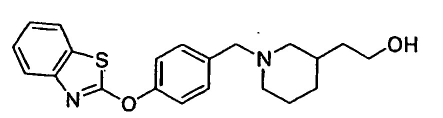

2-{1-[4-(Benzothiazol-2-yloxy)-benzyl]-piperidin-3-yl}ethanol.

The title compound was prepared according to the procedure for EXAMPLE 259, step D using 2-piperidin-3-yl-ethanol. MS (ESI): mass calculated for C₂₁H₂₄N₂O₂S, 368.2; m/z found, 369.4 [M+H]⁺. ¹H NMR (400 MHz, DMSO-*d*₆): 7.71 (d, *J* = 8.4, 1H), 7.47 (d, *J* = 7.9, 1H), 7.25-7.15 (m, 5H), 7.10 (t, *J* = 8.1, 1H), 4.12 (t, *J* = 5.1, 1H), 3.33-3.15 (m, 4H), 2.58-2.42 (m, 2H), 1.67 (t, *J* = 9.6, 1H), 1.53-1.33 (m, 4H), 1.30-1.00 (m, 3H), 0.70-0.58 (m, 1H).

### EXAMPLE 357

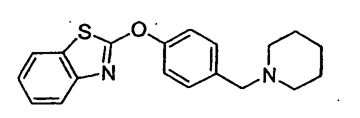

2-(4-Piperidin-1-ylmethyl-phenoxy)-benzothiazole.

The title compound was prepared according to the procedure for EXAMPLE 251, step B using piperidine. MS (ESI): mass calculated for C₁₉H₂₀N₂OS, 324.4; m/z found, 325.3 [M+H]⁺. ¹H NMR (400 MHz, CDCl₃): 7.75 (d, *J* = 8.2, 1H), 7.73 (d, *J* = 8.2, 1H), 7.36-7.42 (m, 3H), 7.24-7.32 (m, 3H), 3.49 (s, 2H), 2.39 (br s, 3H), 1.56-1.64 (m, 5H), 1.40-1.48 (m, 2H).

### EXAMPLE 358

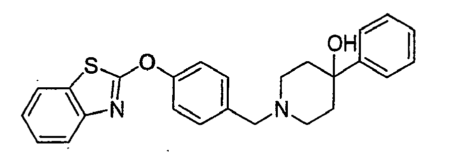

1-[4-(Benzothiazol-2-yloxy)-benzyl]-4-phenyl-piperidin-4-ol.

The title compound was prepared according to the procedure for EXAMPLE 251, step B using 4-phenyl-piperidin-4-ol. MS (ESI): mass calculated for C₂₅H₂₄N₂O₂S, 416.5; m/z found, 417.4 [M+H]⁺. ¹H NMR (400 MHz, CDCl₃): 7.75 (d, *J* = 8.0, 1H), 7.66 (d, *J* = 8.0, 1H), 7.55-7.51 (m, 2H), 7.47-7.25 (m, 9H), 3.63 (s, 2H), 2.85-2.77 (m, 2H), 2.50 (dt, *J* = 11.8, 2.5, 2H), 2.18 (dt, *J* = 13.0, 4.4, 2H), 1.80-1.74 (m, 2H), 1.55 (br s, 1H).

### EXAMPLE 359

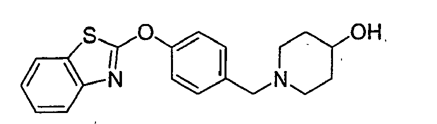

1-[4-(Benzothiazol-2-yloxy)-benzyl]-piperidin-4-ol.

The title compound was prepared according to the procedure for EXAMPLE 251, step B using 4-piperidinol. MS (ESI): mass calculated for C₁₉H₂₀N₂O₂S, 340.4; m/z found, 341.3 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.32-7.24 (m, 3H), 3.68-3.64 (m, 1H), 3.46 (s, 2H), 2.81-2.25 (m, 2H), 2.20-2.15 (m, 2H), 1.95-1.90 (m, 2H), 1.67-1.58 (m, 3H).

### EXAMPLE 360

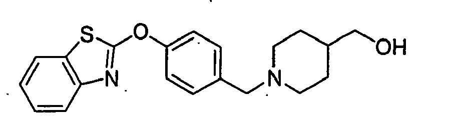

{1-[4-(Benzothiazol-2-yloxy)-benzyl]-piperidin-4-yl}-methanol.

The title compound was prepared according to the procedure for EXAMPLE 251, step B using piperidin-4-yl-methanol. MS (ESI): mass calculated for C₂₀H₂₂N₂O₂S, 354.5; m/z found, 355.3 [M+H]⁺. ¹H NMR (400 MHz, CDCl₃): 7.74 (d, *J* = 8.0, 1H), 7.67 (d, *J* = 8.0, 1H), 7.42-7.37 (m, 3H), 7.32-7.25 (m, 3H), 3.54 (s, 2H), 3.51 (d, *J* = 6.5, 2H), 2.97-2.90 (m, 2H), 2.01 (dt, *J* = 11.6, 2.4, 3H), 1.73 (d, *J* = 12.1, 2H), 1.58-1.47 (m, 1H), 1.37-1.26 (m, 2H).

### EXAMPLE 361

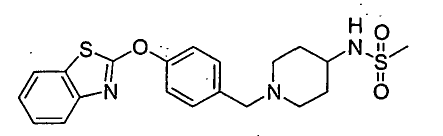

N-{1-[4-(Benzothiazol-2-yloxy)-benzyl]-piperidin-4-yl}-methanesulfonamide.

The title compound was prepared according to the procedure for EXAMPLE 253, step A and B followed by EXAMPLE 258 step C. MS (ESI): mass calculated for C₂₀H₂₃N₃O₃S₂, 417.6; m/z found, 418.3 [M+H]⁺. ¹H NMR (400 MHz, CDCl₃): 7.74 (d, *J* = 8.0, 1H), 7.68 (d, *J* = 8.0, 1H), 7.42-7.36 (m, 3H), 7.35-7.25 (m, 3H), 4.32-4.5 (m, 1H), 3.58 (s, 2H), 3.43-3.32 (m, 1H), 2.99 (s, 3H), 2.87-2.80 (m, 2H), 2.18-2.10 (m, 2H), 2.04-1.96 (m, 2H), 1.64-1.54 (m, 2H).

### EXAMPLE 362

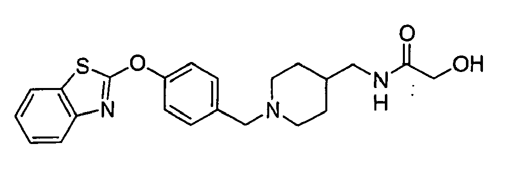

N-{1-[4-(Benzothiazol-2-yloxy)-benzyl]-piperidin-4-ylmethyl}-2-hydroxy-acetamide.

The title compound was prepared according to the procedure for EXAMPLE 258, step A and B followed by EXAMPLE 253 step C using glycolic acid. MS (ESI): mass calculated for C₂₂H₂₅N₃O₃S, 411.5; m/z found, 412.4 [M+H]⁺. ¹H NMR (400 MHz, CDCl₃): 7.74 (d, *J* = 8.2, 1H), 7.67 (d, *J* = 8.2, 1H), 7.42-7.37 (m, 3H), 7.32-7.25 (m, 3H), 4.11 (s, 2H), 3.51 (s, 2H), 3.24 (t, *J* = 6.3, 2H), 2.88-2.94 (m, 2H), 2.20 (d, *J* = 11.5, 1H), 2.02-1.92 (m, 2H), 1.72-1.65 (m, 2H), 1.62-1.50 (m, 2H), 1.36-1.26 (m, 2H).

### EXAMPLE 363

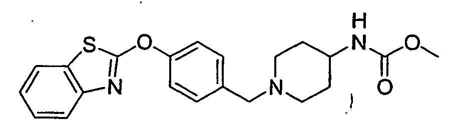

{1-[4-(Benzothiazol-2-yloxy)-benzyl]-piperidin-4-yl}-carbamic acid methyl ester.

The title compound was prepared according to the procedure for EXAMPLE 253, step A and B followed by EXAMPLE 258 step C using methyl isocyanate. MS (ESI): mass calculated for C₂₁H₂₃N₃O₃S, 397.5; m/z found, 398.3 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.32-7.24 (m, 3H), 4.57 (br s, 1H), 3.66 (s, 3H), 3.51 (s, 2H), 2.85-2.79 (m, 2H), 2.18-2.09 (m, 2H), 1.98-1.90 (m, 2H), 1.51-1.40 (m, 2H).

### EXAMPLE 364

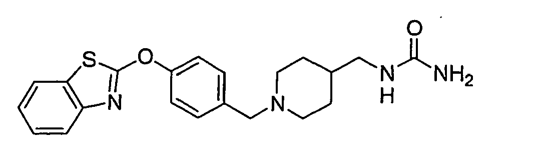

{1-[4-(Benzothiazol-2-yloxy)-benzyl]-piperidin-4-ylmethyl}-urea.

The title compound was prepared according to the procedure for EXAMPLE 258, step A, B and C using trimethylsilyl isocyanate. MS (ESI): mass calculated for C₂₁H₂₄N₄O₂S, 396.5; m/z found, 397.4 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.32-7.24 (m, 3H), 4.64 (br s, 1H), 4.35 (s, 2H), 3.50 (s, 2H), 3.07 (br t, *J* = 6.3, 2H), 2.94-2.88 (m, 2H), 1.86-2.01 (m, 2H), 1.72-1.66 (m, 2H), 1.56-1.44 (s, 1H), 1.24-1.23 (m, 2H).

### EXAMPLE 365

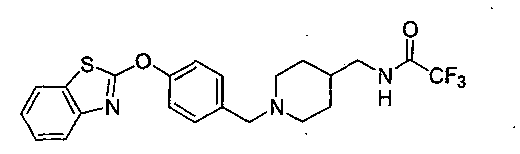

N-{1-[4-(Benzothiazol-2-yloxy)-benzyl]-piperidin-4-ylmethyl}-2,2,2-trifluoroacetamide

The title compound was prepared according to the procedure for EXAMPLE 258, step A and B and EXAMPLE 257, step C using trifluoroacetic acid. MS (ESI): mass calculated for C₂₂H₂₂F₃N₃O₂S, 449.5; m/z found, 450.4 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.32-7.24 (m, 3H), 6.41 (br s, 1H), 3.51 (s, 2H), 3.28 (t, *J* = 6.46, 2H), 2.92 (d, *J* = 11.5, 2H), 2.02-1.94 (m, 2H), 1.74-1.54 (m, 3H), 1.38-1.27 (m, 2H).

### EXAMPLE 366

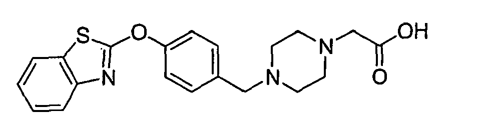

{4-[4-(Benzothiazol-2-yloxy)-benzyl]-piperazin-1-yl}-acetic acid.

The title compound was prepared according to the procedure for EXAMPLE 259, step C and D and EXAMPLE 250, step D using piperazin-1-yl-acetic acid. MS (ESI): mass calculated for C₂₀H₂₁N₃O₃S, 383.5; m/z found, 384.4 [M+H]⁺. ¹H NMR (400 MHz, CD₃OD): 7.77 (d, *J* = 8.0, 1H), 7.62 (d, *J* = 8.0, 1H), 7.50-7.46 (m, 2H), 7.42-7.26 (m, 4H), 6.67 (s, 2H), 3.60 (s, 2H), 3.34 (br s, 4H), 2.77 (br s, 4H).

### EXAMPLE 367

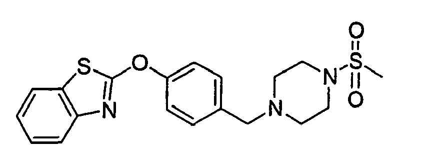

2-[4-(4-Methanesulfonyl-piperazin-1-ylmethyl)-phenoxy]-benzothiazole.

The title compound was prepared according to the procedure for EXAMPLE 257, step A and B and EXAMPLE 258 step C. MS (ESI): mass calculated for C₁₉H₂₁N₃O₃S₂, 403.53; m/z found, 404.3 [M+H]⁺. ¹H NMR (400 MHz, CDCl₃): 7.74 (d, *J* = 8.0, 1H), 7.68 (d, *J* = 8.0, 1H), 7.42-7.36 (m, 3H), 7.35-7.26 (m, 3H), 3.57 (s, 2H), 3.26 (br t, *J* = 4.7, 4H), 2.79 (s, 3H), 2.58 (br t, *J* = 4.7, 4H).

### EXAMPLE 368

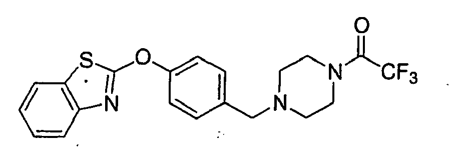

1-{4-[4-(Benzothiazol-2-yloxy)-benzyl]-piperazin-1-yl}-2,2,2-trifluoro-ethanone.

The title compound was prepared according to the procedure for EXAMPLE 257, step A, B and C using trifluoroacetic acid. MS (ESI): mass calculated for C₂₀H₁₈F₃N₃O₂S, 421.44; m/z found, 422.4 [M+H]⁺. ¹H NMR (400 MHz, CDCl₃): 7.74 (d, *J* = 8.0, 1H), 7.68 (d, *J* = 8.0, 1H), 7.42-7.27 (m, 6H), 3.74-3.70 (m, 2H), 3:65-3.60 (m, 2H), 3.57 (s, 2H), 2.56-2.50 (m, 4H).

### EXAMPLE 369

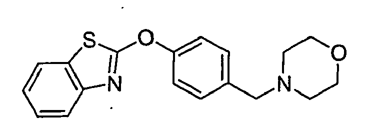

2-(4-Morpholin-4-ylmethyl-phenoxy)-benzothiazole.

The title compound was prepared according to the procedure for EXAMPLE 259, step C and D using morpholine. MS (ESI): mass calculated for C₁₈H₁₈N₂O₂S, 326.42; m/z found, 327.4 [M+H]⁺. ¹H NMR (500 MHz, CDCl₃): 7.74 (d, *J* = 7.7, 1H), 7.67 (d, *J* = 7.7, 1H), 7.42-7.37 (m, 3H), 7.33-7.25 (m, 3H), 3.72 (t, *J* = 3.7, 4H), 3.52 (s, 2H), 2.46 (br s, 4H).

### EXAMPLE 370

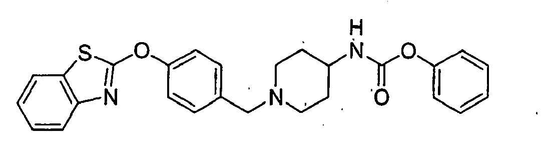

{1-[4-(Benzothiazol-2-yloxy)-benzyl]-piperidin-4-yl}-carbamic acid phenyl ester.

The title compound was prepared according to the procedure for EXAMPLE 253, step A and B followed by EXAMPLE 258 step C using phenyl isocyanate. MS (ESI): mass calculated for C₂₆H₂₅N₃O₃S, 459.57; m/z found, 460.4 [M+H)⁺. ¹H NMR (400 MHz, CDCl₃): 7.74 (d, *J* = 8.0, 1H), 7.67 (d, *J* = 8.0, 1H), 7.42-7.10 (m, 11H), 4.95 (br d, *J* = 7.8, 1H), 3.67-3.58 (m, 1H), 3.53 (s, 2H), 2.88-2.82 (m, 2H), 2.21-2.06 (m, 2H), 2.03-1.99 (m, 2H), 1.61-1.44 (m, 2H).

### EXAMPLE 371

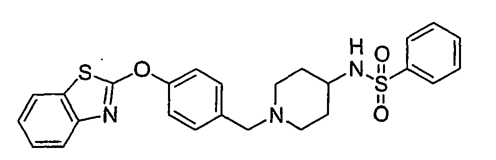

N-{1-[4-(Benzothiazol-2-yloxy)-benzyl]-piperidin-4-yl}-benzenesulfonamide.

The title compound was prepared according to the procedure for EXAMPLE 253, step A and B followed by EXAMPLE 258, step C using benzenesulfonyl chloride. MS (ESI): mass calculated for C₂₅H₂₅N₃O₃S₂, 479.62; m/z found, 480.4 [M+H]⁺. ¹H NMR (400 MHz, CDCl₃): 7.86-7.84 (m, 2H), 7.75 (d, *J* = 8.0, 1H), 7.67 (d, *J* = 8.0, 1H), 7.60-7.48 (m, 3H), 7.41-7.24 (m, 6H), 4.56 (br d, *J* = 7.8, 1H), 3.48 (s, 2H), 3.26-3.16 (m, 1H), 2.76-2.68 (m, 2H), 2.06-1.97 (m, 2H), 1.78-1.70 (m, 1H), 1.52-1.42 (m, 2H).

### EXAMPLE 372

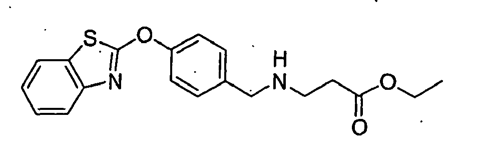

3-[4-(Benzothiazol-2-yloxy)-benzylamino]-propionic acid ethyl ester.

The title compound was prepared according to the procedure for EXAMPLE 251, step A and B using 3-amino-propionic acid ethyl ester. MS (ESI): mass calculated for C₁₉H₂₀N₂O₃S, 356.45; m/z found, 357.3 [M+H]⁺. ¹H NMR (400 MHz, CDCl₃): 7.74 (d, *J* = 8.0, 1H), 7.67 (d, *J* = 8.0, 1H), 7.44-7.25 (m, 6H), 4.21 (br s, 1H), 4.15 (q, *J* = 7.2, 2H), 3.87 (s, 2H), 2.95 (t, *J* = 6.3, 2H), 2.58 (t, *J* = 6.3, 2H), 1.26 (t, *J* = 7.2, 3H).

### EXAMPLE 373

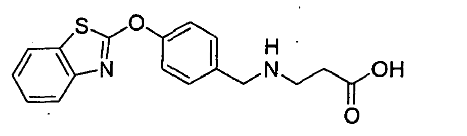

3-[4-(Benzothiazol-2-yloxy)-benzylamino]-propionic acid.

The title compound was prepared according to the procedure for EXAMPLE 372, and EXAMPLE 250, step D. MS (ESI): mass calculated for C₁₇H₁₆N₂O₃S, 328.39; m/z found, 329.3 [M+H]⁺. ¹H NMR (400 MHz, DMSO): 7.87 (d, *J* = 8.0, 1H), 7.62 (d, *J* = 8.0, 1H), 7.44-7.23 (m, 6H), 3.77 (s, 2H), 2.72 (t, *J* = 6.6, 2H), 2.29 (t, *J* = 6.6, 2H).

### EXAMPLE 434

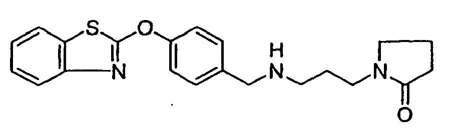

1-{3-[4-(Benzothiazol-2-yloxy)-benzylamino]-propyl}-pyrrolidin-2-one.

The title compound was prepared according to the procedure for EXAMPLE 251, step B using 1-(3-amino-propyl)-pyrrolidine-2-one. MS (ESI): mass calculated for C₂₁H₂₃N₃O₂S, 381.15; m/z found, 382.3 [M+H]⁺. ¹H NMR (400 MHz, CDCl₃): 7.71 (d, *J* = 8.1, 1H), 7.64 (d, *J* = 8.1, 1H), 7.45-7.41 (m, 2H), 7.36 (t, *J* = 8.1, 1H), 7.32-7.28 (m, 2H), 7.24 (t, *J* = 8.1, 1H), 3.81 (s, 2H), 3.38-3.27 (m, 5H), 3.63 (t, *J* = 6.8, 2H), 3.35 (t, *J* = 7.6, 2H), 2.02-1.93 (m, 2H), 1.82-1.73 (m, 2H).

### EXAMPLE 435

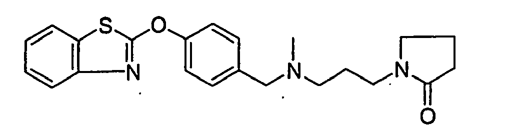

1-(3-{[4-(Benzothiazol-2-yloxy)-benzyl]-methyl-amino}-propyl)-pyrrolidin-2-one.

The title compound was prepared according to the procedure for EXAMPLE 409 using formaldehyde. MS (ESI): mass calculated for C₂₂H₂₅N₃O₂S, 395.17; m/z found, 396.4 [M+H]⁺. ¹H NMR (400 MHz, CDCl₃): 7.71 (d, *J* = 8.1, 1H), 7.64 (d, *J* = 8.1, 1H), 7.43-7.27 (m, 5H), 7.24 (t, *J* = 8.1, 1H), 3.54 (s, 2H), 3.32 (dd, *J* = 7.1, 7.1, 4H), 2.43 (t, *J* = 7.1, 2H), 2.34 (t, *J* = 7.1, 2H), 2.23 (s, 3H), 2.00-1.91 (m, 2H), 1.80-1.70 (m, 2H).

### EXAMPLE 436

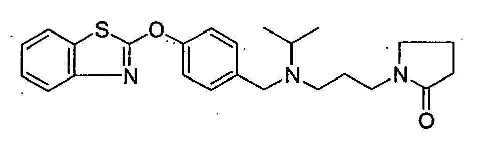

1-(3-{[4-(Benzothiazol-2-yloxy)-benzyl]-isopropyl-amino}-propyl)-pyrrolidin-2-one.

The title compound was prepared according to the procedure for EXAMPLE 409 using acetone. MS (ESI): mass calculated for C₂₄H₂₉N₃O₂S, 423.20; m/z found, 424.4 [M+H]⁺. ¹H NMR (400 MHz, CDCl₃): 7.72 (d, *J* = 8.1, 1H), 7.65 (d, *J* = 8.1, 1H), 7.45-7.40 (m, 2H), 7.37 (t, *J* = 7.8, 1H), 7.30-7.21 (m, 3H), 3.55 (s, 2H), 3.29-3.20 (m, 4H), 3.01-2.90,(m, 1H), 2.42 (t, *J* = 7.1, 2H), 2.32 (t, *J* = 7.8, 2H), 1.97-1.83 (m, 2H), 1.63-1.53 (m, 2H), 1.02 (d, *J* = 6.6, 6H).

### EXAMPLE 437

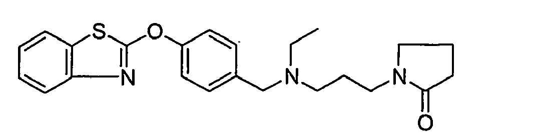

1-(3-{[4-(Benzothiazol-2-yloxy)-benzyl]-ethyl-amino}-propyl)-pyrrolidin-2-one.

The title compound was prepared according to the procedure for EXAMPLE 409 using acetaldehyde. MS (ESI): mass calculated for C₂₃H₂₇N₃O₂S, 409.18; m/z found, 410.4 [M+H]⁺. ¹H NMR (400 MHz, CDCl₃): 7.72 (d, *J* = 8.1, 1H), 7.66 (d, *J* = 8.1, 1H), 7.43-7.34 (m, 3H), 7.32-7.23 (m, 3H), 3.56 (s, 2H), 3.30 (dd, *J* = 7.1, 7.1, 4H), 2.53 (dd, *J* = 7.1, 7.1, 2H), 2.45 (t, *J* = 7.1, 2H), 2.34 (t, *J* = 7.8, 2H), 2.00-1.91 (m, 2H), 1.73-1.64 (m, 2H), 1.04 (t, *J* = 7.1, 3H).

### EXAMPLE 438

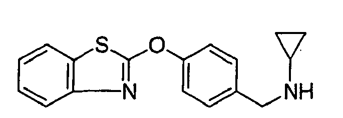

[4-(Benzothiazol-2-yloxy)-benzyl]-cyclopropyl-amine.

The title compound was prepared according to the procedure for EXAMPLE 251, step B using cyclopropylamine. MS (ESI): mass calculated for C₁₇H₁₆N₂OS, 296.10; m/z found, 297.3 [M+H]⁺. ¹H NMR (400 MHz, CDCl₃): 7.72 (d, *J* = 8.1, 1H), 7.63 (d, *J* = 8.1, 1H), 7.40-7.33 (m, 3H), 7.32-7.27 (m, 2H), 7.24 (t, *J* = 8.1, 1H), 3.85 (s, 2H), 2.19-2.12 (m, 1H), 1.86 (br s, 1H), 0.48-0.35 (m, 4H).

### EXAMPLE 439

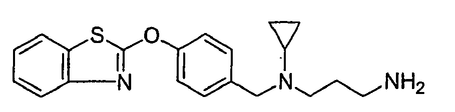

N-1-[4-(Benzothiazol-2-yloxy)-benzyl]-N1-cyclopropyl-propane-1,3-diamine.

The title compound was prepared according to the procedure for EXAMPLE 421 using compound from EXAMPLE 438. MS (ESI): mass calculated for C₂₀H₂₃N₃OS, 353.16; m/z found, 354.3 [M+H]⁺. ¹H NMR (400 MHz, CDCl₃): 7.73 (d, *J* = 8.1, 1H), 7.65 (d, *J* = 8.1, 1H), 7.41-7.32 (m, 3H), 7.30-7.23 (m, 3H), 3.75 (s, 2H), 2.67 (t, *J* = 7.1, 2H), 2.58 (t, *J* = 7.1, 2H), 1.80-1.73 (m, 1H), 1.72-1.64 (m, 2H), 1.19 (br s, 2H), 0.52-0.46 (m, 2H), 0.42-0.36 (m, 2H).

### EXAMPLE 440

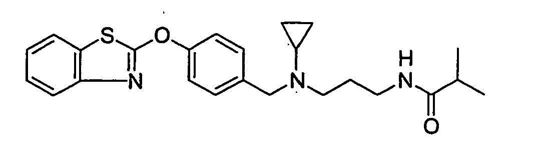

N-(3-{[4-(Benzothiazol-2-yloxy)-benzyl]-cyclopropyl-amino}-propyl)-isobutyramide.

The title compound was prepared according to the procedure for EXAMPLE 422 using isobutyryl chloride. MS (ESi): mass calculated for C₂₄H₂₉N₃O₂S, 423.20; m/z found, 424.4 [M+H]⁺. ¹H NMR (400 MHz, CDCl₃): 7.72 (d, *J* = 8.1, 1H), 7.65 (d, *J* = 8.1, 1H), 7.40-7.22 (m, 6H), 6.26 (br s, 1H), 3.73 (s, 2H), 3.23 (dd, *J* = 6.3, 5.8, 2H), 2.61 (t, *J* = 6.6, 2H), 2.29-2.18 (m, 1H), 1.79-1.68 (m, 3H), 1.09 (d, *J* = 6.8, 6H), 0.54-0.47 (m, 2H), 0.43-0.37 (m, 2H).

### EXAMPLE 441

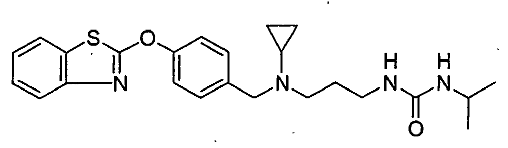

1-(3-{[4-(Benzothiazol-2-yloxy)-benzyl]-cyclopropyl-amino}-propyl)-3-isopropyl-urea.

The title compound was prepared according to the procedure for EXAMPLE 422 using isopropyl isocyanate. MS (ESI): mass calculated for C₂₄H₃₀N₄O₂S, 438.21; m/z found, 439.4 [M+H]⁺. ¹H NMR (400 MHz, CDCl₃): 7.69 (d, *J* = 8.1, 1H), 7.63 (d, *J* = 8.1, 1H), 7.38-7.29 (m, 3H), 7.27-7.20 (m, 3H), 5.44 (br s, 1H), 5.15 (d, *J* = 7.8, 1H), 3.87-3.76 (m, 1H), 3.70 (s, 2H), 3.12 (dd, *J* = 6.3, 6.1, 2H), 2.56 (t, *J* = 7.1, 2H), 1.76-1.67 (m, 3H), 1.07 (d, *J* = 6.3, 6H), 0.49-0.42 (m, 2H), 0.40-0.34 (m, 2H).

### EXAMPLE 442

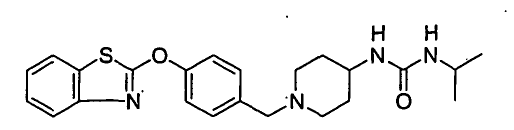

1-{1-[4-(Benzothiazol-2-yloxy)-benzyl]-piperidin-4-yl}-3-isopropyl-urea.

The title compound was prepared according to the procedure for EXAMPLE 253, step C using isopropyl isocyanate. MS (ESI): mass calculated for C₂₃H₂₈N₄O₂S, 424.19; m/z found, 425.3 (M⁺H]⁺. ¹H NMR (400 MHz, CDCl₃): 7.73 (d, *J* = 8.1, 1H), 7.67 (d, *J* = 8.1, 1H), 7.41-7.35 (m, 3H), 7.32-7.24 (m, 3H), 4.77 (dd, *J* = 8.1, 7.8, 1H), 3.88-3.78 (m, 1H), 3.66-3.55 (m, 1H), 3.51 (s, 2H), 2.82 (d, *J* = 11.6, 2H), 2.13 (t, *J* = 10.9, 2H), 1.94 (d, *J*= 11.9, 2H), 1.69 (br s, 1H), 1.48-1.36 (m, 2H), 1.14 (d, *J* = 6.6, 6H).

### EXAMPLE 443

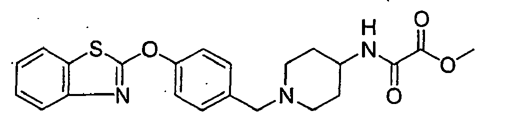

N-{1-[4-(Benzothiazol-2-yloxy)-benzyl]-piperidin-4-yl}-oxalamic acid methyl ester.

The title compound was prepared according to the procedure for EXAMPLE 253, step C using chloro-oxo-acetic acid methyl ester. MS (ESI): mass calculated for C₂₂H₂₃N₃O₄S, 425.14; m/z found, 426.2 [M+H]⁺. ¹H NMR (400 MHz, CDCl₃): 7.78 (d, *J* = 8.1, 1H), 7.64 (d, *J* = 8.1, 1H), 7.55-7.50 (m, 2H), 7.44-7.36 (m, 3H), 7.31 (t, *J* = 8.1, 1H), 3.84 (s, 2H), 3.81-3.72 (m, 1H), 3.41 (s, 3H), 3.11 (d, *J* = 10.9, 2H), 2.59-2.47 (m, 2H), 2.00-1.91 (m, 2H), 1.79-1.66 (m, 2H).

### EXAMPLE 444

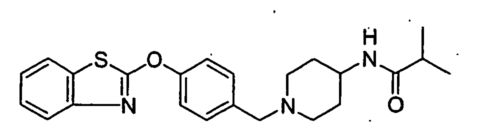

N-{1-[4-(Benzothiazol-2-yloxy)-benzyl]-piperidin-4-yl}-isobutyramide.

The title compound was prepared according to the procedure for EXAMPLE 253 using isobutyryl chloride. MS (ESI): mass calculated for C₂₃H₂₇N₃O₂S, 409.18; m/z found, 410.3 [M+H]⁺. ¹H NMR (400 MHz, CDCl₃): 7.84 (d, *J* = 8.6, 2H), 7.69 (t, *J* = 7.8, 2H), 7.44 (d, *J* = 8.6, 2H), 7.41-7.33 (m, 2H), 7.27 (t, *J* = 8.1, 1H), 4.33 (s, 2H), 4.16-4.00 (m, 1H), 3.57-3.42 (m, 2H), 3.31-3.16 (m, 2H), 2.58-2.45 (m, 1H), 2.31-2.07 (m, 4H), 1.10 (d, *J* = 6.8, 6H).

### EXAMPLE 445

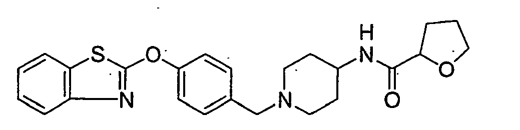

Tetrahydro-furan-2-carboxylic acid {1-[4-(benzothiazol-2-yloxy)-benzyl]-piperidin-4-yl}-amide.

The title compound was prepared from EXAMPLE 253, step D following the procedure for EXAMPLE 431. MS (ESI): mass calculated for C₂₄H₂₇N₃O₃S, 437.18; m/z found, 438.3 [M+H]⁺. ¹H NMR (400 MHz, CDCl₃): 11.03 (br s, 1H), 7.73 (d, *J* = 7.8, 1H), 7.67 (d, *J* = 7.8, 1H), 7.46-7.42 (m, 2H), 7.41-7.32 (m, 3H), 7.27 (d, *J* = 7.8, 1H), 4.31 (t, *J* = 8.34, 1H), 3.98-3.89 (m, 1H), 3.84 (s, 2H), 3.19 (d, *J* = 11.6, 2H), 2.43 (t, *J* = 10.4, 2H), 2.34-2.21 (m, 2H), 2.08-1.69 (m, 8H).

### EXAMPLE 446

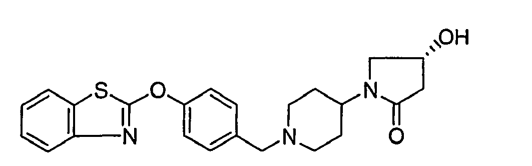

1-{1-[4-(Benzothiazol-2-yloxy)-benzyl]-piperidin-4-yl}-4-hydroxy-pyrrolidin-2-one.

The title compound was prepared from EXAMPLE 253, step D following the procedure for EXAMPLE 254, step D. MS (ESI): mass calculated for C₂₃H₂₅N₃O₃S, 423.16; m/z found, 424.3 [M+H]⁺. ¹H NMR (400 MHz, CD₃OD): 7.80 (d, *J* = 8.1, 1H), 7.68-7.62 (m, 3H), 7.53-7.49 (m, 2H), 7.42 (t, *J* = 8.1, 1H), 7.32 (t, *J* = 8.3, 1H), 4.44 (t, *J* = 6.3, 1H), 4.38 (s, 2H), 4.25-4.15 (m, 1H), 3.68-3.57 (m, 3H), 3.33-3.13 (m, 3H), 2.71 (dd, *J* = 10.9, 6.3, 1H), 2.28 (t, *J* = 17.7, 1H), 2.13-2.02 (m, 2H), 2.01-1.92 (m, 2H).

### EXAMPLE 447

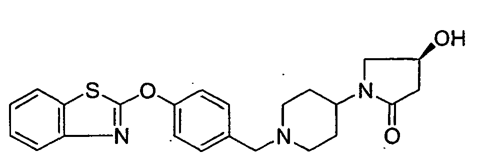

1-{1-[4-(Benzothiazo)-2-yloxy)-benzyl]-piperidin-4-yl}-4-hydroxy-pyrrolidin-2-one.

The title compound was prepared from EXAMPLE 253, step D following the procedure for EXAMPLE 254, step D. MS (ESI): mass calculated for C₂₃H₂₅N₃O₃S, 423.16; m/z found, 424.3 [M+H]⁺. ¹H NMR (400 MHz, CD₃OD): 7.80 (d, *J* = 8.1, 1H), 7.68-7.62 (m, 3H), 7.53-7.49 (m, 2H), 7.42 (t, *J* = 8.1, 1H), 7.32 (t, *J* = 8.3, 1H), 4.44 (t, *J* = 6.3, 1H), 4.38 (s, 2H), 4.25-4.15 (m, 1H), 3.68-3.57 (m, 3H), 3.33-3.13 (m, 3H), 2.71 (dd, J = 10.9, 6.3, 1H), 2.28 (t, *J* = 17.7, 1H), 2.13-2.02 (m, 2H), 2.01-1.92 (m, 2H).

### EXAMPLE 448

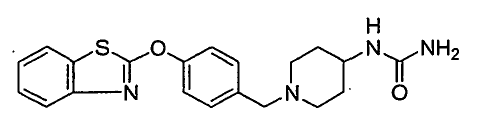

{1-[4-(Benzothiazol-2-yloxy)-benzyl]-piperidin-4-yl}-urea.

The title compound was prepared according to the procedure for EXAMPLE 253, step C using trimethylsilyl isocyanate: MS (ESI): mass calculated for C₂₀H₂₂N₄O₂S, 382.15; m/z found, 383.3 [M+H]⁺. ¹H NMR (400 MHz, CDCl₃): 7.71 (d, *J* = 7.8, 1H), 7.63 (d, *J* = 7.8, 1H), 7.40-7.33 (m, 3H), 7.30-7.21 (m, 3H), 5.82 (d, *J* = 7.8, 1H), 5.10 (d, *J* = 4.6, 1H), 3.84 (s, 1H), 3.56 (br s, 1H), 3.49 (s, 2H), 2.81 (d, *J* = 11.6, 2H), 2.12 (t, *J* = 11.1, 2H), 1.90 (d, *J* = 11.9, 2H), 1.51-1.39 (m, 2H).

### EXAMPLE 449

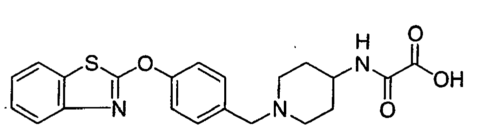

N-{1-[4-(Benzothiazol-2-yloxy)-benzyl]-piperidin-4-yl}-oxalamic acid.

The title compound was prepared from EXAMPLE 443 following the procedure for EXAMPLE 433, step C. MS (ESI): mass calculated for C₂₁H₂₁N₃O₄S, 411.13; m/z found, 412.3 [M+H]⁺. ¹H NMR (400 MHz, CD₃OD): 7.78 (d, *J* = 8.1, 1H), 7.64 (d, *J* = 8.1, 1H), 7.55-7.50 (m, 2H), 7.44-7.36 (m, 3H), 7.31 (t, *J* = 8.1, 1H), 3.84 (s, 2H), 3.81-3.72 (m, 1H), 3.11 (d, *J* = 10.9, 2H), 2.59-2.47 (m, 2H), 2.00-1.91 (m, 2H), 1.79-1.66 (m, 2H).

### EXAMPLE 450

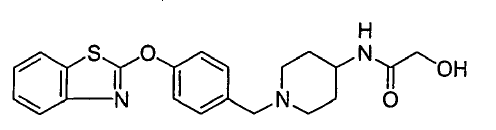

N-{1-[4-(Benzothiazol-2-yloxy)-benzyl]-piperidin-4-yl}-2-hydroxy-acetamide.

The title compound was prepared according to the procedure for EXAMPLE 431 using glycolic acid. MS (ESI): mass calculated for C₂₁H₂₃N₃O₃S, 397.15; m/z found, 398.3 [M+H]⁺. ¹H NMR (400 MHz, CDCl₃): 7.71 (d, *J* = 7.8, 1H), 7.65 (d, *J* = 8.1, 1H), 7.40-7.35 (m, 3H), 7.31-7.23 (m, 3H), 6.83 (d, *J* = 8.1, 1H), 5.33, (br s, 1H), 3.99 (s, 2H), 3.87-3.75 (m, 1H), 3.50 (s, 2H), 2.85 (d, *J* = 11.4, 2H), 2.14 (t, *J* = 10.9, 2H), 1.92 (d, *J* = 12.6, 2H), 1.56-1.43 (m, 2H).

### EXAMPLE 451

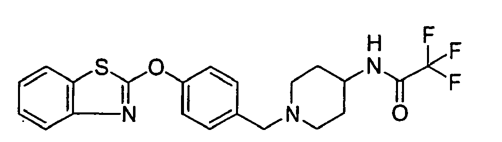

N-{1-[4-(Benzothiazol-2-yloxy)-benzyl]-piperidin-4-yl}-2,2,2-trifluoro-acetamide.

The title compound was prepared according to the procedure for EXAMPLE 431 using trifluoroacetic acid. MS (ESI): mass calculated for C₂₁H₂₀F₃N₃O₂S, 435.12; m/z found, 436.3 [M+H]⁺. ¹H NMR (400 MHz, CDCl₃): 7.71 (d, *J* = 8.3, 2H), 7.54 (d, *J* = 8.6, 2H), 7.47 (d, *J* = 8.8, 2H), 7.41 (t, *J* = 8.3, 1H), 7.34-7.28 (m, 1H), 4.24 (s, 2H), 4.15-4.02 (m, 1H), 3.59 (d, *J =* 12.1, 2H), 3.40, (br s, 1H), 2.87 (t, *J* = 11.9, 2H), 2.22-2.02 (m, 4H).

### EXAMPLE 452

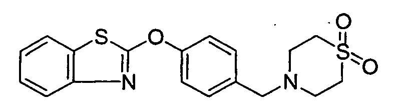

2-[4-(1,1-Dioxo-1|6-thiomorpholin-4-ylmethyl)-phenoxy]-benzothiazole.

The title compound was prepared according to the procedure for EXAMPLE 251 using thiomorpholine -1,1-dioxide. MS (ESI): mass calculated for C₁₈H₁₈N₂O₃S₂, 374.08; m/z found, 375.2 [M+H]⁺. ¹H NMR (400 MHz, CDCl₃): 7.72 (d, *J* = 8.1, 1H), 7.67 (d, *J* = 8.1, 1H), 7.42-7.31 (m, 5H), 7.27 (t, *J* = 7.8, 1H), 3.66 (s, 2H), 3.03 (d, *J* = 26.3, 8H).

### EXAMPLE 453

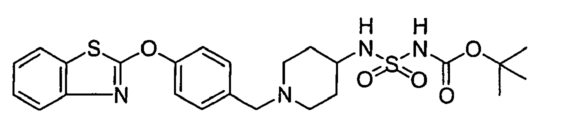

N-{1-[4-(benzothiazol-2-yloxy)-benzyl]-piperidin-4-amino sulfonyl)-carbamic acid tert-butyl ester

The title compound was prepared according to the procedure for EXAMPLE 253 using carbamic acid, N-(sulfonyl chloride) tert butyl ester. MS (ESI): mass calculated for C₂₄H₃₀N₄O₅S₂, 518.17; m/z found, 519.5 [M+H]⁺. ¹H NMR (400 MHz, CDCl₃): 7.73 (d, *J* = 8.2, 1H), 7.67 (d, *J* = 8.2, 1H), 7.42-7.35 (m, 3H), 7.34-7.24 (m, 3H), 5.18 (br s, 1H), 3.55 (s, 2H), 3.39-3.29 (m, 1H), 2.83 (d, *J* = 11.7, 2H), 2.31-2.18 (m, 2H), 1.98 (d, *J* = 11.7, 2H), 1.72-1.59 (m, 2H), 1.47 (s, 9H).

### EXAMPLE 454

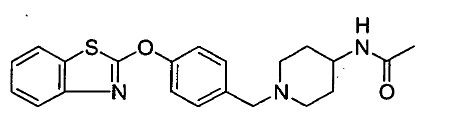

N-{1-[4-(Benzothiazol-2-yloxy)-benzyl]-piperidin-4-yl}-acetamide.

The title compound was prepared according to the procedure for EXAMPLE 253, step C using acetyl chloride. MS (ESI): mass calculated for C₂₁H₂₃N₃O₂S, 381.15; m/z found, 382.4 [M+H)⁺. ¹H NMR (400 MHz, CDCl₃): 7.73 (d, *J* = 7.8, 1H), 7.67 (d, *J* = 8.1, 1H), 7.43-7.36 (m, 3H), 7.33-7.24 (m, 3H), 5.65 (br s, 1H), 3.87-3.76 (m, 1H), 3.54 (s, 2H), 2.86 (d, *J* = 12.1, 2H), 2.17 (t, *J* = 11.4, 2H), 1.97 (s, 3H), 1.93 (d, *J* = 11.9, 2H), 1.56-1.44 (m, 2H).

### EXAMPLE 455

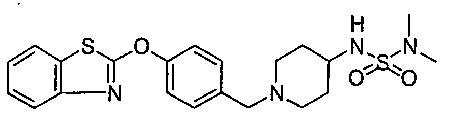

N-{1-[4-(Benzothiazol-2-yloxy)-benzyl]-piperidin-4-yl}-N,N-dimethylsulfamide

The title compound was prepared according to the procedure for EXAMPLE 253, step C using N,N-dimethyl-sulfamoyl chloride. MS (ESI): mass calculated for C₂₁H₂₆N₄O₃S₂, 446.14; m/z found, 447.4 [M+H]⁺. ¹H NMR (400 MHz, CDCl₃): 7.73 (d, *J* = 8.1, 1H), 7.66 (d, *J* = 8.1, 1H), 7.41-7.35 (m, 3H), 7.32-7.23 (m, 3H), 4.37 (d, *J* = 7.8, 1H), 3.49 (s, 2H), 3.28-3.17 (m, 1H), 2.81 (d, *J* = 10.9, 2H), 2.78 (s, 6H), 2.11 (t, *J* = 11.1, 2H), 1.98 (d, *J* = 10.9, 2H), 1.61-1.50 (m, 2H).

### EXAMPLE 456

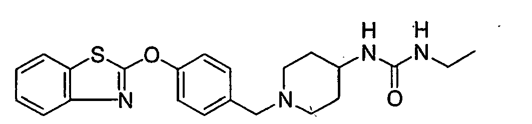

1-{1-[4-(Benzothiazol-2-yloxy)-benzyl]-piperidin-4-yl}-3-ethyl-urea.

The title compound was prepared according to the procedure for EXAMPLE 253, step C using ethyl isocyanate. MS (ESI): mass calculated for C₂₂H₂₆N₄O₂S, 410.18; m/z found, 411.5 [M+H]⁺. ¹H NMR (400 MHz, CDCl₃): 7.72 (d, *J* = 8.1, 1H), 7.66 (d, *J* = 8.1, 1H), 7.42-7.34 (m, 3H), 7.32-7.23 (m, 3H), 4.54 (br s, 1H), 4.45 (d, *J* = 7.6, 1H), 3.67-3.55 (m, 1H), 3.50 (s, 2H), 3.23-3.14 (m, 2H), 2.80 (d, *J* = 11.9, 2H), 2.13 (t, *J* = 11.4, 2H), 1.93 (d, *J* = 12.6, 2H), 1.48-1.36 (m, 2H), 1.12 (t, *J* = 7.3, 3H).

### EXAMPLE 457

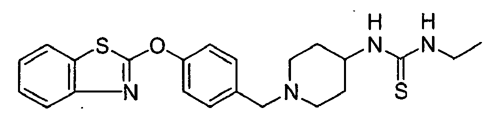

1-{1-[4-(Benzothiazol-2-yloxy)-benzyl]-piperidin-4-yl}-3-ethyl-thiourea.

The title compound was prepared according to the procedure for EXAMPLE 253, step C using ethyl isothiocyanate. MS (ESI): mass calculated for C₂₂H₂₆N₄OS₂, 426.15; m/z found, 427.4 [M+H]⁺. ¹H NMR (400 MHz, CDCl₃): 7.71 (d, *J* = 7.8, 1H), 7.67 (d, *J* = 8.1, 1H), 7.41-7.34 (m, 3H), 7.32-7.24 (m, 3H), 6.03 (br s, 1H), 5.74 (br s, 1H), 4.20-4.00 (m, 1H), 3.51 (s, 2H), 3.47-3.35 (m, 2H), 2.82 (d, *J* = 11.6, 2H), 2.05 (t, *J* = 11.1, 2H), 2.05 (d, *J* = 11.9, 2H), 1.57-1.45(m, 2H), 1.21 (t, *J* = 7.3, 3H).

### EXAMPLE 458

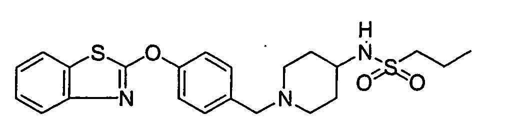

Propane-1-sulfonic acid {1-[4-(benzothiazol-2-yloxy)-benzyl]-piperidin-4-yl}-amide.

The title compound was prepared according to the procedure for EXAMPLE 253, step C using 1-propanesulfonyl chloride. MS (ESI): mass calculated for C₂₂H₂₇N₃O₃S₂, 445.15; m/z found, 446.4 [M+H]⁺. ¹H NMR (400 MHz, CDCl₃): 7.73 (d, *J*= 8.1, 1H), 7.65 (d, *J* = 8.1, 1H), 7.40-7.34 (m, 3H), 7.32-7.22 (m, 3H), 4.85 (d, *J* = 8.1, 1H), 3.48 (s, 2H), 3.36-3.24 (m, 1H), 3.02-2.94 (m, 2H), 2.81 (d, *J* = 11.9, 2H), 2.11 (t, *J* = 10.6, 2H), 1.95 (d, *J* = 12.6, 2H), 1.89-1.78 (m, 2H), 1.65-1.54 (m, 2H), 1.04 (t, *J* = 7.3, 3H).

### EXAMPLE 459

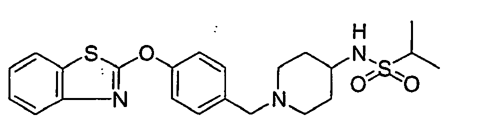

Propane-2-sulfonic acid {1-[4-(benzothiazol-2-yloxy)-benzyl]-piperidin-4-yl}-amide.

The title compound was prepared according to the procedure for EXAMPLE 253, step C using 2-propanesulfonyl chloride. MS (ESI): mass calculated for C₂₂H₂₇N₃O₃S₂, 445.15; m/z found, 446.4 [M+H]⁺. ¹H NMR (400 MHz, CDCl₃): 7.73 (d, *J* = 8.1, 1H), 7.66 (d, *J* = 8.1, 1H), 7.41-7.35 (m, 3H), 7.32-7.24 (m, 3H), 3.79 (d, *J* = 5.6, 1H), 3.51 (s, 2H), 3.38-3.27 (m, 1H), 2.89-2.78 (m, 2H), 2.19-1.94 (m, 5H), 1.76 (d, *J* = 30.8, 6H), 1.69-1.55 (m, 2H).

### EXAMPLE 460

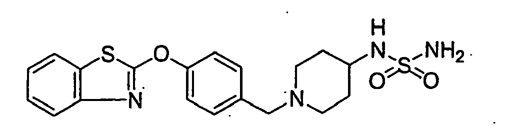

N-{1-[4-(Benzothiazol-2-yloxy)-benzyl]-piperidin-4-yl}-sulfamide

The title compound was prepared from EXAMPLE 453 following the procedure for EXAMPLE 253, step B. MS (ESI): mass calculated for C₁₉H₂₂N₄O₃S₂, 418.11; m/z found, 419.4 [M+H]⁺. ¹H NMR (400 MHz, CDCl₃): 10.68 (br s, 1H), 7.60 (d, *J* = 8.3, 1H), 7.56 (d, *J* = 8.3, 1H), 7.48-7.38 (m, 2H), 7.32-7.24 (m, 3H), 7.18 (t, *J* = 7.8, 1H), 6.24 (br s, 2H), 4.10 (s, 2H), 3.80-3.66 (m, 1H), 3.57-3.10 (m, 2H), 3.04-2.61 (m, 2H), 2.41-1.61 (m, 4H).

### EXAMPLE 461

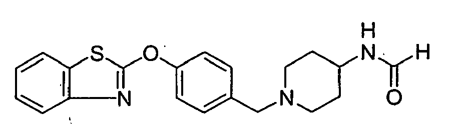

N-{1-[4-(Benzothiazol-2-yloxy)-benzyl]-piperidin-4-yl}-formamide.

The title compound was prepared according to the procedure for EXAMPLE 431, formic acid. MS (ESI): mass calculated for C₂₀H₂₁N₃O₂S, 367.14; m/z found, 368.4 [M+H]⁺. ¹H NMR (400 MHz, CDCl₃): 12.28 (br s, 1H), 7.73 (d, *J* = 7.8, 1H), 7.68 (d, *J* = 7.8, 1H), 7.47-7.24 (m, 6H), 6.33 (d, *J* = 7.3, 1H), 4.05-3.94 (m, 1H), 3.80 (s, 2H), 3.14 (d, *J* = 11.6, 2H), 2.41 (t, *J* = 11.9, 2H), 1.99 (d, *J* = 13.1, 2H), 1.83-1.70 (m, 2H).

### EXAMPLE 462

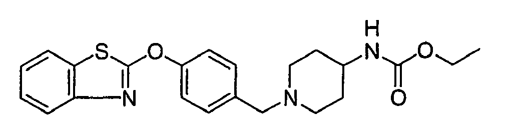

{1-[4-(Benzothiazol-2-yloxy)-benzyl]-piperidin-4-yl}-carbamic acid ethyl ester.

The title compound was prepared according to the procedure for EXAMPLE 253, step C using ethyl chloroformate. MS (ESI): mass calculated for C₂₂H₂₅N₃O₃S, 411.16; m/z found, 412.4 [M+H]⁺. ¹H NMR (400 MHz, CDCl₃): 7.73 (d, *J* = 8.1, 1H), 7.66 (d, *J* = 8.1, 1H), 7.41-7.34 (m, 3H), 7.32-7.22 (m, 3H), 4.62 (br s, 1H), 4.10 (dd, *J* = 7.3, 6.8, 2H), 3.59-3.51 (m, 1H), 3.49 (s, 2H), 2.80 (d, *J* = 11.6, 2H), 2.12 (t, *J* = 11.6, 2H), 1.93 (d, *J* = 12.1, 2H), 1.51-1.39 (m, 2H), 1.23 (t, *J* = 7.1, 3H).

### EXAMPLE 463

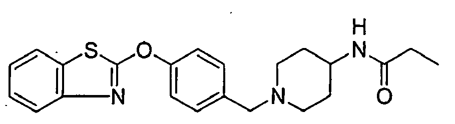

N-{1-[4-(Benzothiazol-2-yloxy)-benzyl]-piperidin-4-yl}-propionamide.

The title compound was prepared according to the procedure for EXAMPLE 253, step C using propionyl chloride. MS (ESI): mass calculated for C₂₂H₂₅N₃O₂S, 395.17; m/z found, 396.4 [M+H]⁺. ¹H NMR (400 MHz, CDCl₃): 7.72 (d, *J* = 8.1, 1H), 7.65 (d, *J* = 8.1, 1H), 7.41-7.34 (m, 3H), 7.32-7.22 (m, 3H), 5.61 (d, *J* = 8.1, 1H), 3.87-3.76 (m, 1H), 3.49 (s, 2H), 2.82 (d, *J* = 11.9, 2H), 2.22-2.07 (m, 4H), 1.90 (d, *J* = 13.1, 2H), 1.51-1.39 (m, 2H), 1.14 (t, *J* = 7.6, 3H).

### EXAMPLE 464

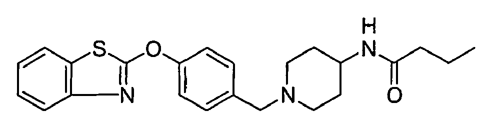

N-{1-[4-(Benzothiazol-2-yloxy)-benzyl)-piperidin-4-yl}-butyramide.

The title compound was prepared according to the procedure for EXAMPLE 253, step C using butyryl chloride. MS (ESI): mass calculated for C₂₃H₂₇N₃O₂S, 409.18; m/z found, 410.4 [M+H]⁺. ¹H NMR (400 MHz, CDCl₃): 7.72 (d, *J* = 8.1, 1H), 7.65 (d, *J* = 8.1, 1H), 7.40-7.35 (m, 3H), 7.31-7.23 (m, 3H), 5.64 (d, *J* = 8.1, 1H), 3.87-3.77 (m, 1H), 3.49 (s, 2H), 2.81 (d, *J* = 11.6, 2H), 2.16-2.07 (m, 4H), 1.91 (d, *J* = 12.9, 2H), 1.71-1.60 (m, 2H), 1.51-1.39 (m, 2H), 0.93 (t, *J* = 7.3, 3H).

### EXAMPLE 465

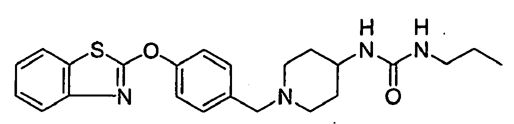

1-{1-[4-(Benzothiazol-2-yloxy)-benzyl]-piperidin-4-yl}-3-propyl-urea.

The title compound was prepared according to the procedure for EXAMPLE 253, step C using propyl isocyanate. MS (ESI): mass calculated for C₂₃H₂₈N₄O₂S, 424.19; m/z found, 425.5 [M+H]⁺. ¹H NMR (400 MHz, CDCl₃): 7.70 (d, *J* = 8.1, 1H), 7.63 (d, *J* = 8.1, 1H), 7.39-7.33 (m, 3H), 7.28-7.21 (m, 3H), 5.54 (br s, 1H), 5.37 (d, *J* = 8.1, 1H), 3.66-3.56 (m, 1H), 3.48 (s, 2H), 3.11 (dd, *J* = 7.1, 6.8, 2H), 2.80 (d, *J* = 11.1, 2H), 2.11 (t, *J* = 11.1, 2H), 1.91 (d, *J* = 11.1, 2H), 1.54-1.38 (m, 4H), 0.91 (t, *J* = 7.3, 3H).

### EXAMPLE 466

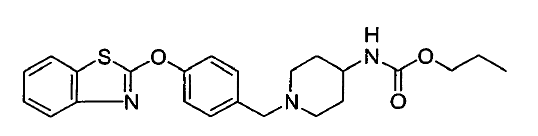

{1-[4-(Benzothiazol-2-yloxy)-benzyl]-piperidin-4-yl}-carbamic acid propyl ester.

The title compound was prepared according to the procedure for EXAMPLE 253, step C using propyl chloroformate. MS (ESI): mass calculated for C₂₃H₂₇N₃O₃S, 425.18; m/z found, 426.4 [M+H]⁺. ¹H NMR (400 MHz, CDCl₃): 7.72 (d, *J* = 8.1, 1H), 7.63 (d, *J* = 8.1, 1H), 7.39-7.33 (m, 3H), 7.31-7.21 (m, 3H), 4.83 (d, *J* = 7.6, 1H), 4.00 (t, *J* = 6.6, 2H), 3.58-3.49 (m, 1H), 3.47 (s, 2H), 2.79 (d, *J* = 11.6, 2H), 2.10 (t, *J* = 10.9, 2H), 1.91 (d, *J* = 11.1, 2H), 1.67-1.56 (m, 2H), 1.52-1.40 (m, 2H), 0.92 (t, *J* = 7.6, 3H).

### EXAMPLE 467

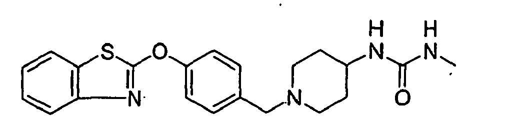

1-{1-[4-(Benzothiazol-2-yloxy)-benzyl]-piperidin-4-yl}-3-methyl-urea.

The title compound was prepared according to the procedure for EXAMPLE 253, step C using methyl isocyanate. MS (ESI): mass calculated for C₂₁H₂₄N₄O₂S, 396.16; m/z found, 397.4 [M+H]⁺. ¹H NMR (400 MHz, CDCl₃): 7.71 (d, *J* = 8.1, 1H), 7.65 (d, *J* = 8.1, 1H), 7.45-7.34 (m, 3H), 7.33-7.22 (m, 3H), 5.71 (br s, 2H), 3.69-3.59 (m, 1H), 3.56 (s, 2H), 2.87 (d, *J* = 11.9, 2H), 2.73 (d, *J* = 4.8, 3H), 2.21 (t, *J* = 10.1, 2H), 1.93 (d, *J* = 10.4, 2H), 1.58-1.45 (m, 2H).

### EXAMPLE 469

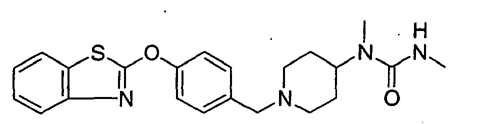

1-{1-[4-(Benzothiazol-2-yloxy)-benzyl]-piperidin-4-yl}-1,3-dimethyl-urea.

The title compound was prepared according to the procedure for EXAMPLE 255, step C using methyl isocyanate. MS (ESI): mass calculated for C₂₂H₂₆N₄O₂S, 410.18; m/z found, 411.4 [M+H]⁺. ¹H NMR (400 MHz, CDCl₃): 7.72 (d, *J* = 8.1, 1H), 7.65 (d, *J* = 8.1, 1H), 7.41-7.34 (m, 3H), 7.31-7.22 (m, 3H), 4.63 (dd, *J* = 4.6, 4.6, 1H), 4.21-4.11 (m, 1H), 3.49 (s, 2H), 2.93 (d, *J* = 11.9, 2H), 2.79 (d, *J* = 4.8, 3H), 2.71 (s, 3H), 2.08 (t, *J* = 11.9, 2H), 1.76-1.64 (m, 2H), 1.63-1.54 (m, 2H).

### EXAMPLE 470

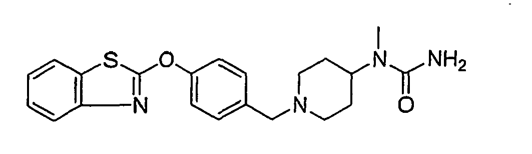

1-{1-[4-(Benzothiazol-2-yloxy)-benzyl]-piperidin-4-yl}-1-methyl-urea.

The title compound was prepared according to the procedure for EXAMPLE 255, step C using trimethylsilyl isocyanate. MS (ESI): mass calculated for C₂₁H₂₄N₄O₂S, 396.16; m/z found, 397.4 [M+H]⁺. ¹H NMR (400 MHz, CDCl₃): 7.72 (d, *J* = 8.1, 1H), 7.65 (d, *J* = 8.1, 1H), 7.42-7.34 (m, 3H), 7.32-7.21 (m, 3H), 4.93 (s, 2H), 4.16-4.03 (m, 1H), 3.50 (s, 2H), 2.94 (d, *J* = 11.4, 2H), 2.76 (s, 3H), 2.09 (t, *J* = 11.4, 2H), 1.79-1.66 (m, 2H), 1.65-1.56 (m, 2H).

### EXAMPLE 471

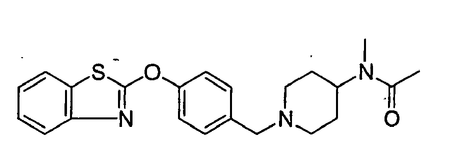

N-{1-[4-(Benzothiazol-2-yloxy)-benzyl]-piperidin-4-yl}-N-methyl-acetamide.

The title compound was prepared according to the procedure for EXAMPLE 255, step C using acetyl chloride. MS (ESI): mass calculated for C₂₂H₂₅N₃O₂S, 395.17; m/z found, 396.4 [M+H]⁺. ¹H NMR (400 MHz, CDCl₃): 7.72 (d, *J* = 8.1, 1H), 7.65 (d, *J* = 8.1, 1H), 7.41-7.34 (m, 3H), 7.31-7.22 (m, 3H), 4.55-4.44 (m, 1H), 3.49 (s, 2H), 3.01-2.90 (m, 2H), 2.84 (s, 3H), 2.15-2.09 (m, 2H), 2.07 (s, 3H), 1.77-1.65 (m, 2H), 1.64-1.53 (m, 2H).

### EXAMPLE 472

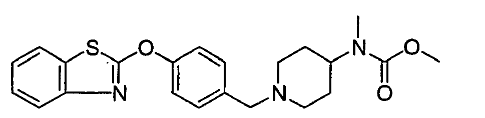

{1-[4-(Benzothiazol-2-yloxy)-benzyl]-piperidin-4-yl}-methyl-carbamic acid methyl ester.

The title compound was prepared according to the procedure for EXAMPLE 255, step C using methyl chloroformate. MS (ESI): mass calculated for C₂₁H₂₅N₃O₃S, 411.16; m/z found, 412.3 [M+H]⁺. ¹H NMR (400 MHz, CDCl₃): 7.73 (d, *J* = 8.1, 1H), 7.66 (d, *J* = 8.1, 1H), 7.42-7.35 (m, 3H), 7.33-7.24 (m, 3H), 4.16-3.96 (m, 1H), 3.69 (s, 3H), 3.51 (s, 2H), 2.95 (d, *J* = 11.4, 2H), 2.78 (s, 3H), 2.08 (t, *J* = 10.9, 2H), 1.82-1.69 (m, 2H), 1.65-1.57 (m, 2H).

### EXAMPLE 473

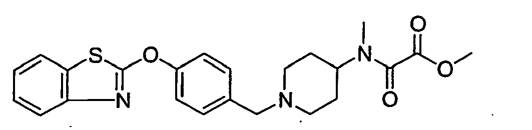

N-{1-[4-(Benzothiazol-2-yloxy)-benzyl]-piperidin-4-yl}-N-methyl-oxalamic acid methyl ester.

The title compound was prepared according to the procedure for EXAMPLE 255, step C using chloro-oxo-acetic acid methyl ester. MS (ESI): mass calculated for C₂₃H₂₅N₃O₄S, 439.16; m/z found, 440.4 [M+H]⁺. ¹H NMR (400 MHz, CDCl₃): 7.73 (d, *J* = 8.1, 1H), 7.66 (d, *J* = 8.1, 1H), 7.41-7.34 (m, 3H), 7.33-7.23 (m, 3H), 4.41-4.32 (m, 1H), 3.87 (s, 3H), 3.51 (s, 2H), 2.97 (d, *J* = 11.9, 2H), 2.88 (s, 3H), 2.13 (t, *J* = 11.9, 2H), 1.95-1.83 (m; 2H), 1.72-1.62 (m, 2H).

### EXAMPLE 474

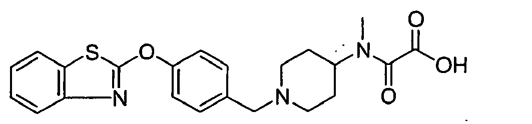

N-{1-[4-(Benzothiazol-2-yloxy)-benzyl]-piperidin-4-yl}-N-methyl-oxalamic acid.

The title compound was prepared from EXAMPLE 473 following the procedure for EXAMPLE 433, step C. MS (ESI): mass calculated for C₂₂H₂₃N₃O₄S, 425.14; m/z found, 426.3 [M+H]⁺. ¹H NMR (400 MHz, CDCl₃): 7.68 (d, *J* = 8.1, 1H), 7.59 (d, *J* = 8.1, 1H), 7.37-7.28 (m, 3H), 7.27-7.16 (m, 3H), 4.27-4.16 (m, 1H), 3.43 (s, 2H), 2.93 (d, *J* = 11.897, 2H), 2.75 (s, 3H), 2.01 (t, *J* = 11.9, 2H), 1.82-1.65 (m, 2H), 1.58-1.47 (m, 2H).

### EXAMPLE 475

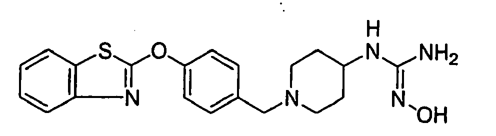

Guanidine, N-{1-[4-(Benzothiazol-2-yloxy)-benzyl]-piperidin-4-yl}-N'-hydroxy
A. 1-[4-(Benzothiazol-2-yloxy)-benzyl]-piperidin-4-yl-cyanamide. To a suspension of 1-[4-(benzothiazol-2-yloxy)-benzyl]-piperidin-4-ylamine (339 mg, 1.0 mmol) in CH₃OH (2.0 mL) was added sodium acetate (180 mg, 2.2 mmol) at room temperature. The suspension became a clear solution. Cooled the solution to 0 °C. Cyanogen bromide (159 mg, 1.5 mmol) in CH₃OH (0.7 mL) was added dropwise. The reaction mixture was stirred at 5 °C for 2 h and then at ambient temperature overnight. The mixture was filtered through a frit fnnel and the filtrate was absorbed onto silica gel (40 g) and purified (0-10% CH₃OH/CH₂Cl₂) to give an unstable oil (123 mg, 34% yield). MS (ESI): mass calculated for C₂₀H₂₀N₄OS, 364.14; m/z found, 365.3 [M+H]⁺.
B. Guanidine, N-{1-[4-(Benzothiazol-2-yloxy)-benzyl]-piperidin-4-yl}-N'-hydroxy. To a solution of 1-[4-(benzothiazol-2-yloxy)-benzyl]-piperidin-4-yl-cyanamide (123 mg, 0.34 mmol) in DMF (2 mL) was added hydroxylamine hydrochloride (41.5 mg, 0.60 mmol), followed by sodium carbonate (119 mg, 1.12 mmol) in small portions. The reaction mixture was stirred at room temerature for 2 h and partitioned between EtOAc (20 mL) and H₂O (20 mL). The organic phase was washed with brine, dried and concentrated under reduced pressure to yield the crude product as a pale solid. The crude product was purified on SiO₂ (12 g; 0-10% CH₃OH/CH₂Cl₂) to give a white soid (117 mg, 86% yield). MS (ESl): mass calculated for C₂₀H₂₃N₅O₄S, 397.16; m/z found, 398.4 [M+H]⁺. ¹H NMR (400 MHz, CDCl₃): 7.71 (d, *J* = 8.1, 1H), 7.63 (d, *J* = 8.1, 1H), 7.38-7.32 (m, 3H), 7.28-7.21 (m, 3H), 4.69 (br s, 2H), 3.46 (s, 2H), 3.27-3.16 (m, 1H), 2.78 (d, *J* = 10.9, 2H), 2.04 (t, *J*= 11.1, 2H), 1.92 (d, *J*= 11.1, 2H), 1.51-1.38 (m, 2H).

### EXAMPLE 476

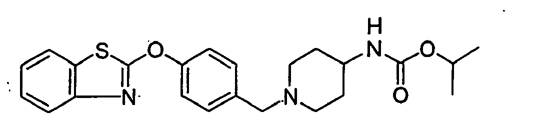

{1-[4-(Benzothiazol-2-yloxy)-benzyl]-piperidin-4-yl}-carbamic acid isopropyl ester.

The title compound was prepared according to the procedure for EXAMPLE 253, step C using isopropyl isocyanate. MS (ESI): mass calculated for C₂₃H₂₇N₃O₃S, 425.18; m/z found; 426.4 [M+H]⁺. ¹H NMR (400 MHz, CDCl₃): 7.72 (d, *J* = 8.1, 1H), 7.63 (d, *J* = 8.1, 1H), 7.38-7.33 (m, 3H), 7.31-7.21 (m, 3H), 4.95-4.85 (m, 1H), 4.75 (d, *J* = 7.1, 1H), 3.58-3.49 (m, 1H), 3.47 (s, 2H), 2.79 (d, *J* = 11.6, 2H), 2.09 (t, *J* = 11.4, 2H), 1.91 (d, *J* = 11.9, 2H), 1.50-1.39 (m, 2H), 1.21 (d, *J* = 6.5, 6H).

### EXAMPLE 477

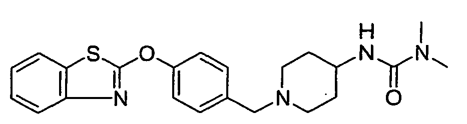

3-{1-[4-(Benzothiazol-2-yloxy)-benzyl]-piperidin-4-yl}-1,1-dimethyl-urea.

The title compound was prepared according to the procedure for EXAMPLE 253, step C using N,N-dimethylcarbamoyl chloride. MS (ESI): mass calculated for C₂₂H₂₆N₄O₂S, 410.18; m/z found, 411.4 [M+H]⁺. ¹H NMR (400 MHz, CDCl₃): 7.71 (d, *J* = 8.1, 1H), 7.64 (d, *J* = 8.1, 1H), 7.39-7.33 (m, 3H), 7.30-7.21 (m, 3H), 4.41 (d, *J* = 7.8, 1H), 3.73-3.62 (m, 1H), 3.48 (s, 2H), 2.87 (s, 6H), 2.81 (d, *J* = 11.6, 2H), 2.11 (t, *J* = 11.9, 2H), 1.93 (d, *J* = 11.6, 2H), 1.50-1.38 (m, 2H).

### EXAMPLE 478

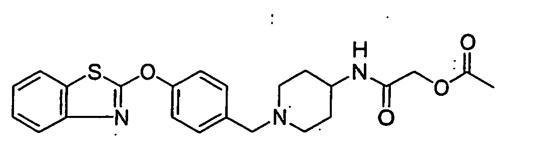

Acetic acid {1-[4-(benzothiazol-2-yloxy)-benzyl]-piperidin-4-ylcarbamoyl}-methyl ester.

The title compound was prepared according to the procedure for EXAMPLE 253, step C using acetic acid chlorocarbonyl methyl ester. MS (ESI): mass calculated for C₂₃H₂₅N₃O₄S, 439.16; m/z found, 440.4 [M+H]⁺. ¹H NMR (400 MHz, CDCl₃): 7.72 (d, *J* = 7.8, 1H), 7.65 (d, *J* = 8.1, 1H), 7.40-7.35 (m, 3H), 7.32-7.23 (m, 3H), 6.11 (d, *J* = 8.3, 1H), 4.53, (s, 2H), 3.93-3.82 (m, 1H), 3.50 (s, 2H), 2.83 (d, *J* = 11.9, 2H), 2.15 (s, 3H), 2.14 (t, *J* = 11.9, 2H), 1.93 (d, *J* = 12.1, 2H), 1.56-1.45 (m, 2H).

### EXAMPLE 479

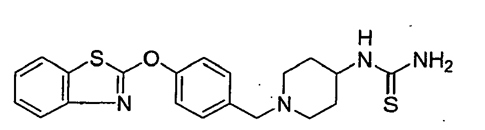

{1-[4-(Benzothiazol-2-yloxy)-benzyl]-piperidin-4-yl}-thiourea.
A. 1-{1-[4-(Benzothiazol-2-yloxy)-benzyl]-piperidin-4-yl}-3-benzoyl-thiourea. The title compound was prepared according to the procedure for EXAMPLE 253, step C using benzoyl isocyanate. MS (ESI): mass calculated for C₂₇H₂₆N₄O2S₂, 502.15; m/z found, 503.3 [M+H]⁺. ¹H NMR (400 MHz, CDCl₃): 10.80 (d, *J* = 8.1, 1H), 9.06 (s, 1H), 7.81 (d, *J* = 8.3, 2H), 7.72 (d, *J* = 8.3, 1H), 7.65 (d, *J* = 7.8, 1H), 7.59 (t, *J* = 7.6, 1H), 7.50-7.45 (m, 2H), 7.42-7.34 (m, 3H), 7.33-7.22 (m, 3H), 4.40-4.29 (m, 1H), 3.53 (s, 2H), 2.80 (d, *J* = 11.1, 2H), 2.26 (t, *J* = 10.1, 2H), 2.12 (d, *J* = 11.9, 2H), 1.76-1.64 (m, 2H).
B. {1-[4-(Benzothiazol-2-yloxy)-benzyl]-piperidin-4-yl}-thiourea. To 1-{1-[4-(benzothiazol-2-yloxy)-benzyl]-piperidin-4-yl}-3-benzoyl-thiourea (300 mg, 0.60 mmol) was added 10% aqueous sodium hydroxide (10 mL). The reaction mixture was heated at reflux for 1 h. The resulting white suspension was cooled to room temperature and extracted with 10% CH₃OH/CH₂Cl₂ (3 x 50 mL). The organic layer was dried (Na₂SO₄) and concentrated under reduced pressure to yield the crude product as an off-white solid. The crude product was purified on SiO₂ (40 g; 0-10% CH₃OH/CH₂Cl₂) to give the title compound as a white solid (219 mg, 92% yield). MS (ESI): mass calculated for C₂₀H₂₂N₄OS₂, 398.12; m/z found, 399.3 [M+H]⁺. ¹H NMR (400 MHz, CDCl₃): 7.72 (d, *J* = 8.1, 1H), 7.68 (d, *J* = 8.1, 1H), 7.42-7.36 (m, 3H), 7.34-7.26 (m, 3H), 6.09 (m, 1H), 4.29-4.02 (m, 1H), 3.57 (s, 2H), 2.88 (d, *J* = 11.1, 2H), 2.29 (s, 2H), 2.20 (t, *J* = 11.1, 2H), 2.04 (d, *J* = 10.1, 2H), 1.60-1.45 (m, 2H).

### Assay Methods

Assay results providd herein are illustrative results of the assays that were performed for compounds of this invention.

### Recombinant Human LTA4 Hydrolase Assay for LTA4 Hydrolase Inhibitor Activity

Compounds of the present invention were tested for LTA4 hydrolase inhibitor activity against recombinant human LTA4 hydrolase (rhLTA4H). Vectors were prepared and used to express rhLTA4H essentially as follows: LTA4 hydrolase encoding DNA was amplified by polymerase chain reaction (PCR) using a human placental cDNA library as a template. Oligonucleotide primers for the PCR reaction were based on the 5'-end, and the complement of the 3'-end, of the published nucleotide sequence for the coding region of the human LTA4 hydrolase gene (C.D. Funk et al., Proc. Natl. Acad. Sci. USA 1987, 84:6677-6681). The amplified 1.9 kb DNA fragment encoding LTA4 hydrolase was isolated and cloned into the pFastBac1 vector (Invitrogen). Recombinant baculovirus was generated as described by the manufacturer, and used to infect Spodoptera frugiperda (Sf-9) cells. Recombinant LTA4 hydrolase enzyme was purified from the infected Sf-9 cells essentially as described by J.K. Gierse et al. (Protein Expression and Purification 1993, 4:358-366). The purified enzyme solution was adjusted to contain 0.29 mg/mL LTA4 hydrolase, 50 mM Tris (pH 8.0), 150 mM NaCl, 5 mM dithiothreitol, 50% glycerol, and EDTA-free Complete protease inhibitor cocktail (Roche). The specific activity of the enzyme was about 3.8 µmol/min/mg.

LTA4 substrate was prepared from the methyl ester of LTA4 (Cayman Chemical) by treatment with 67 equiv of NaOH under nitrogen at room temperature for 40 min. The LTA4 substrate in its free acid form was kept frozen at -80 °C until needed. Each compound was diluted to different concentrations in assay buffer (0.1 M potassium phosphate (pH 7.4), 5 mg/mL fatty acid free BSA) containing 10% DMSO. A 25-uL aliquot of each compound dilution was incubated for 10 min at room temperature with an equal volume of assay buffer containing 36 ng of recombinant human LTA4H. The solution was then adjusted to 200 µL with assay buffer. LTA4,(free acid) was thawed and diluted in assay buffer to a concentration of 357 ng/mL, and 25 µL (9 ng) of LTA4 substrate was added to the reaction mixture (total volume = 225 µL) at time zero. Each reaction was carried out at room temperature for 10 min. The reaction was stopped by diluting 10 µL of the reaction mixture with 200 µL of assay buffer. LTB4 was quantified in the diluted sample by a commercially available enzyme-linked immunoassay (Cayman Chemical Co.), as recommended by the manufacturer. Positive controls, under essentially identical conditions but without addition of an inhibitor compound, and negative controls, containing all assay components except enzyme, were routinely run in each experiment. IC₅₀ values were determined by fitting the activity data at different compound concentrations to a 4-parameter equation using the Grafit program (Erithacus software).

The IC₅₀ values presented in the tables below should be expected to fall within the typical three-fold variability of assays of this type. The values presented here are, in general, an average of one to three determinations.

**Table 1**

| Example | IC₅₀ (nM) | Example | IC₅₀ (nM) | Example | IC₅₀ (nM) |
|---|---|---|---|---|---|
| **11** | 6 | **45** | 15 | **94** | 1 |
| **13** | 2 | **46** | 17 | **135** | 92 |
| **14** | 9 | **59** | 5 | **136** | 25 |
| **27** | 7 | **77** | 10 | **271** | 2 |
| **36** | 14 | **78** | 3 | **481** | 55 |
| **44** | 8 | **92** | 1 | **484** | 19 |

**Table 2**

| Example | IC₅₀ (nM) | Example | IC₅₀ (nM) | Example | IC₅₀ (nM) |
|---|---|---|---|---|---|
| **12** | 100 | **176** | 12 | **298** | 15 |
| **15** | 22 | **180** | 2 | **302** | 60 |
| **18** | 6 | **183** | 18 | **325** | 12 |
| **22** | 7 | **192** | 3 | **328** | 22 |
| **23** | 1 | **195** | 58 | **331** | 13 |
| **25** | 4 | **202** | 4 | **334** | 100 |
| **31** | 17 | **206** | 4 | **336** | 24 |
| **80** | 23 | **207** | 16 | **353** | 7 |
| **81** | 5 | **211** | 9 | **357** | 45 |
| **102** | 3 | **214** | 18 | **358** | 11 |
| **104** | 14 | **224** | 14 | **359** | 48 |
| **109** | 21 | **225** | 25 | **360** | 33 |
| **110** | 40 | **250** | 26 | **361** | 8 |
| **143** | 64 | **251** | 16 | **363** | 4 |
| **146** | 43 | **255** | 1 | **366** | 39 |
| **147** | 33 | **259** | 14 | **380** | 6 |
| **150** | 1 | **261** | 33 | **389** | 2 |
| **153** | 8 | **262** | 4 | **419** | 4 |
| **155** | 9 | **263** | 5 | **437** | 21 |
| **156** | 7 | **265** | 112 | **446** | 8 |
| **157** | 1 | **270** | 16 | **447** | 28 |
| **161** | 1 | **274** | 29 | **450** | 7 |
| **163** | 9 | **285** | 76 | **454** | 4 |
| **167** | 37 | **287** | 28 | **462** | 27 |
| **168** | 27 | **288** | 4 | **471** | 19 |
| **169** | 32 | **296** | 33 | **479** | 7 |
| **172** | 27 | **294** | 6 | **483** | 45 |

**Table 3**

| Example | IC₅₀ (nM) | Example | IC₅₀ (nM) |
|---|---|---|---|
| **37** | 10 | **134** | 110 |
| **123** | 4 | **230** | 36 |
| **127** | 29 | **231** | 18 |
| **132** | 139 | **485** | 39 |
| **133** | 111 | | |

### LTB4 Production bv Calcium lonophore-Stimulated Murine Blood for LTA4H Inhibitor Activity

CD-1 mice were sacrificed, and blood was collected in heparin-containing syringes by cardiac puncture. The blood was diluted 1:15 with RPMI-1640 medium, and 200-µL aliquots of the diluted blood were added to wells of a 96-well microtiter plate. LTA4H inhibitor test compounds were prepared at different concentrations in RPMI-1640 medium containing 1% DMSO, and 20 µL of each test solution was added to a well containing diluted whole blood (final DMSO concentration of 0.1%). After the microtiter plate contents were incubated for 15 min at 37 °C in a humidified incubator, calcium ionophore A23187 (Sigma Chemical Co., St. Louis, Mo.) was added to each sample well (final concentration = 20 ng/mL). The incubation was continued under the same conditions for an additional 10 min to allow LTB4 formation. The reaction was terminated by centrifugation (833 x g, 10 min at 4 °C), and supernatants were analyzed for LTB4 by a commercially available enzyme-linked immunoassay (Cayman Chemical Co.) according to the manufacturer's instructions. Positive controls, under essentially identical conditions but without addition of an inhibitor compound, and negative unstimulated controls, containing all assay components except calcium ionophore, were routinely run in each experiment. IC₅₀ values were determined by fitting the activity data at different compound concentrations to a 4-parameter equation using the Grafit program (Erithacus software).

**Table 4**

| Example | IC₅₀ (nM) | Example | IC₅₀ (nM) | Example | IC₅₀ (nM) |
|---|---|---|---|---|---|
| **11** | 44 | **27** | 143 | **94** | 72 |
| **13** | 41 | **44** | 23 | **481** | 264 |
| **14** | 89 | **46** | 29 | **484** | 11 |

**Table 5**

| Example | IC₅₀ (nM) | Example | IC₅₀ (nM) | Example | IC₅₀ (nM) |
|---|---|---|---|---|---|
| **15** | 251 | **250** | 99 | **328** | 143 |
| **1.8** | 162 | **198** | 39 | **331** | 85 |
| **22** | 123 | **206** | 43 | **334** | 453 |
| **23** | 93 | **207** | 81 | **336** | 153 |
| **81** | 41 | **251** | 159 | **359** | 446 |
| **102** | 55 | **259** | 49 | **360** | 246 |
| **104** | 520 | **261** | 81 | **380** | 17 |
| **109** | 189 | **263** | 49 | **437** | 105 |
| **150** | 52 | **274** | 331 | **446** | 131 |
| **155** | 127 | **288** | 57 | **447** | 240 |
| **156** | 2000 | **296** | 133 | **462** | 144 |
| **161** | 100 | **298** | 69 | **483** | 161 |
| **180** | 15 | **302** | 212 | | |

**Table 6**

| Example | IC₅₀ (nM) | Example | IC₅₀ (nM) |
|---|---|---|---|
| **127** | 376 | **133** | 290 |
| **132** | 347 | **134** | 436 |

### Murine arachidonic acid-induced inflammation model

LTA4H inhibitor compounds of the present invention were dissolved in 20% cyclodextran/H₂O at a concentration of 3 mg/mL. The solutions were administered by oral gavage to female Balb/c mice weighing approximately 20 grams each (0.2 mL per mouse, 30 mg of LTA4H inhibitor compound per kg). Sixty minutes after being administered an LTA4 inhibitor, each mouse received topical application of 20 µL of arachidonic acid (100 mg/mL in acetone) to the left ear and 20 µL of acetone only to the right ear. After 3 h, the mice were sacrificed, blood was withdrawn in heparinized syringes, and 8 mm ear biopsies were taken. Ear biopsies were weighed to determine edema and then frozen at -80 °C until needed for determination of neutrophil influx.

One hundred-microliter aliquots of heparinized blood were added to wells of a microtiter plate, along with equal volumes of RPMI-1640 medium, and calcium ionophore A23187 was added to each sample well (final concentration = 20 ng/µL). The microtiter plate contents were incubated for 10 min at 37 °C in a humidified incubator. The reaction was terminated by centrifugation (833 x g, 10 min at 4 °C). Supernatants were analyzed for LTB4 by a commercially available enzyme-linked immunoassay (Cayman Chemical Co.) in accordance with the manufacturer's instructions. The percent inhibition of ex vivo stimulated LTB4 production (% Inh. LTB4) was determined by comparison to animals treated identically except that the solution admininstered by oral gavage was devoid of inhibitor compound.

Neutrophil influx was quantified by measuring the activity of myeloperoxidase (MPO), a neutrophil-specific enzyme. The ear biopsies were homogenized in 0.5 mL extraction buffer (0.3 M sucrose, 0.22% (w/v) hexadecyl trimethyl ammonium bromide (CTAB), and 2.5 mM citrate prepared from 0.5 M citrate stock solution (pH 5.0)). Debris was removed by centrifugation at 14000 x g for 10 min. Aliquots of 10 µL of the resulting supernatant were added to wells of a microtiter plate, along with 90-µL aliquots of dilution buffer (10 mM citrate, 0.22% CTAB), followed by addition of 20 µL TMB liquid substrate system (Sigma Chemical Co.) to each sample well. The microtiter plate contents were held at room temperature for 1 h. The reaction was stopped by addition of 100 µL 1 M H₂SO₄ to each sample well, and the myeloperoxidase activity in each sample was determined from the absorbance at 405 nm. The background value from the right ear, treated only with acetone, was subtracted from that for the left ear, treated with arachidonic acid in acetone, for each animal. The percent inhibition of neutrophil influx (% lnh. MPO) by compounds of the invention was determined by comparison to animals treated identically, except that the solution administered by oral gavage was devoid of inhibitor compound.

**Table 7**

| Example | % lnh. LTB4 | % Inh. MPO |
|---|---|---|
| **11** | 83 | 95 |
| **14** | 81 | 56 |
| **27** | 50 | 72 |

**Table 8**

| Example | % Inh. LTB4 | % Inh. MPO | Example | % Inh. LTB4 | % Inh. MPO |
|---|---|---|---|---|---|
| **15** | 32 | 29 | **251** | 79 | 66 |
| **18** | 78 | 83 | **259** | 95 | 74 |
| **22** | 79 | 79 | **263** | 0 | 19 |
| **23** | 67 | 84 | **274** | 93 | 87 |
| **81** | 78 | 83 | **325** | 76 | 67 |
| **109** | 51 | 44 | **336** | 67 | 0 |
| **150** | 81 | 91 | **360** | 87 | 88 |
| **155** | 96 | 93 | **361** | 86 | 90 |
| **180** | 87 | 87 | **446** | 64 | 43 |
| **250** | 80 | 94 | **447** | 90 | 48 |
| **206** | 67 | 76 | **471** | 90 | 89 |
| **250** | 80 | 94 | | | |

Having described the invention in specific detail and exemplified the manner in which it may be carried into practice, it will be apparent to those skilled in the art that innumerable variations, applications, modifications, and extensions of the basic principles involved may be made.

## Claims

1. A compound of formula (II): 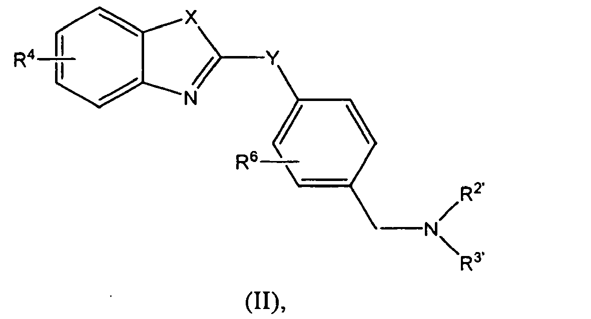 or an enantiomer, diasteromer, racemic, tautomer, hydrate, solvate, or a pharmaceutically acceptable salt, ester, or amide thereof,
wherein
X is selected from the group consisting of NR⁵, O, and S, with R⁵ being one of H and CH₃;
Y is selected from the group consisting of CH₂, and O;
R⁴ is selected from the group consisting of H, OCH₃, Cl, F, Br, I, OH, NH₂, CN, CF₃ and CH₃;
R⁶ is H or F; and
R^{2'} is defined as R² and R^{3'} is defined as R³, as follows:
R² and R³ are each independently selected from the group consisting of
A) H, C₁₋₇alkyl, C₃₋₇alkenyl, wherein the carbon in said alkenyl that is attached to the nitrogen member has only single bonds, C₃₋₇alkynyl, wherein the carbon in said alkynyl that is attached to the nitrogen member has only single bonds, C₃₋₇cycloalkyl optionally benzofused, C₅₋₇cycloalkenyl, -C₃₋₇cycloalkylC₁₋₇alkyl, -C₁₋₇alkylC₃₋₇cycloalkyl and phenyl, wherein each of the substituents A) is independently substituted with 0, 1, or 2 R^{Q}, and each of said R^{Q} is a substituent at a carbon member that is at least one carbon member removed from the nitrogen member;
B) a substituent HetR^{a};
C) -C₁₋₇alkylC(O)R^{x}, optionally substituted with CH₂R^{Ar} or CH₂R^{Ar'};
D) -C₂₋₅alkylC(O)R^{x}, wherein two valence allowed carbon members in the C₂₋₅alkyl of said -C₂₋₅alkylC(O)R^{x} are part of a saturated C₃₋₆carbocycle;
E) -C₂₋₅alkylOH wherein two valence allowed carbon members in the C₂₋₅alkyl of said - C₂₋₅alkylOH are part of a saturated C₃₋₆carbocycle;
F) -C₀₋₄alkylphenyl, wherein the phenyl in said -C₀₋₄alkylphenyl is fused at two adjacent carbon members in said phenyl to R^{f}, or is benzofused;
G) -C₀₋₄alkylAr⁶, where Ar⁶ is a 6-membered heteroaryl having a carbon member point of attachment and having one or two -N= heteroatom members, and benzofused;
H) -C₀₋₄alkylAr⁵, where Ar⁵ is a 5-membered heteroaryl, having one heteroatom member selected from the group consisting of O, S, and >NR^{Y}, and having 0 or 1 -N= additional heteroatom member, optionally containing two carbonyl groups, and optionally benzofused;
I) -C₁₋₄alkylAr^{5'}, where Ar^{5'} is a 5-membered heteroaryl containing 3 or 4 nitrogen members, optionally substituted with R^{Y}, and having a valence allowed site as a point of attachment;
J) -C₀₋₄alkylAr⁶⁻⁶, where Ar⁶⁻⁶ is a C₀₋₄alkyl-attached phenyl fused at valence allowed sites to a 6-membered heteroaryl, wherein said 6-membered heteroaryl has one or two - N= heteroatom members;
K) -C₀₋₄alkylAr⁶⁻⁵, where Ar⁶⁻⁵ is a C₀₋₄alkyl-attached phenyl fused at valence allowed sites to a 5-membered heteroaryl, said 5-membered heteroaryl having one heteroatom member selected from the group consisting of O, S, and >NR^{Y}, and said 5-membered heteroaryl having 0 or 1 additional heteroatom member which is -N=;
L) one of 2-(4-ethyl-phenoxy)-benzothiazole, 2-(4-ethyl-phenoxy)-benzooxazole, and 2-(4-ethyl-phenoxy)-1*H*-benzoimidazole; and
M) SO₂C₁₋₄alkyl;
alternatively R² and R³ are taken together with the nitrogen to which they are attached to form a heterocyclic ring that contains at least one heteroatom member that is said attachment nitrogen, said heterocyclic ring being selected from the group consisting of
i) a 4-7 membered heterocyclic ring HetR^{b}, said 4-7 membered heterocyclic ring HetR^{b} having one heteroatom member that is said attachment nitrogen, and being substituted with 0, 1, or 2 substituents at the same or at different substitution members, said substituents being selected from the group consisting of -R^{Y}, -CN, -C(O)R^{Y}, -C₀₋₄alkylCO₂R^{Y}, -C₀₋₄alkylC(O)CO₂R^{Y}, -C₀₋₄alkylOR^{Y}, -C₀₋₄alkylC(O)NR^{Y}R^{Z}, - C₀₋₄alkylNR^{Y}C(O)R^{Z}, -C(O)NR^{Z}OR^{Y}, -C₀₋₄alkylNR^{Y}C(O)CH₂OR^{Y}, - C₀₋₄alkylNR^{Y}C(O)CH₂C(O)R^{Y}, -C₀₋₄alkylNR^{Y}CO₂R^{Y}, -C₀₋₄alkylNR^{Y}C(O)NR^{Y}R^{Z}, -C_{0- 4}alkylNR^{Y}C(S)NR^{Y}R^{Z}, -NR^{Y}C(O)CO₂R^{Y},-NR^{Y}R^{Z}, -C₀₋₄alkylNR^{W}SO₂R^{Y},1,3-dihydro-indol-2-one-1-yl, 1,3-dihydro-benzoimidazol-2-one-1-yl, tetrazol-5-yl, 1-R^{Y}-1*H-*tetrazol-5-yl, R^{Y}-triazolyl, 2-R^{Y}-2*H*-tetrazol-5-yl, pyrrolidine-2-thion-1-yl, piperidine-2-thion-1-yl, -C₀₋₄alkylC(O)N(R^{Y})(SO₂R^{Y}), -C₀₋₄alkylN(R^{Y})(SO₂)NR^{Y}R^{Y}, -C₀₋₄alkylN(R^{Y})(SO₂)NR^{Y}CO₂R^{Y}, halo, 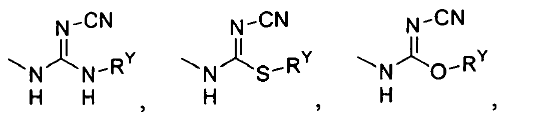 and 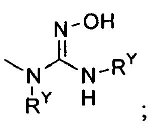
ii) a 5-7 membered heterocyclic ring HetR^{c}, said 5-7 heterocyclic ring HetR^{c} having one additional heteroatom member separated from said attachment nitrogen by at least one carbon members, said additional heteroatom member being selected from the group consisting of O, S(=O)₀₋₂, and >NR^{M}, said 5-7 membered heterocyclic ring HetR^{c} having 0 or 1 carbonyl members, and being substituted with 0, 1, or 2 substituents at the same or at different carbon substitution members, said substituents being selected from the group consisting of -C(O)R^{Y}, -CO₂R^{Y} -C₃₋₄alkylCO₂R^{Y} and R^{Z};
iii) one of imidazolidin-1-yl, 2-imidazolin-1-yl, pyrazol-1-yl, imidazol-1-yl, 2*H-*tetrazol-2-yl, 1*H*-tetrazol-1-yl, pyrrol-1-yl, 2-pyrrolin-1-yl, and 3-pyrrolin-1-yl, wherein each of said 2*H*-tetrazol-2-yl and 1*H*-tetrazol-1-yl is substituted at the carbon member with 0 or 1 of -C₀₋₄alkylR^{Z}, -C₀₋₄alkylSR^{Y}, -C₀₋₄alkylCO₂R^{Y}, and substituent HetR^{a}; and
iv) one of 1,2,3,4-tetrahydro-quinolin-1-yl, 1,2,3,4-tetrahydro-isoquinolin-2-yl, indol-1-yl, isoindol-2-yl, indolin-1-yl, benzimidazol-1-yl, 2,8-diaza-spiro[4.5]decan-1-one-8-yl, 4-{[(2-tert-butoxycarbonylamino-cyclobutanecarbonyl)-amino]-methyl}-piperidin-1-yl, 4-{[(2-amino-cyclobutanecarbonyl)-amino]-methyl}-piperidin-1-yl, 3,9-diaza-spiro[5.5]undecane-3-carboxylic acid-9-yl tert-butyl ester, 4-oxo-1-phenyl-1,3,8-triaza-spiro[4.5]dec-8-yl, and 4-oxo-1,3,8-triaza-spiro[4.5]dec-8-yl;
wherein
substituent HetR^{a} is a 4-7 membered heterocyclic ring having a carbon member point of attachment and containing a member >NR^{M} as a heteroatom member, and said heteroatom member being separated from said carbon member point of attachment by at least 1 additional carbon member;
R^{K} is selected from the group consisting of H, -C₁₋₄alkyl, -C₀₋₄alkylR^{Ar} each optionally substituted with 1, 2, or 3 substituents R^{N}
R^{L} is selected from the group consisting of -CO₂R^{S} and -C(O)NR^{S}R^{S'};
R^{M} is selected from the group consisting of R^{Z}, indol-7-yl, -SO₂R^{Y}, -C₃₋₄alkylCO₂R^{Y}, - CO₂R^{Y}, -C(O)NR^{Z}OR^{Y}, -C(O)R^{Y}, -C(O)C₁₋₄alkylOR^{Y}, -C₀₋₄alkylC(O)NR^{S}R^{S'}, C₀₋₄alkylC(O)CO₂R^{Y}, 1,3-dihydro-indol-2-one-1-yl, 1,3-dihydro-benzoimidazol-2-one-1-yl, tetrazol-5-yl, 1-R^{Y}-1*H*-tetrazol-5-yl, R^{Y}-triazolyl, 2-R^{Y}-2*H*-tetrazol-5-yl and -C₀₋₄alkylC(O)N(R^{Y})(SO₂R^{Y}), each optionally substituted with 1, 2 or 3 substituents R^{N};
R^{N} is selected from the group consisting of OCH₃, Cl, F, Br, I, OH, NH₂, CN, CF₃, CH₃, OC(O)CH₃, and NO₂;
R^{P} is selected from the group consisting of R^{Y}, -C₂₋₄alkylOR^{Y}, R^{Ar}, -C₁₋₂alkylCO₂R^{Y}, - C₁₋₂alkylCONR^{S}R^{S'}, indol-7-yl, and -SO₂C₁₋₄alkyl;
R^{Q} is selected from the group consisting of fluoro, chloro, bromo, iodo, trifluoromethyl, trichloromethyl, -CN, -C₁₋₄alkyl, -C₀₋₄alkylR^{Ar}, -C₀₋₄alkylR^{Ar'}, -C₀₋₄alkylOR^{Y}, -C₀₋₄alkylCO₂R^{Y}, -C₀₋₄alkylNR^{Y}R^{Z}, -C₀₋₄alkylNR^{Y}COR^{Y}, -C₀₋₄alkylNR^{Y}CONR^{Y}R^{Z}, -C₀₋₄alkylNR^{Y}SO₂R^{Y}, and -C₀₋₄alkylSR^{Y};
R^{S} and R^{S'} are independently selected from the group consisting of H, -C₁₋₄alkyl, and - C₀₋₄alkylphenyl; alternatively, R^{S} and R^{S'} are taken together with the nitrogen member to which said R^{S} and R^{S'} are attached to form a 4-7 membered heterocyclic ring having 0 or 1 additional heteroatom member selected from the group consisting of O, S, and >NR^{Y}, provided that said additional heteroatom member is separated by at least two carbon members from said nitrogen member to which said R^{S} and R^{S'} are attached, and provided that where R^{Y} is C₀₋₄alkylR^{Ar}, then R^{Ar} is not substituted with R^{L};
R^{W} is selected from the group consisting of R^{Y}, and -C₃₋₇cycloalkyl;
R^{X} is selected from the group consisting of -OR^{Y}, -NR^{Y}R^{Z}, -C₁₋₄alkyl, and -C₀₋₄alkylR^{Ar}; R^{Y} is selected from the group consisting of H, -C₁₋₄alkyl, -C₀₋₄alkylR^{Ar} and -C₀₋₄alkylR^{Ar'}, each optionally substituted with 1, 2, or 3 substituents R^{N};
R^{Z} is selected from the group consisting of R^{Y}, -C₂₋₄alkylOR^{Y}, -C₁₋₂alkylCO₂R^{Y}, -C₁₋₂alkylC(O)NR^{S}R^{S'}, and -C₂₋₄alkylNR^{S}R^{S'};
when R^{Y} and R^{Z} are attached to a nitrogen member, R^{Y} and R^{Z} are selected as defined above, or R^{Y} and R^{Z} are taken together with the R^{Y}- and R^{Z}- attached nitrogen member to form a 4-7 membered heterocyclic ring HetR^{d} having 0 or 1 additional heteroatom members selected from the group consisting of O, S, and >NR^{M}, said 4-7 membered heterocyclic ring HetR^{d} having 0 or 1 carbonyl members, and said 4-7 membered heterocyclic ring HetR^{d} having 0 or I valence allowed carbon members substituted with at least one of R^{M}, -CO₂H, and -C₀₋₁alkylOR^{Y};
R^{Ar} is a moiety with a carbon member attachment point and said moiety is selected from the group consisting of phenyl, pyridyl, pyrimidyl, and pyrazinyl, wherein each valence allowed carbon member in each of said moieties is independently substituted with at least one of 0, 1, 2 or 3 R^{N}, and 0 or 1 R^{L};
R^{Ar'} is a 3-8 membered ring, having 0, 1 or 2 heteroatom members selected from the group consisting of O, S, N, and >NR^{Y}, having 0, 1, or 2 unsaturated bonds, having 0 or 1 carbonyl members, wherein each valence allowed member in each of said rings is independently substituted with 0, 1, or 2 R^{K}; and
R^{f} is a linear 3- to 5-membered hydrocarbon moiety having 0 or 1 unsaturated carbon-carbon bonds and having 0 or 1 carbonyl members; provided that
(a) said R^{2'} and R^{3'} further satisfy the following conditions:
(e1): said R^{2'} and R^{3'} are not both H, when Y is O, and X is S;
(e2): when Y is CH₂, X is N, and said R^{2'} and R^{3'} are parts of a primary or secondary amino group, then said R^{2'} and R^{3'} are not selected from the group consisting of H and
methyl;
(e3): said R²' and R³' taken together with the nitrogen member to which they are attached do not form a piperazine group, when X is O, and Y is one of O and CH₂;
(e4): said R²' and R³' taken together with the nitrogen member to which they are attached do not form a piperidine group that is monosubstituted with a saturated 6-membered cyclic group, when X is O, and Y is one of O and CH₂; and
(e5): said R^{2'} and R^{3'} taken together with the nitrogen member to which they are attached do not form either a substituted piperidine group or a substituted piperazine group, wherein said substituted piperidine group or said substituted piperazine group is substituted in the 4-position with a substituent XG, said XG having the structure 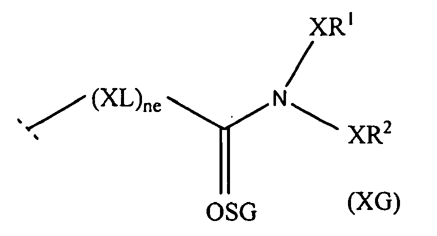
wherein n = 0, 1, and when ne = 1 then XL is a C₁₋₆alkyl, OSG is 0 or S, and XR¹ and XR² taken together with the nitrogen member to which they are attached form one of a piperidine group, a piperazine group, a morpholine group, a thiomorpholine group, and a pyrrolidine group, or each of XR¹ and XR² taken independently are one of H, C₁₋₆alkyl, aryl, aralkyl, C₃₋₈cycloalkyl, C₃₋₈cyloalkyl-C₁₋₆alkyl, heteroalkyl, heteroaryl-C₁₋₆alkyl, heterocycloalkyl and heterocycloalkyl-C₁₋₆alkyl; wherein the aryl, aralkyl, cycloalkyl, heteroaryl or heterocycloalkyl may be optionally substituted with one or more substituents independently selected from halogen, hydroxy, C₁₋₆alkyl, C₁₋₆alkoxy, halogenatedC₁₋₆alkyl, halogenatedC₁₋₆alkoxy, nitro, cyano, amino, C₁₋₄alkylamino, di(C₁₋₄alkyl)amino, heteroaryl or heterocycloalkyl; and
(b) further provided that when X is S, and Y is O, then one of R^{2'} and R^{3'} is not XCG when the other is C₁₋₆alkyl, wherein XCG is the group 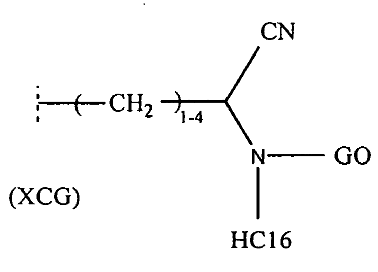
wherein HC16 is one of H, C₁₋₆alkyl, haloC₁₋₆alkyl, allyl, and C₁₋₆alkoxymethyl, and GO is a group attached by a carbon member that has a =O substituent forming an amido group with the nitrogen member to which said GO group is attached.

2. A compound as claimed in claim 1, wherein said R⁴ is H.

3. A compound as claimed in claim 1, wherein said R² and R³ are each independently selected from
(i) the group consisting of A), B), C), D), E), and I); or
(ii) group A); or
(iii) group B); or
(iv) group C); or
(v) group D); or
(vi) group E); or
(vii) group I),
as defined in claim 1.

4. A compound as claimed in claim 1 wherein said R² and R³ are taken together with the nitrogen to which they are attached to form a heterocyclic ring that contains at least one heteroatom member that is said attachment nitrogen, said heterocyclic ring being selected from any one of
(a) the group consisting of i) and ii);
(b) the group i), and
(c) the group ii)
as defined in claim 1.

5. A compound as claimed in claim 1, wherein said compound is one of: 2-(4-Piperidin-1-ylmethyl-phenoxy)-benzooxazole; and
2-(2-Fluoro-4-piperidin-1-ylmethyl-phenoxy)-benzooxazole.

6. A compound as claimed in claim 1, wherein said compound is one of:
1-[4-(Benzothiazol-2-yloxy)-benzyl]-piperidine-4-carboxylic acid;
1-{1-[4-(Benzothiazol-2-yloxy)-benzyl]-piperidin-4-yl}-pyrrolidin-2-one;
2-(2-Fluoro-4-piperidin-1-ylmethyl-phenoxy)-benzothiazole;
1-(2-{[4-(Benzothiazol-2-yloxy)-benzyl]-cyclopropyl-amino}-ethyl)-4-hydroxy-pyrrolidin-2-one;
N-{1-[4-(Benzothiazol-2-yloxy)-benzyl]-piperidin-4-yl}-N-methyl-methanesulfonamide;
2-{4-[4-(1H-Tetrazol-5-yl)-piperidin-1-ylmethyl]-phenoxy}-benzothiazole;
1-{4-[4-(Benzothiazol-2-yloxy)-benzyl]-piperazin-1-yl}-2-hydroxy-ethanone;
N-{1-[4-(Benzothiazol-2-yloxy)-benzyl]-piperidin-4-ylmethyl}-methanesulfonamide;
3-{1-[4-(Benzothiazol-2-yloxy)-benzyl]-piperidin-4-yl}-oxazolidin-2-one;
4- {1-[4-(Benzothiazol-2-yloxy)-benzyl]-piperidin-4-yl}-morpholin-3-one;
2-(4-Piperidin-1-ylmethyl-phenoxy)-benzothiazole;
1-[4-(Benzothiazol-2-yloxy)-benzyl]-4-phenyl-piperidin-4-ol;
1-[4-(Benzothiazol-2-yloxy)-benzyl]-piperidin-4-ol;
{1-[4-(Benzothiazol-2-yloxy)-benzyl]-piperidin-4-yl}-methanol;
N-{{1-[4-(Benzothiazol-2-yloxy)-benzyl]-piperidin-4-yl}-methanesulfonamide;
N-{1-[4-(Benzothiazol-2-yloxy)-benzyl]-piperidin-4-ylmethyl}-2-hydroxy-acetamide;
{1-[4-(Benzothiazol-2-yloxy)-benzyl]-piperidin-4-yl}-carbamic acid methyl ester;
{1-[4-(Benzothiazol-2-yloxy)-benzyl]-piperidin-4-ylmethyl}-urea;
N-{1-[4-(Benzothiazol-2-yloxy)-benzyl]-piperidin-4-ylmethyl}-2,2,2-trifluoro-acetamide;
{4-[4-(Benzothiazol-2-yloxy)-benzyl]-piperazin-1-yl}-acetic acid;
2-[4-(4-Methanesulfonyl-piperazin-1-ylmethyl)-phenoxy]-benzothiazole;
1-{4-[4-(Benzothiazol-2-yloxy)-benzyl]-piperazin-1-yl}-2,2,2-trifluoroethanone;
2-(4-Morpholin-4-ylmethyl-phenoxy)-benzothiazole;
{1-[4-(Benzothiazol-2-yloxy)-benzyl]-piperidin-4-yl}-carbamic acid phenyl ester;
N-{1-[4-(Benzothiazol-2-yloxy)-benzyl]-piperidin-4-yl}-benzenesulfonamide;
3-[4-(Benzothiazol-2-yloxy)-benzylamino]-propionic acid ethyl ester;
3-[4-(Benzothiazol-2-yloxy)-benzylamino]-propionic acid;
1-{3-[4-(Benzothiazol-2-yloxy)-benzylamino]-propyl}-pyrrolidin-2-one;
1-(3-{[4-(Benzothiazol-2-yloxy)-benzyl]-methyl-amino}-propyl)-pyrrolidin-2-one;
1-(3-{[4-(Benzothiazol-2-yloxy)-benzyl]-isopropyl-amino}-propyl)-pyrrolidin-2-one;
1-(3-{[4-(Benzothiazol-2-yloxy)-benzyl]-ethyl-amino}-propyl)-pyrrolidin-2-one;
[4-(Benzothiazol-2-yloxy)-benzyl]-cyclopropyl-amine;
N-1-[4-(Benzothiazol-2-yloxy)-benzyl]-N1-cyclopropyl-propane-1,3-diamine;
N-(3-{[4-(Benzothiazol-2-yloxy)-benzyl]-cyclopropyl-amino}-propyl)-isobutyramide;
1-(3-{[4-(Benzothiazol-2-yloxy)-benzyl]-cyclopropyl-amino}-propyl)-3-isopropyl-urea;
1-{1-[4-(Benzothiazol-2-yloxy)-benzyl]-piperidin-4-yl}-3-isopropyl-urea;
N-{1-[4-(Benzothiazol-2-yloxy)-benzyl]-piperidin-4-yl}-oxalamic acid methyl ester;
N-{1-[4-(Benzothiazol-2-yloxy)-benzyl]-piperidin-4-yl}-isobutyramide;
Tetrahydro-furan-2-carboxylic acid {1-[4-(benzothiazol-2-yloxy)-benzyl]-piperidin-4-yl}-amide;
1-{1-[4-(Benzothiazol-2-yloxy)-benzyl]-piperidin-4-yl}-4-hydroxy-pyrrolidin-2-one;
1-{1-[4-(Benzothiazol-2-yloxy)-benzyl]-piperidin-4-yl}-4-hydroxy-pyrrolidin-2-one;
{1-[4-(Benzothiazol-2-yloxy)-benzyl]-piperidin-4-yl}-urea;
N- {1-[4-(Benzothiazol-2-yloxy)-benzyl]-piperidin-4-yl}-oxalamic acid;
N-{1-[4-(Benzothiazol-2-yloxy)-benzyl]-piperidin-4-yl}-2-hydroxy-acetamide;
N-{1-[4-(Benzothiazol-2-yloxy)-benzyl]-piperidin-4-yl}-2,2,2-trifluoroacetamide;
2-[4-(1,1-Dioxo-116-thiomorpholin-4-ylmethyl)-phenoxy]-benzothiazole;
N-{1-[4-(benzothiazol-2-yloxy)-benzyl]-piperidin-4-amino sulfonyl}-carbamic acid tert-butyl ester;
N-1-[4-(Benzothiazol-2-yloxy)-benzyl]-piperidin-4-yl}-acetamide;
N-{1-[4-(Benzothiazol-2-yloxy)-benzyl]-piperidin-4-yl}-N,N-dimethylsulfamide;
1-{1-[4-(Benzothiazol-2-yloxy)-benzyl]-piperidin-4-yl}-3-ethyl-urea;
1-{1-[4-(Benzothiazol-2-yloxy)-benzyl]-piperidin-4-yl}-3-ethyl-thiourea;
Propane-1-sulfonic acid {1-[4-(benzothiazol-2-yloxy)-benzyl]-piperidin-4-yl}-amide;
Propane-2-sulfonic acid {1-[4-(benzothiazol-2-yloxy)-benzyl]-piperidin-4-yl}-amide;
N-{1-[4-(Benzothiazol-2-yloxy)-benzyl]-piperidin-4-yl}-sulfamide;
N-{1-[4-(Benzothiazol-2-yloxy)-benzyl]-piperidin-4-yl}-formamide;
{1-[4-(Benzothiazol-2-yloxy)-benzyl]-piperidin-4-yl}-carbamic acid ethyl ester;
N-{1-[4-(Benzothiazol-2-yloxy)-benzyl]-piperidin-4-yl}-propionamide;
N-{1-[4-(Benzothiazol-2-yloxy)-benzyl]-piperidin-4-yl}-butyramide;
1-{1-[4-(Benzothiazol-2-yloxy)-benzyl]-piperidin-4-yl}-3-propyl-urea;
{1-[4-(Benzothiazol-2-yloxy)-benzyl]-piperidin-4-yl}-carbamic acid propyl ester;
1-{1-[4-(Benzothiazol-2-yloxy)-benzyl]-piperidin-4-yl}-3-methyl-urea;
1-{1-[4-(Benzothiazol-2-yloxy)-benzyl]-piperidin-4-yl}-1,3-dimethyl-urea;
1-{1-[4-(Benzothiazol-2-yloxy)-benzyl]-piperidin-4-yl}-1-methyl-urea;
N-{1-[4-(Benzothiazol-2-yloxy)-benzyl]-piperidin-4-yl}-N-methylacetamide;
{1-[4-(Benzothiazol-2-yloxy)-benzyl]-piperidin-4-yl}-methyl-carbamic acid methyl ester;
N-{1-[4-(Benzothiazol-2-yloxy)-benzyl]-piperidin-4-yl}-N-methyl-oxalamic acid methyl ester;
N-{1-[4-(Benzothiazol-2-yloxy)-benzyl]-piperidin-4-yl}-N-methyl-oxalamic acid;
Guanidine, N-{1-[4-(Benzothiazol-2-yloxy)-benzyl]-piperidin-4-yl}-N'-hydroxy;
{1-[4-(Benzothiazol-2-yloxy)-benzyl]-piperidin-4-yl}-carbamic acid isopropyl ester;
3-{1-[4-(Benzothiazol-2-yloxy)-benzyl]-piperidin-4-yl}-1,1-dimethyl-urea;
Acetic acid {1-[4-(benzothiazol-2-yloxy)-benzyl]-piperidin-4-ylcarbamoyl}-methyl ester;
{1-[4-(Benzothiazol-2-yloxy)-benzyl]-piperidin-4-yl}-thiourea; 1'-[4-(Benzothiazol-2-yloxy)-benzyl]-[1,4']bipiperidinyl;
{4-[4-(Benzothiazol-2-yloxy)-benzyl]-piperazin-1-yl}-(tetrahydro-furan-2-yl)-methanone;
{1-[4-(Benzothiazol-2-yloxy)-benzyl]-piperidin-4-yl}-carbamic acid tert-butyl ester;
4-[4-(Benzothiazol-2-yloxy)-benzyl]-piperazine-1-carboxylic acid tert-butyl ester;
1-[4-(Benzothiazol-2-yloxy)-benzyl]-piperidin-4-ylamine;
2-(4-Piperazin-1-ylmethyl-phenoxy)-benzothiazole;
4-[4-(Benzothiazol-2-yloxy)-benzyl]-piperazine-1-carboxylic acid amide;
2-[4-(4-Benzenesulfonyl-piperazin-1-ylmethyl)-phenoxy]-benzothiazole;
C-{1-[4-(Benzothiazol-2-yloxy)-benzyl]-piperidin-4-yl}-methylamine;
2-(2-{4-[4-(Benzothiazol-2-yloxy)-benzyl]-piperazin-1-yl}-2-oxo-ethyl)-cyclopentanone;
{4-[4-(Benzothiazol-2-yloxy)-benzyl]-piperazin-1-yl}-acetic acid ethyl ester;
4-{1-[4-(Benzothiazol-2-yloxy)-benzyl]-piperidin-4-yl}-butyric acid 4-(benzothiazol-2-yloxy)-benzyl ester;
1-(3-{[4-(Benzothiazol-2-yloxy)-benzyl]-cyclopropyl-amino}-propyl)-pyrrolidin-2-one;
(3-{[4-(Benzothiazol-2-yloxy)-benzyl]-cyclopropyl-amino}-propyl)-carbamic acid tert-butyl ester;
Tetrahydro-furan-2-carboxylic acid (3-{[4-(benzothiazol-2-yloxy)-benzyl]-cyclopropyl-amino}-propyl)-amide;
1-(3-{[4-(Benzothiazol-2-yloxy)-benzyl]-cyclopropyl-amino}-propyl)-4-hydroxy-pyrrolidin-2-one;
(2-{[4-(Benzothiazol-2-yloxy)-benzyl]-cyclopropyl-amino}-ethyl)-carbamic acid tert-butyl ester;
N 1-[4-(Benzothiazol-2-yloxy)-benzyl]-N1-cyclopropyl-ethane-1,2-diamine;
Ethanesulfonic acid {1-[4-(benzothiazol-2-yloxy)-benzyl]-piperidin-4-yl}-amide;
1-{1-[4-(Benzothiazol-2-yloxy)-benzyl]-piperidin-4-yl}-pyrrole-2,5-dione;
{1-[4-(Benzothiazol-2-yloxy)-benzyl]-piperidin-4-yl}-methyl-carbamic acid tert-butyl ester;
{1-[4-(Benzothiazol-2-yloxy)-benzyl]-piperidin-4-yl}-methyl-amine;
1-[4-(Benzothiazol-2-yloxy)-benzyl]-piperazine-2-carboxylic acid;
[4-(Benzothiazol-2-yloxy)-benzyl]-methyl-amine;
Benzothiazol-2-yl-{1-[4-(benzothiazol-2-yloxy)-benzyl]-piperidin-4-yl}-amine;
(3-{[4-(Benzothiazol-2-yloxy)-benzyl]-methyl-amino}-propyl)-carbamic acid tert-butyl ester;
4-({[4-(Benzothiazol-2-yloxy)-benzyl]-methyl-amino}-methyl)-phenol;
N 1-[4-(Benzothiazol-2-yloxy)-benzyl]-N1-methyl-propane-1,3-diamine;
Acetic acid (3-{[4-(benzothiazol-2-yloxy)-benzyl]-methyl-amino}-propylcarbamoyl)-methyl ester;
N-(3-{[4-(Benzothiazol-2-yloxy)-benzyl]-methyl-amino}-propyl)-2-hydroxy-acetamide;
N-(3-{[4-(Benzothiazol-2-yloxy)-benzyl]-methyl-amino}-propyl)-methanesulfonamide;
6-Chloro-2-(4-piperidin-1-ylmethyl-phenoxy)-benzothiazole;
6-Methoxy-2-(4-piperidin-1-ylmethyl-phenoxy)-benzothiazole;
4-[4-(Benzothiazol-2-yloxy)-benzyl]-piperazine-1-sulfonic acid dimethylamide;
2-{4-[4-(Benzothiazol-2-yloxy)-benzyl]-piperazin-1-yl}-1-pyrrolidin-1-yl-ethanone;
2- {4-[4-(Benzothiazol-2-yloxy)-benzyl]-piperazin-1-yl}-1-morpholin-4-yl-ethanone;
{4-[4-(Benzothiazol-2-yloxy)-benzyl]-piperazin-1-yl}-thiophen-2-yl-methanone;
4-Methyl-2-(4-piperidin-1-ylmethyl-phenoxy)-benzothiazole;
4-Chloro-2-(4-piperidin-1-ylmethyl-phenoxy)-benzothiazole;
2-[4-(4,4-Difluoro-piperidin-1-ylmethyl)-phenoxy]-benzothiazole;
2-Amino-cyclobutanecarboxylic acid {1-[4-(benzothiazol-2-yloxy)-benzyl]-piperidin-4-ylmethyl}-amide;
[4-(Benzothiazol-2-yloxy)-benzyl]-methyl-(1-methyl-piperidin-4-yl)-amine;
3-{[4-(Benzothiazol-2-yloxy)-benzyl]-methyl-amino}-propionitrile;
4-[4-(Benzothiazol-2-yloxy)-benzyl]-piperazin-2-one;
2-[4-(3-Methyl-piperidin-1-ylmethyl)-phenoxy]-benzothiazole;
Acetic acid ({1-[4-(benzothiazol-2-yloxy)-benzyl]-piperidin-4-yl}-methyl-carbamoyl)-methyl ester;
N- {1-[4-(Benzothiazol-2-yloxy)-benzyl]-piperidin-4-yl}-2-hydroxy-N-methyl-acetamide; and
1-[4-(Benzothiazol-2-yloxy)-benzyl]-piperidine-4-carboxylic acid ethyl ester.

7. A compound as claimed in claim 1, wherein said compound is one of: 2-(4-Piperidin-1-ylmethyl-phenoxy)-H-benzoimidazole; and 1-[4-(1H-Benzoimidazol-2-yloxy)-benzyl]-piperidine-4-carboxylic acid.

8. A pharmaceutical composition comprising at least one compound as claimed in any one of claims 1 - 7.

9. Use of a therapeutically effective amount of at least one LTA4H modulator selected from compounds of claim 1, in the manufacture of a medicament for the treatment, prevention or inhibition of inflammation in a subject.

10. Use as claimed in claim 9 wherein said inflammation is due to at least one of asthma, chronic obstructed pulmonary disease, atherosclerosis, rheumatoid arthritis, multiple sclerosis, inflammatory bowel disease, and psoriasis.

11. Use of an inhibitory amount of at least one LTA4H modulator selected from compounds as claimed in claim 1 in the manufacture of a medicament for the inhibition of LTA4H enzyme activity.

12. A use as claimed in any one of claims 9-11, wherein said R⁴ is H.

13. A use as claimed in any one of claims 9-11, wherein said R² and R³ are each independently selected from
(i) the group consisting of A), B), C), D), E), and I); or
(ii) group A); or
(iii) group B); or
(iv) group C); or
(v) group D); or
(vi) group E); or
(vii) group I),
as defined in claim 1.

14. A use as claimed in any one of claims 9-11, wherein said R² and R³ are taken together with the nitrogen to which they are attached to form a heterocyclic ring that contains at least one heteroatom member that is said attachment nitrogen, said heterocyclic ring being selected from any one of
(d) the group consisting of i) and ii);
(e) the group i), and
(f) the group ii)
as defined in claim 1.

15. A use as claimed in any one of claims 9-11, wherein said at least one LTA4H modulator is selected from the compounds as claimed in any one of claims 5 - 7.

## Patentansprüche

1. Verbindung von Formel (II) 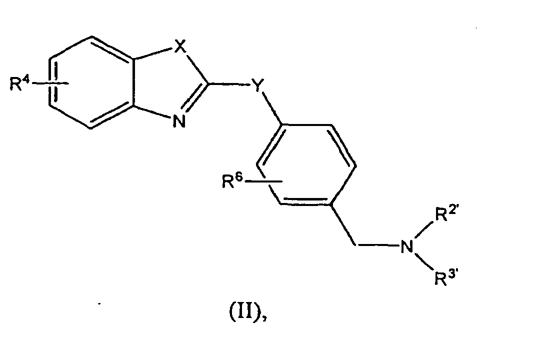 oder ein Enantiomer, Diastereomer, Razemat, Tautomer, Hydrat, Solvat oder ein pharmazeutisch verträgliches/r Salz, Ester oder Amid davon,
worin
X ausgewählt ist aus der Gruppe, bestehend aus NR⁵, O und S, wobei R⁵ eines von H und CH₃ ist;
Y ausgewählt ist aus der Gruppe, bestehend aus CH₂ und O;
R⁴ ausgewählt ist aus der Gruppe, bestehend aus H, OCH₃, Cl, F, Br, I, OH, NH₂, CN, CF₃ und CH₃;
R⁶ H oder F ist; und
R^{2'} wie R² definiert ist und R^{3'} wie R³ definiert ist, wie folgt:
R² und R³ sind jeweils unabhängig ausgewählt aus der Gruppe, bestehend aus
A) H, C₁₋₇-Alkyl, C₃₋₇-Alkenyl, wobei der Kohlenstoff im Alkenyl, der an das Stickstoffglied gebunden ist, nur Einfachbindungen aufweist, C₃₋₇-Alkinyl, wobei der Kohlenstoff im Alkinyl, der an das Stickstoffglied gebunden ist, nur Einfachbindungen aufweist, C₃₋₇-Cycloalkyl, gegebenenfalls benzokondensiert, C₅₋₇-Cyloalkenyl, -C₃₋₇-Cycloalkyl-C₁₋₇-alkyl, -C₁₋₇-Alkyl-C₃₋₇-cycloalkyl und Phenyl, wobei jeder der Substituenten A) unabhängig mit 0, 1 oder 2 R^{Q} substituiert ist und R^{Q} jeweils ein Substituent an einem Kohlenstoffglied ist, das wenigstens ein Kohlenstoffglied vom Stickstoffglied entfernt ist;
B) einem Substituenten HetR^{a};
C) -C₁₋₇-AlkylC(O)R^{x}, gegebenenfalls substituiert mit CH₂R^{Ar} oder CH₂R^{Ar'};
D) -C₂₋₅-AlkylC(O)R^{x}, worin zwei valenzerlaubte Kohlenstoffglieder im C₂₋₅-Alkyl vom -C₂₋₅-AlkylC(O)R^{x} Teil eines gesättigten C₃₋₆-Carbocyclus sind;
E) -C₂₋₅-AlkylOH, worin zwei valenzerlaubte Kohlenstoffglieder im C₂₋₅-Alkyl vom -C₂₋₅-AlkylOH Teil eines gesättigten C₃₋₆-Carbocyclus sind;
F) -C₀₋₄-Alkylphenyl, worin das Phenyl im -C₀₋₄-Alkylphenyl an zwei benachbarten Kohlenstoffgliedern im Phenyl an R^{f} kondensiert ist oder benzokondensiert ist;
G) -C₀₋₄-AlkylAr⁶, worin Ar⁶ ein 6-gliedriges Heteroaryl mit einem Kohlenstoffglied-Bindungspunkt und mit einem oder zwei -N=-Heteroatomgliedem und benzokondensiert ist;
H) -C₀₋₄-AlkylAr⁵, worin Ar⁵ ein 5-gliedriges Heteroaryl mit einem Heteroatomglied, das ausgewählt ist aus der Gruppe, bestehend aus O, S und >NR^{Y}, und mit 0 oder 1 zusätzlichen -N=-Heteroatomgliedem, die gegebenenfalls zwei Carbonylgruppen enthalten, und gegebenenfalls benzokondensiert ist;
I) -C₁₋₄-AlkylAr^{5'}, worin Ar^{5'} ein 5-gliedriges Heteroaryl ist, das 3 oder 4 Stickstoffglieder enthält, gegebenenfalls substituiert mit R^{Y}, und mit einer valenzerlaubten Stelle als einem Bindungspunkt;
J) -C₀₋₄-AlkylAr⁶⁻⁶, worin Ar⁶⁻⁶ ein an C₀₋₄-Alkyl gebundenes Phenyl ist, das an valenzerlaubten Stellen an ein 6-gliedriges Heteroaryl kondensiert ist, wobei das 6-gliedrige Heteroaryl ein oder zwei -N=-Heteroatomglieder aufweist;
K) -C₀₋₄-AlkylAr⁶⁻⁵, worin Ar⁶⁻⁵ ein an C₀₋₄-Alkyl gebundenes Phenyl ist, das an valenzerlaubten Stellen an ein 5-gliedriges Heteroaryl kondensiert ist, wobei das 5-gliedrige Heteroaryl ein Heteroatomglied aufweist, das ausgewählt ist aus der Gruppe, bestehend aus O, S und >NR^{Y}, und das 5-gliedrige Heteroaryl 0 oder 1 zusätzliches Heteroatomglied aufweist, das -N= ist;
L) einem von 2-(4-Ethylphenoxy)-benzothiazol, 2-(4-Ethylphenoxy)-benzooxazol und 2-(4-Ethylphenoxy)-1H-benzoimidazol; und
M) SO₂C₁₋₄-Alkyl;
alternativ sind R² und R³ mit dem Stickstoff, an das sie gebunden sind, zusammengenommen, um einen heterocyclischen Ring zu bilden, der wenigstens ein Heteroatomglied enthält, das der Bindungsstickstoff ist, wobei der heterocyclische Ring ausgewählt ist aus der Gruppe, bestehend aus
i) einem 4- bis 7-gliedrigen Ring HetR^{b}, wobei der 4- bis 7-gliedrige heterocyclische Ring HetR^{b} ein Heteroatomglied aufweist, das der Bindungsstickstoff ist, und mit 0, 1 oder 2 Substituenten an denselben oder an verschiedenen Substitutionsgliedern substituiert ist, wobei die Substituenten ausgewählt sind aus der Gruppe, bestehend aus -R^{Y}, -CN, -C(O)R^{Y}, -C₀₋₄-AlkylCO₂R^{Y}, -C₀₋₄-AlkylC(O)CO₂R^{Y}, -C₀₋₄-AlkylOR^{Y}, -C₀₋₄-AlkylC(O)NR^{Y}R^{Z}, -C₀₋₄-AlkylNR^{Y}C(O)R^{Z}, -C(O)NR^{Z}OR^{Y}, -C₀₋₄-AlkylNR^{Y}C(O)CH₂OR^{Y}, -C₀₋₄-AlkylNR^{Y}C(O)CH₂C(O)R^{Y}, -C₀₋₄-AlkylNR^{Y}CO₂R^{Y}, -C₀₋₄-AlkylNR^{Y}C(O)NR^{Y}R^{Z}, -C₀₋₄-AlkylNR^{Y}C(S)NR^{Y}R^{Z}, -NR^{Y}C(O)CO₂R^{Y}, -NR^{Y}R^{Z}, -C₀₋₄-AlkylNR^{W}SO₂R^{Y}, 1,3-Dihydroindol-2-on-1-yl, 1,3-Dihydrobenzoimidazol-2-on-1-yl, Tetrazol-5-yl, 1-R^{Y}-1H-Tetrazol-5-yl, R^{Y}-Triazolyl, 2-R^{Y}-2H-Tetrazol-5-yl, Pyrrolidin-2-thion-1-yl, Piperidin-2-thion-1-yl, -C₀₋₄-AlkylC(O)N(R^{Y})(SO₂R^{Y}), -C₀₋₄-AlkylN(R^{Y})(SO₂)NR^{Y}R^{Y}, -C₀₋₄-AlkylN(R^{Y})(SO₂)NR^{Y}CO₂R^{Y}, Halo, 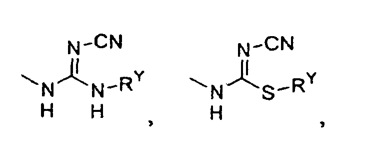 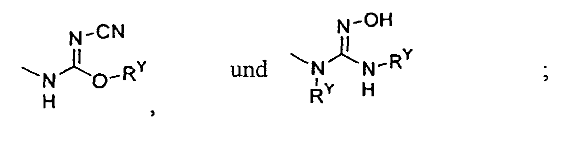
ii) einem 5- bis 7-gliedrigen heterocyclischen Ring HetR^{c}, wobei der 5- bis 7-gliedrige heterocyclische Ring HetR^{c} ein zusätzliches Heteroatomglied aufweist, das vom Bindungsstickstoff durch wenigstens ein Kohlenstoffglied getrennt ist, wobei das zusätzliche Heteroatomglied ausgewählt ist aus der Gruppe, bestehend aus O, S(=O)₀₋₂ und >NR^{M}, wobei der 5- bis 7-gliedrige heterocyclische Ring HetR^{c} 0 oder 1 Carbonylglieder aufweist und mit 0, 1 oder 2 Substituenten an denselben oder an verschiedenen Kohlenstoffsubstitutionsgliedern substituiert ist, wobei die Substituenten ausgewählt sind aus der Gruppe, bestehend aus -C(O)R^{Y}, -CO₂R^{Y}, -C₃₋₄-AlkylCO₂R^{Y} und R^{Z};
iii) einem von Imidazolidin-1-yl, 2-Imidazolin-1-yl, Pyrazol-1-yl, Imidazol-1-yl, 2H-Tetrazol-2-yl, 1H-Tetrazol-1-yl, Pyrrol-1-yl, 2-Pyrrolin-1-yl und 3-Pyrrolin-1-yl, wobei 2H-Tetrazol-2-yl und 1H-Tetrazol-1-yl jeweils am Kohlenstoffglied mit 0 oder 1 von -C₀₋₄-AlkylR^{Z}, -C₀₋₄-AlkylSR^{Y}, -C₀₋₄-AlkylCO₂R^{Y} und Substituent HetR^{a} substituiert ist; und
iv) einem von 1,2,3,4-Tetrahydrochinolin-1-yl, 1,2,3,4-Tetrahydroisochinolin-2-yl, Indol-1-yl, Isoindol-2-yl, Indolin-1-yl, Benzimidazol-1-yl, 2,8-Diazaspiro[4,5]decan-1-on-8-yl, 4-{[(2-tert-Butoxycarbonylaminocyclobutan-carbonyl)-amino]-methyl}-piperidin-1-yl, 4-{[(2-Aminocyclobutancarbonyl)-amino]-methyl}-piperidin-1-yl, 3,9-Diazaspiro[5.5]undecan-3-carbonsäure-9-yl-tert-butylester, 4-Oxo-1-phenyl-1,3,8-triazaspiro[4.5]dec-8-yl und 4-Oxo-1,3,8-triazaspiro[4.5]-dec-8-yl;
wobei
Substituent HetR^{a} ein 4- bis 7-gliedriger heterocyclischer Ring ist, der einen Kohlenstoffglied-Bindungspunkt aufweist und ein Glied >NR^{M} als ein Heteroatomglied enthält und das Heteroatomglied vom Kohlenstoffglied-Bindungspunkt durch wenigstens 1 zusätzliches Kohlenstoffglied getrennt ist;
R^{K} ausgewählt ist aus der Gruppe, bestehend aus H, -C₁₋₄-Alkyl, -C₀₋₄-AlkylR^{Ar}, jeweils gegebenenfalls substituiert mit 1, 2 oder 3 Substituenten R^{N};
R^{L} ausgewählt ist aus der Gruppe, bestehend aus -CO₂R^{S} und -C(O)₂NR^{S}R^{S'};
R^{M} ausgewählt ist aus der Gruppe, bestehend aus R^{Z}, Indol-7-yl, -SO₂R^{Y}, -C₃₋₄-AlkylCO₂R^{Y}, -CO₂R^{Y}, -C(O)NR^{Z}OR^{Y}, -C(O)R^{Y}, -C(O)C₁₋₄-AlkylOR^{Y}, -C₀₋₄-AlkylC(O)NR^{S}R^{S'}, C₀₋₄-AlkylC(O)CO₂R^{Y}, 1,3-Dihydroindol-2-on-1-yl, 1,3-Dihydrobenzoimidazol-2-on-1-yl, Tetrazol-5-yl, 1-R^{Y}-1H-Tetrazol-5-yl, R^{Y}-Triazolyl, 2-R^{Y}-2H-Tetrazol-5-yl und -C₀₋₄-AlkylC(O)N(R^{Y})(SO₂R^{Y}), jeweils gegebenenfalls substituiert mit 1, 2 oder 3 Substituenten R^{N};
R^{N} ausgewählt ist aus der Gruppe, bestehend aus OCH₃, Cl, F, Br, I, OH, NH₂, CN, CF₃, CH₃, OC(O)CH₃ und NO₂;
R^{p} ausgewählt ist aus der Gruppe, bestehend aus R^{Y}, -C₂₋₄-AlkylOR^{Y}, R^{Ar}, -C₁₋₂-AlkylCO₂R^{Y}, -C₁₋₂-AlkylCONR^{S}R^{S'}, Indol-7-yl und -SO₂C₁₋₄-Alkyl;
R^{Q} ausgewählt ist aus der Gruppe, bestehend aus Fluor, Chlor, Brom, Iod, Trifluormethyl, Trichlormethyl, -CN, -C₁₋₄-Alkyl, -C₀₋₄-AlkylR^{Ar}, -C₀₋₄-AlkylR^{Ar'}, -C₀₋₄-AlkylOR^{Y}, -C₀₋₄-AlkylCO₂R^{Y}, -C₀₋₄-AlkylNR^{Y}R^{Z}, -C₀₋₄-AlkylNR^{Y}COR^{Y}, -C₀₋₄-AlkylNR^{Y}CONR^{Y}R^{Z}, -C₀₋₄-AlkylNR^{Y}SO₂R^{Y} und -C₀₋₄-AlkylSR^{Y};
R^{S} und R^{S'} unabhängig ausgewählt sind aus der Gruppe, bestehend aus H, -C₁₋₄-Alkyl und -C₀₋₄-Alkylphenyl; alternativ R^{S} und R^{S'} mit dem Stickstoffglied, an das das R^{S} und R^{S'} gebunden sind, zusammengenommen sind, um einen 4- bis 7-gliedrigen heterocyclischen Ring mit 0 oder 1 zusätzlichem Heteroatomglied, das ausgewählt ist aus der Gruppe, bestehend aus O, S und >NR^{Y}, zu bilden, vorausgesetzt, dass das zusätzliche Heteroatomglied vom Stickstoffglied, an das das R^{S} und R^{S'} gebunden sind, durch wenigstens zwei Kohlenstoffglieder getrennt ist, und vorausgesetzt, dass, wenn R^{Y} C₀₋₄-AlkylR^{Ar} ist, dann R^{Ar} nicht mit R^{L} substituiert ist;
R^{W} ausgewählt ist aus der Gruppe, bestehend aus R^{Y} und -C₃₋₇-Cycloalkyl;
R^{X} ausgewählt ist aus der Gruppe, bestehend aus -OR^{Y}, -NR^{Y}R^{Z}, -C₁₋₄-Alkyl und -C₀₋₄-AlkylR^{Ar};
R^{Y} ausgewählt ist aus der Gruppe, bestehend aus H, -C₁₋₄-Alkyl, -C₀₋₄-AlkylR^{Ar} und -C₀₋₄-AlkylR^{Ar'}, jeweils gegebenenfalls substituiert mit 1, 2 oder 3 Substituenten R^{N};
R^{Z} ausgewählt ist aus der Gruppe, bestehend aus R^{Y}, -C₂₋₄-AlkylOR^{Y}, -C₁₋₂-AlkylCO₂R^{Y}, -C₁₋₂-AlkylC(O)NR^{S}R^{S'} und -C₂₋₄-AlkylNR^{S}R^{S'};
wenn R^{Y} und R^{Z} an ein Stickstoffglied gebunden sind, R^{Y} und R^{Z} ausgewählt sind, wie oben definiert, oder R^{Y} und R^{Z} mit dem an R^{Y} und R^{Z} gebundenen Stickstoffglied zusammengenommen sind, um einen 4- bis 7-gliedrigen heterocyclischen Ring HetR^{d} mit 0 oder 1 zusätzlichen Heteroatomgliedem, die ausgewählt sind aus der Gruppe, bestehend aus O, S und >NR^{M}, zu bilden, wobei der 4- bis 7-gliedrige heterocyclische Ring HetR^{d} 0 oder 1 Carbonylglieder aufweist und der 4- bis 7-gliedrige heterocyclische Ring HetR^{d} 0 oder 1 valenzerlaubte Kohlenstoffglieder aufweist, die mit wenigstens einem von R^{M}, -CO₂H und -C₀₋₁-AlkylOR^{Y} substituiert sind;
R^{Ar} ein Rest mit einem Kohlenstoffglied-Bindungspunkt ist und der Rest ausgewählt ist aus der Gruppe, bestehend aus Phenyl, Pyridyl, Pyrimidyl und Pyrazinyl, wobei jedes valenzerlaubte Kohlenstoffglied in jedem der Reste unabhängig mit wenigstens einem von 0, 1, 2 oder 3 R^{N} und 0 oder 1 R^{L} substituiert ist;
R^{Ar'} ein 3- bis 8-gliedriger Ring ist, mit 0, 1 oder 2 Heteroatomgliedem, die ausgewählt sind aus der Gruppe, bestehend aus O, S, N und >NR^{Y}, mit 0, 1 oder 2 ungesättigten Bindungen, mit 0 oder 1 Carbonylgliedem, wobei jedes valenzerlaubte Glied in jedem der Ringe unabhängig mit 0, 1 oder 2 R^{K} substituiert ist; und
R^{f} ein linearer 3- bis 5-gliedriger Kohlenwasserstoffrest mit 0 oder 1 ungesättigten Kohlenstoff-Kohlenstoff-Bindungen und mit 0 oder 1 Carbonylgliedern ist;
vorausgesetzt, dass
(a) das R^{2'} und R^{3'} weiter die folgenden Bedingungen erfüllen:
(e1): das R^{2'} und R^{3'} sind nicht beide H, wenn Y O ist und X S ist;
(e2): wenn Y CH₂ ist, X N ist und das R^{2'} und R^{3'} Teile einer primären oder sekundären Aminogruppe sind, dann sind das R^{2'} und R^{3'} nicht aus der Gruppe ausgewählt, die aus H und Methyl besteht;
(e3): das R^{2'} und R^{3'}, zusammengenommen mit dem Stickstoffglied, an das sie gebunden sind, bilden keine Piperazingruppe, wenn X O ist und Y eines von O und CH₂ ist;
(e4): das R^{2'} und R^{3'}, zusammengenommen mit dem Stickstoffglied, an das sie gebunden sind, bilden keine Piperidingruppe, die mit einer gesättigten 6-gliedrigen cyclischen Gruppe monosubstituiert ist, wenn X O ist und Y eines von O und CH₂ ist; und
(e5): das R^{2'} und R^{3'}, zusammengenommen mit dem Stickstoffglied, an das sie gebunden sind, bilden weder eine substituierte Piperidingruppe noch eine substituierte Piperazingruppe, wobei die substituierte Piperidingruppe und die substituierte Piperazingruppe in der 4-Position mit einem Substituenten XG substituiert ist, wobei das XG die Struktur aufweist 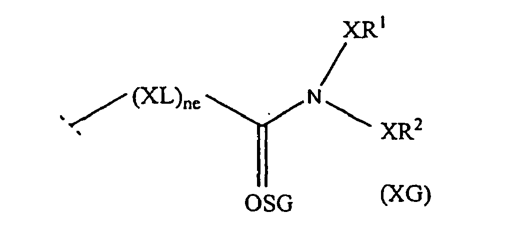
worin n = 0, 1, und wenn ne = 1, dann XL ein C₁₋₆-Alkyl ist, OSG O oder S ist und XR¹ und XR², zusammengenommen mit dem Stickstoffglied, an das sie gebunden sind, eine von einer Piperidingruppe, einer Piperazingruppe, einer Morpholingruppe, einer Thiomorpholingruppe und einer Pyrrolidingruppe bilden oder XR¹ und XR² jeweils unabhängig genommen eines von H, C₁₋₆-Alkyl, Aryl, Aralkyl, C₃₋₈-Cycloalkyl, C₃₋₈-Cycloalkyl-C₁₋₆-alkyl, Heteroalkyl, Heteroaryl-C₁₋₆-alkyl, Heterocycloalkyl und Heterocycloalkyl-C₁₋₆-alkyl sind; wobei das Aryl, Aralkyl, Cycloalkyl, Heteroaryl oder Heterocycloalkyl gegebenenfalls mit einem oder mehreren Substituenten substituiert sein kann, die unabhängig ausgewählt sein können aus Halogen, Hydroxy, C₁₋₆-Alkyl, C₁-₆-Alkoxy, halogeniertem C₁₋₆-Alkyl, halogeniertem C₁₋₆-Alkoxy, Nitro, Cyano, Amino, C₁₋₄Alkylamino, Di(C₁₋₄-alkyl)amino, Heteroaryl oder Heterocycloalkyl; und
(b) weiter vorausgesetzt, dass, wenn X S ist und Y O ist, dann eines von R²' und R³' nicht XCG ist, wenn das andere C₁₋₆-Alkyl ist, wobei XCG die Gruppe ist 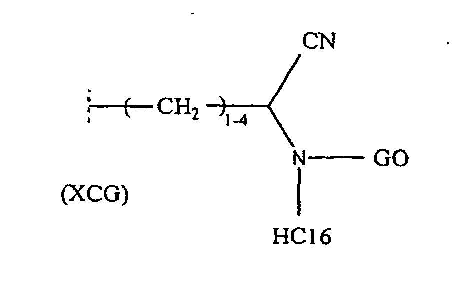
worin HC16 eines von H, C₁₋₆-Alkyl, Halo-C₁₋₆-alkyl, Allyl und C₁₋₆-Alkoxymethyl ist und GO eine Gruppe ist, die durch ein Kohlenstoffglied gebunden ist, das einen =O-Substituenten aufweist, wodurch eine Amidogruppe mit dem Stickstoffglied gebildet wird, an das die GO-Gruppe gebunden ist.

2. Verbindung, wie beansprucht in Anspruch 1, worin das R⁴ H ist.

3. Verbindung, wie beansprucht in Anspruch 1, worin das R² und R³ jeweils unabhängig ausgewählt sind aus
(i) der Gruppe, bestehend aus A), B), C), D), E) und I); oder
(ii) Gruppe A); oder
(iii) Gruppe B); oder
(iv) Gruppe C); oder
(v) Gruppe D); oder
(vi) Gruppe E); oder
(vii) Gruppe I), wie definiert in Anspruch 1.

4. Verbindung, wie beansprucht in Anspruch 1, worin das R² und R³ mit dem Stickstoff, an das sie gebunden sind, zusammengenommen sind, um einen heterocyclischen Ring zu bilden, der wenigstens ein Heteroatomglied enthält, das der Bindungsstickstoff ist, wobei der heterocyclische Ring ausgewählt ist aus einer von
(a) der Gruppe, bestehend aus i) und ii);
(b) der Gruppe i) und
(c) der Gruppe ii),
wie definiert in Anspruch 1.

5. Verbindung, wie beansprucht in Anspruch 1, wobei die Verbindung eine von:
2-(4-Piperidin-1-ylmethylphenoxy)-benzooxazol; und
2-(2-Fluor-4-piperidin-1-ylmethylphenoxy)-benzooxazol
ist.

6. Verbindung, wie beansprucht in Anspruch 1, wobei die Verbindung eine von:
1-[4-(Benzothiazol-2-yloxy)-benzyl]-piperidin-4-carbonsäure;
1 - {1-[4-(Benzothiazol-2-yloxy)-benzyl]-piperidin-4-yl}-pyrrolidin-2-on;
2-(2-Fluor-4-piperidin-1-ylmethylphenoxy)-benzothiazol;
1-(2-{[4-(Benzothiazol-2-yloxy)-benzyl]-cyclopropylamino}-ethyl)- 4-hydroxypyrrolidin-2-on;
N-{1-[4-(Benzothiazol-2-yloxy)-benzyl]-piperidin-4-yl}-N-methylmethansulfonamid;
2-{4-[4-(1H-Tetrazol-5-yl)-piperidin-1-ylmethyl]-phenoxy}-benzothiazol;
1-{4-[4-(Benzothiazol-2-yloxy)-benzyl]-piperazin-1-yl}-2-hydroxyethanon;
N-{1-[4-(Benzothiazol-2-yloxy)-benzyl]-piperidin-4-ylmethyl}-methansulfonamid;
3-{1-[4-(Benzothiazol-2-yloxy)-benzyl]-piperidin-4-yl}-oxazolidin-2-on;
4-{1-[4-(Benzothiazol-2-yloxy)-benzyl]-piperidin-4-yl}-morpholin-3-on;
2-(4-Piperidin-1-ylmethylphenoxy)-benzothiazol;
1-[4-(Benzothiazol-2-yloxy)-benzyl]-4-phenylpiperidin-4-ol;
1-[4-(Benzothiazol-2-yloxy)-benzyl]-piperidin-4-ol;
{1-[4-(Benzothiazol-2-yloxy)-benzyl]-piperidin-4-yl}-methanol;
N-{1-[4-(Benzothiazol-2-yloxy)-benzyl]-piperidin-4-yl}-methansulfonamid;
N-{1-[4-(Benzothiazol-2-yloxy)-benzyl]-piperidin-4-ylmethyl}-2-hydroxyacetamid;
{1-[4-(Benzothiazol-2-yloxy)-benzyl]-piperidin-4-yl}-carbamidsäuremethylester;
{1-[4-(Benzothiazol-2-yloxy)-benzyl]-piperidin-4-ylmethyl}-harnstoff ;
N-{1-[4-(Benzothiazol-2-yloxy)-benzyl]-piperidin-4-ylmethyl}-2,2,2-trifluoracetamid;
{4-[4-(Benzothiazol-2-yloxy)-benzyl]-piperazin-1-yl]-essigsäure;
2-[4-(4-Methansulfonylpiperazin-1-ylmethyl)-phenoxy]-benzothiazol;
1-{4-[4-(Benzothiazol-2-yloxy)-benzyl]-piperazin-1-yl}-2,2,2-trifluorethanon;
2-(4-Morpholin-4-ylmethylphenoxy)-benzothiazol;
{1-[4-(Benzothiazol-2-yloxy)-benzyl]-piperidin-4-yl}-carbamidsäurephenylester;
N- {1-[4-(Benzothiazol-2-yloxy)-benzyl]-piperidin-4-yl}-benzolsulfonamid;
3-[4-(Benzothiazol-2-yloxy)-benzylamino]-propionsäureethylester;
3-[4-(Benzothiazol-2-yloxy)-benzylamino]-propionsäure;
1-{3-[4-(Benzothiazol-2-yloxy)-benzylamino]-propyl}-pyrrolidin-2-on;
1-(3 {[4-(Benzothiazol-2-yloxy)-benzyl]-methylamino}-propyl)-pyrrolidin-2-on;
1-(3-{[4-(Benzothiazol-2-yloxy)-benzyl]-isopropylamino}-propyl)-pyrrolidin-2-on;
1-(3-{[4-(Benzothiazol-2-yloxy)-benzyl]-ethylamino}-propyl)-pyrrolidin-2-on;
[4-(Benzothiazol-2-yloxy)-benzyl]-cyclopropylamin;
N-1-[4-(Benzothiazol-2-yloxy)-benzyl]-N1-cyclopropylpropan-1,3-diamin;
N-(3-{[4-(Benzothiazol-2-yloxy)-benzyl]-cyclopropylamino}-propyl)-isobutyramid;
1-(3-{[4-(Benzothiazol-2-yloxy)-benzyl]-cyclopropylamino}-propyl)-3-isopropyl-harnstoff;
1-{1-[4-(Benzothiazol-2-yloxy)-benzyl]-piperidin-4-yl}-3-isopropylharnstoff;
N-1-[4-(Benzothiazol-2-yloxy)-benzyl]-piperidin-4-yl}-oxalamidsäuremethylester;
N-{1-[4-(Benzothiazol-2-yloxy)-benzyl]-piperidin-4-yl}-isobutyramid;
Tetrahydrofuran-2-carbonsäure-{1-[4-(benzothiazol-2-yloxy)-benzyl]-piperidin-4-yl}-amid;
1-{1-[4-(Benzothiazol-2-yloxy)-benzyl]-piperidin-4-yl}-4-hydroxypyrrolidin-2-on;
1-{1-[4-(Benzothiazol-2-yloxy)-benzyl]-piperidin-4-yl}-4-hydroxypyrrolidin-2-on;
{1-[4-(Benzothiazol-2-yloxy)-benzyl]-piperidin-4-yl]-harnstoff;
N-{1-[4-(Benzothiazol-2-yloxy)-benzyl]-piperidin-4-yl}-oxalamidsäure;
N- {1-[4-(Benzothiazol-2-yloxy)-benzyl]-piperidin-4-yl}-2-hydroxyacetamid;
N-{1-[4-(Benzothiazol-2-yloxy)-benzyl]-piperidin-4-yll-2,2,2-trifluoracetamid;
2-[4-(1,1-Dioxo-1 | 6-thiomorpholin-4-ylmethyl)-phenoxy]-benzothiazol;
N-{1-[4-(Benzothiazol-2-yloxy)-benzyl]-piperidin-4-aminosulfonyl}-carbamidsäure-tert-butylester;
N-{1-[4-(Benzothiazol-2-yloxy)-benzyl]-piperidin-4-yl}-acetamid;
N-{1-[4-(Benzothiazol-2-yloxy)-benzyl]-piperidin-4-yl}-N,N-dimethylsulfamid;
1- {1-[4-(Benzothiazol-2-yloxy)-benzyl]-piperidin-4-yl}-3-ethylharnstoff;
1-{1-[4-(Benzothiazol-2-yloxy)-benzyl]-piperidin-4-yl}-3-ethylthioharnstoff;
Propan-1-sulfonsäure- {1-[4-(benzothiazol-2-yloxy)-benzyl]-piperidin-4-yl}-amid;
Propan-2-sulfonsäure-{1-[4-(benzothiazol-2-yloxy)-benzyl]-piperidin-4-yl}-amid;
N-{1-[4-(Benzothiazol-2-yloxy)-benzyl]-piperidin-4-yl}-sulfamid;
N- {1-[4-(Benzothiazol-2-yloxy)-benzyl]-piperidin-4-yl}-formamid;
{1-[4-(Benzothiazol-2-yloxy)-benzyl]-piperidin-4-yl}-carbamidsäureethylester;
N-{1-[4-(Benzothiazol-2-yloxy)-benzyl]-piperidin-4-yl}-propionamid;
N-{1-[4-(Benzothiazol-2-yloxy)-benzyl]-piperidin-4-yl}-butyramid;
1-{1-[4-(Benzothiazol-2-yloxy)-benzyl]-piperidin-4-yl}-3-propylharnstoff;
{1-[4-(Benzothiazol-2-yloxy)-benzyl]-piperidin-4-yl}-carbamidsäurepropylester;
{1-[4-(Benzothiazol-2-yloxy)-benzyl]-piperidin-4-yl}-3-methylharnstoff;
{1-[4-(Benzothiazol-2-yloxy)-benzyl]-piperidin-4-yl}-1,3-dimethylharnstoff;
{1-[4-(Benzothiazol-2-yloxy)-benzyl]-piperidin-4-yl}-1-methylharnstoff;
N-{1-[4-(Benzothiazol-2-yloxy)-benzyl]-piperidin-4-yl}-N-methylacetamid;
{1-[4-(Benzothiazol-2-yloxy)-benzyl]-piperidin-4-yl}-N-methylcarbamidsäure-methylester;
N-{1-[4(Benzothiazol-2-yloxy)-benzyl]-piperidin-4-yl}-N-methyloxalamidsäure-methylester;
N-{1-[4-(Benzothiazol-2-yloxy)-benzyl]-piperidin-4-yl}-N-methyloxalamidsäure;
Guanidin, N-{1-[4-(Benzothiazol-2-yloxy)-benzyl]-piperidin-4-yl}-N'-hydroxy;
{1-[4-(Benzothiazol-2-yloxy)-benzyl]-piperidin-4-yl}-carbamidsäureisopropylester;
3- {1-[4-(Benzothiazol-2-yloxy)-benzyl]-piperidin-4-yl}-1,1-dimethylharnstoff;
Essigsäure-{1-[4-(benzothiazol-2-yloxy)-benzyl]-piperidin-4-ylcarbamoyl}-methylester;
{1-[4-(Benzothiazol-2-yloxy)-benzyl]-piperidin-4-yl}-thioharnstoff;
1'-[4-(Benzothiazol-2-yloxy)-benzyl]-[1,4']bipiperidinyl ;
{4-[4-(Benzothiazol-2-yloxy)-benzyl]-piperazin-1-yl}-(tetrahydrofuran-2-yl)-methanon;
{1-[4-(Benzothiazol-2-yloxy)-benzyl]-piperidin-4-yl}-carbamidsäure-tert-butylester;
4-[4-(Benzothiazol-2-yloxy)-benzyl]-piperazin-1-carbonsäure-tert-butylester;
1-[4-(Benzothiazol-2-yloxy)-benzyl]-piperidin-4-ylamin;
2-(4-Piperazin-1-ylmethylphenoxy)-benzothiazol;
4-[4-(Benzothiazol-2-yloxy)-benzyl]-piperazin-1-carbonsäureamid;
2-[4-(4-Benzolsulfonylpiperazin-1-ylmethyl)-phenoxy]-benzothiazol;
C-{1-[4-(Benzothiazol-2-yloxy)-benzyl]-piperidin-4-yl)-methylamin;
2-(2- {4-[4-(Benzothiazol-2-yloxy)-benzyl]-piperazin-1-yl}-2-oxoethyl)-cyclopentanon;
{4-[4-(Benzothiazol-2-yloxy)-benzyl]-piperazin-1-yl}-essigsäureethylester;
4- {1-[4-(Benzothiazol-2-yloxy)-benzyl]-piperidin-4-yl]-buttersäure-4-(benzothiazol-2-yloxy)-benzylester;
1-(3- {[4-(Benzothiazol-2-yloxy)-benzyl]-cyclopropylamino}-propyl)-pyrrolidin-2-on;
(3-{[4-(Benzothiazol-2-yloxy)-benzyl]-cyclopropylamino}-propyl)-carbamidsäure-tert-butylester;
Tetrahydrofuran-2-carbonsäure-(3-{[4-(benzothiazol-2-yloxy)-benzyl]-cyclopropylamino}-propyl)-amid;
1-(3- {[4-(Benzothiazol-2-yloxy)-benzyl]-cyclopropylamino}-propyl)-4-hydroxypyrrolidin-2-on;
(2- {[4-(Benzothiazol-2-yloxy)-benzyl]-cyclopropylamino}-ethyl)-carbamidsäure-tert-butylester;
N 1-[4-(Benzothiazol-2-yloxy)-benzyl]-N1-cyclopropylethan-1,2-diamin;
Ethansulfonsäure-{1-[4-(benzothiazol-2-yloxy)-benzyl]-piperidin-4-yl}-amid;
1-{1-[4-(Benzothiazol-2-yloxy)-benzyl]-piperidin-4-yl}-pyrrol-2,5-dion;
{1-[4-(Benzothiazol-2-yloxy)-benzyl]-piperidin-4-yl}-methylcarbamidsäure-tert-butylester;
{1-[4-(Benzothiazol-2-yloxy)-benzyl]-piperidin-4-yl}-methylamin;
1-[4-(Benzothiazol-2-yloxy)-benzyl]-piperazin-2-carbonsäure;
[4-(Benzothiazol-2-yloxy)-benzyl]-methylamin;
Benzothiazol-2-yl-{1-[4-(benzothiazol-2-yloxy)-benzyl]-piperidin-4-yl}-amin;
(3-{[4-(Benzothiazol-2-yloxy)-benzyl]-methylamino)-propyl)-carbamidsäure-tert-butylester;
4-({[4-(Benzothiazol-2-yloxy)-benzyl]-methylamino}-methyl)-phenol;
N1-[4-(Benzothiazol-2-yloxy)-benzyl]-N1-methylpropan-1,3-diamin;
Essigsäure-(3{[4-(benzothiazol-2-yloxy)-benzyl]-methylamino}-propylcarbamoyl)-methylester;
N-(3-{[4-(Benzothiazol-2-yloxy)-benzyl]-methylamino}-propyl)-2-hydroxyacetamid;
N-(3-{[4-(Benzothiazol-2-yloxy)-benzyl]-methylamino}-propyl)-methansulfonamid;
6-Chlor-2-(4-piperidin-1-ylmethylphenoxy)-benzothiazol;
6-Methoxy-2-(4-piperidin-1-ylmethylphenoxy)-benzothiazol;
4-[4-(Benzothiazol-2-yloxy)-benzyl]-piperazin-1-sulfonsäuredimethylamid;
2-{4-[4-(Benzothiazol-2-yloxy)-benzyl]-piperazin-1-yl}-1-pyrrolidin-1-ylethanon;
2-{4-[4-(Benzothiazol-2-yloxy)-benzyl]-piperazin-1-yl}-1-morpholin- 4-ylethanon;
{4-[4-(Benzothiazol-2-yloxy)-benzyl]-piperazin-1-yl}-thiophen-2-ylmethanon;
4-Methyl-2-(4-piperidin-1-ylmethylphenoxy)-benzothiazol;
4-Chlor-2-(4-piperidin-1-ylmethylphenoxy)-benzothiazol;
2-[4-(4,4-Difluorpiperidin-1-ylmethyl)-phenoxy]-benzothiazol;
2-Aminocyclobutancarbonsäure-{1-[4-(benzothiazol-2-yloxy)-benzyl]-piperidin-4-ylmethyl}-amid;
[4- (Benzothiazol-2-yloxy)-benzyl]-methyl-(1-methylpiperidin-4-yl)-amin;
3-{[4-(Benzothiazol-2-yloxy)-benzyl]-methylamino}-propionitiril;
4-[4-(Benzothiazol-2-yloxy)-benzyl]-piperazin-2-on;
2-[4-(3-Methylpiperidin-1-ylmethyl)-phenoxy]-benzothiazol;
Essigsäure-({1-[4-(benzothiazol-2-yloxy)-benzyl]-piperidin-4-yl}-methylcarbamoyl)-methylester;
N-{1-[4-(Benzothiazol-2-yloxy)-benzyl]-piperidin-4-yl}-2-hydroxy-N-methylacetamid; und
1-[4-(Benzothiazol-2-yloxy)-benzyl]-piperidin-4-carbonsäureethylester
ist.

7. Verbindung, wie beansprucht in Anspruch 1, wobei die Verbindung eine von:
2-(4-Piperidin-1-ylmethylphenoxy)-1H-benzoimidazol; und
1-[4-(1H-Benzoimidazol-2-yloxy)-benzyl]-piperidin-4-carbonsäure
ist.

8. Pharmazeutische Zusammensetzung, die wenigstens eine Verbindung umfasst, wie beansprucht in einem der Ansprüche 1-7.

9. Verwendung einer therapeutisch wirksamen Menge wenigstens eines LTA4H-Modulators, ausgewählt aus den Verbindungen von Anspruch 1, zur Herstellung eines Arzneimittels zur Behandlung, Verhinderung oder Hemmung von Entzündung bei einem Patienten.

10. Verwendung, wie beansprucht in Anspruch 9, wobei die Entzündung auf wenigstens einem von Asthma, chronischer obstruktiver Lungenerkrankung, Atherosklerose, rheumatoider Arthritis, multipler Sklerose, entzündlicher Darmerkrankung und Psoriasis beruht.

11. Verwendung einer hemmenden Menge wenigstens eines LTA4H-Modulators, ausgewählt aus Verbindungen, wie beansprucht in Anspruch 1, zur Herstellung eines Arzneimittels zur Hemmung der LTA4H-Enzymaktivität.

12. Verwendung, wie beansprucht in einem der Ansprüche 9-11, wobei das R⁴ H ist.

13. Verwendung, wie beansprucht in einem der Ansprüche 9-11, wobei das R² und R³ jeweils unabhängig ausgewählt sind aus
(i) der Gruppe, bestehend aus A), B), C), D), E) und I); oder
(ii) Gruppe A); oder
(iii) Gruppe B); oder
(iv) Gruppe C); oder
(v) Gruppe D); oder
(vi) Gruppe E); oder
(vii) Gruppe I),
wie definiert in Anspruch 1.

14. Verwendung wie beansprucht in einem der Ansprüche 9-11, worin das R² und R³ mit dem Stickstoff, an das sie gebunden sind, zusammengenommen sind, um einen heterocyclischen Ring zu bilden, der wenigstens ein Heteroatomglied enthält, das der Bindungsstickstoff ist, wobei der heterocyclische Ring ausgewählt ist aus einer von
(a) der Gruppe, bestehend aus i) und ii);
(b) der Gruppe i) und
(c) der Gruppe ii), wie definiert in Anspruch 1.

15. Verwendung, wie beansprucht in einem der Ansprüche 9-11, wobei der wenigstens eine LTA4H-Modulator ausgewählt ist aus den Verbindungen, wie beansprucht in einem der Ansprüche 5-7.

## Revendications

1. Composé de formule (II) : 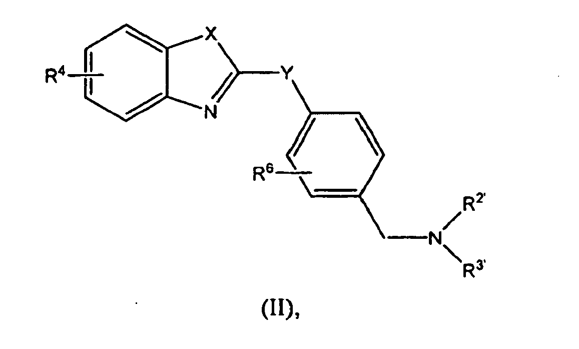 ou un énantiomère, un diastéromère, un racémique, un tautomère, un hydrate, un solvate, ou un sel pharmaceutiquement acceptable, un ester, ou un amide de celui-ci,
où
X est choisi parmi le groupe constitué par NR⁵, O et S, R⁵ étant l'un parmi H et CH₃ ;
Y est choisi parmi le groupe constitué par CH₂ et O ;
R⁴ est choisi parmi le groupe constitué par H, OCH₃, Cl, F, Br, I, OH, NH₂, CN, CF₃ et CH₃ ;
R⁶ est H ou F ; et
R^{2'} est défini en tant que R² et R^{3'} est défini en tant que R³, de la façon suivante :
R² et R³ sont chacun indépendamment choisi parmi le groupe constitué par
A) H, alkyle en C₁₋₇, alcényle en C₃₋₇, où le carbone dans ledit alcényle qui est fixé au chaînon azote n'a que des liaisons simples, alcynyle en C₃₋₇, où le carbone dans ledit alcynyle qui est fixé au chaînon azote n'a que des liaisons simples, cycloalkyle en C₃₋₇ éventuellement benzofusé, cycloalcényle en C₅₋₇, -C₃₋₇cycloalkylC₁₋₇alkyle, -C₁₋₇alkylC₃₋₇cycloalkyle et phényle, où chacun des substituants A) est indépendamment substitué avec 0, 1 ou 2 R^{Q}, et chacun desdits R^{Q} est un substituant au niveau d'un chaînon carbone qui est au moins un chaînon carbone éliminé du chaînon azote ;
B) un substituant HetR^{a} ;
C) -C₁₋₇alkylC(O)R^{x} , éventuellement substitué avec CH₂R^{Ar} ou CH₂R^{Ar'} ;
D) -C₂₋₅alkylC(O)R^{x}, où deux chaînons carbone à valence permise dans l'alkyle en C₂₋₅ dudit -C₂₋₅alkylC(O)R^{x} font partie d'un C₃₋₆carbocycle saturé ;
E) -C₂₋₅alkylOH, où deux chaînons carbone à valence permise dans l'alkyle en C₂₋₅ dudit -C₂₋₅alkylOH font partie d'un carbocycle en C₃₋₆ saturé ;
F) alkylphényle en C₀₋₄, où le phényle dans ledit alkylphényle en C₀₋₄ est fondu au niveau de deux chaînons carbone adjacents dans ledit phényle au R^{f}, ou est benzofusé ;
G) -C₀₋₄alkylAr⁶ où Ar⁶ est un hétéroaryle à 6 chaînons ayant un point de fixation de chaînon carbone et ayant un ou deux chaînons hétéroatomes -N=, et benzofusé ;
H) -C₀₋₄alkylAr⁵, où Ar⁵ est un hétéroaryle à 5 chaînons, ayant un chaînon hétéroatome choisi parmi le groupe constitué par O, S et <NR^{Y}, et ayant 0 ou 1 chaînon hétéroatome -N= supplémentaire, contenant éventuellement deux groupes carbonyles, et éventuellement benzofusé ;
I) -C₁₋₄alkylAr^{5'}, où Ar^{5'} est un hétéroaryle à 5 chaînons contenant 3 ou 4 chaînons azote, éventuellement substitué avec R^{Y}, et ayant un site à valence permise en tant que point de fixation ;
J) -C₀₋₄alkylAr⁶⁻⁶, où Ar⁶⁻⁶ est un phényle fixé à un alkyle en C₀₋₄ fusé au niveau de sites à valence permise à un hétéroaryle à 6 chaînons, où ledit hétéroaryle à 6 chaînons a un ou deux chaînons hétéroatomes -N= ;
K) -C₀₋₄alkylAr⁶⁻⁵, où Ar⁶⁻⁵ est un phényle fixé à un alkyle en C₀₋₄ fusé au niveau de sites à valence permise à un hétéroaryle à 5 chaînons, ledit hétéroaryle à 5 chaînons ayant un chaînon hétéroatome choisi parmi le groupe constitué par O, S et <NR^{Y}, et ledit hétéroaryle à 5 chaînons ayant 0 ou 1 chaînon hétéroatome supplémentaire qui est -N= ;
L) l'un parmi 2-(4-éthyl-phénoxy)-benzothiazole, 2-(4-éthyl-phénoxy)-benzooxazole, et 2-(4-éthyl-phénoxy)-1*H*-benzoimidazole ; et
M) SO₂C₁₋₄alkyle ;
de façon alternative, R² et R³ sont pris ensemble avec l'azote auquel ils sont fixés pour former un hétérocycle qui contient au moins un chaînon hétéroatome qui est ledit azote de fixation, ledit hétérocycle étant choisi parmi le groupe constitué par
i) un hétérocycle HetR^{b} à 4 à 7 chaînons, ledit hétérocycle HetR^{b} à 4 à 7 chaînons ayant un chaînon hétéroatome qui est ledit azote de fixation, et étant substitué avec 0, 1 ou 2 substituants au niveau de chaînons de substitution identiques ou différents, lesdits substituants étant choisis parmi le groupe constitué par -R^{Y}, -CN, -C (O) R^{Y}, -C₀₋₄alkylCO₂R^{Y}, -C₀₋₄alkylC (O) CO₂R^{Y}, -C₀₋₄alkylOR^{Y}, -C₀₋₄alkylC (O) NR^{Y}R^{Z}, -C₀₋₄alkylNR^{Y}C (O) R^{Z}, -C (O) NR^{Z}OR^{Y}, -C₀₋₄alkylNR^{Y}C (O) CH₂OR^{Y}, -C₀₋₄alkylNR^{Y}C (O) CH₂C (O) R^{y}, -C₀₋₄alkylNR^{Y}CO₂R^{Y}, -C₀₋₄alkylNR^{Y}C (O) NR^{Y}R^{Z}, -C₀₋₄alkylNR^{Y}C (S) NR^{Y}R^{Z}, -NR^{Y}C (O) CO₂R^{Y}, -NR^{Y}R^{Z}, -C₀₋₄alkylNR^{W}SO₂R^{Y}, 1,3-dihydro-indol-2-one-1-yle, 1,3-dihydro-benzoimidazol-2-one-1-yle, tétrazol-5-yle, 1-R^{Y}-1*H*-tétrazol-5-yle, R^{Y}-triazol, 2-R^{Y}-2*H*-tétrazol-5-yle, pyrrolidine-2-thion-1-yle, pipéridine-2-thion-1-yle, -C₀₋₄alkylC (O) N (R^{Y}) (SO₂R^{Y}), -C₀₋₄alkylN (R^{Y}) (SO₂) NR^{Y}R^{Y}, -C₀₋₄alkylN (R^{Y}) (SO₂) NR^{Y}CO₂R^{Y}, halo, 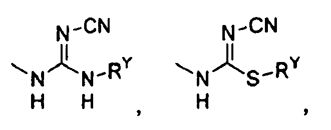 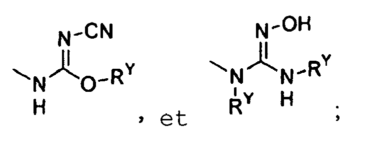
ii) un hétérocycle HetP^{c} à 5 à 7 chaînons, ledit hétérocycle HetR^{c} à 5 à 7 chaînons ayant un chaînon hétéroatome supplémentaire séparé dudit azote de fixation par au moins un chaînon carbone, ledit chaînon hétéroatome supplémentaire étant choisi parmi le groupe constitué par O, S(=O)₀₋₂, et >NR^{M}, ledit hétérocycle HetR^{c} à 5 à 7 chaînons ayant 0 ou 1 chaînon carbonyle, et étant substitué avec 0, 1 ou 2 substituants au niveau de chaînons de substitution de carbone identiques ou différents, lesdits substituants étant choisis parmi le groupe constitué par -C(O)R^{Y}, -CO₂R^{Y}, -C₃₋₄alkylCO₂R^{Y} et R^{Z} ;
iii) l'un parmi imidazolidin-1-yle, 2-imidazolin-1-yle, pyrazol-1-yle, imidazol-1-yle, 2*H*-tétrazol-2-yle, 1*H*-tétrazol-1-yle, pyrrol-1-yle, 2-pyrrolin-1-yle, et 3-pyrrolin-1-yle, où chacun desdits 2*H*-tétrazol-2-yle et 1*H*-tétrazol-1-yle est substitué au niveau du chaînon carbone avec 0 ou 1 de -C₀₋₄alkylR^{Z}, -C₀₋₄alkylSR^{Y}, -C₀₋₄alkylCO₂R^{Y}, et le substituant HetR^{a} ; et
iv) l'un parmi 1,2,3,4-tétrahydro-quinolin-1-yle, 1,2,3,4-tétrahydro-isoquinolin-2-yle, indol-1-yle, isoindol-2-yle, indolin-1-yle, benzimidazol-1-yle, 2,8-diaza-spiro[4,5]décan-1-one-8-yle, 4-{[2-tert-butoxycarbonylamino-cyclobutanecarbonyl)-amino]-méthyl}-pipéridin-1-yle, 4-{[2-amino-cyclobutanecarbonyl)-amino]-méthyl}-pipéridine-1-yle, 9-yl-tert-butylester d'acide 3,9-diaza-spiro[5,5]undécane-3-carboxylique, 4-oxo-1-phényl-1,3,8-triaza-spiro[4,5]déc-8-yle, et 4-oxo-1,3,8-triaza-spiro[4,5]déc-8-yle ;
où
le substituant HetR^{a} est un hétérocycle à 4 à 7 chaînons ayant un point de fixation de chaînon carbone et contenant un chaînon >NR^{M} en tant que chaînon hétéroatome, et ledit chaînon hétéroatome étant séparé dudit point de fixation de chaînon carbone par au moins un chaînon carbone supplémentaire ;
R^{K} est choisi parmi le groupe constitué par H, alkyl en C₁₋₄, -C₀₋₄alkylR^{Ar}, chacun étant éventuellement substitué avec 1, 2 ou 3 substituants R^{N}
R^{L} est choisi parmi le groupe constitué par -CO₂R^{S} et -C (O)NR^{S}R^{S} ;
R^{M} est choisi parmi le groupe constitué par R^{Z}, indol-7-yle, -SO₂R^{Y}, -C₃₋₄alkylCO₂R^{Y}, -CO₂R^{Y}, -C(O)NR^{Z}OR^{Y}, -C(O)R^{Y}, -C (O) C₁₋₄alkylOR^{Y}, -C₀₋₄alkylC (O) NR^{S}R^{S'}, -C₀₋₄alkylC(O)CO₂R^{Y}, 1,3-dihydro-indol-2-one-1-yle, 1,3-dihydro-benzoimidazol-2-one-1-yle, tétrazol-5-yle, 1-R^{Y}-1H-tétrazol-5-yle, R^{Y}-triazol, 2-R^{Y}-2*H*-tétrazol-5-yle, et -C₀₋₄alkylC (O) N (R^{Y}) (SO₂R^{Y}), chacun étant éventuellement substitué avec 1, 2 ou 3 substituants R^{N} ;
R^{N} est choisi parmi le groupe constitué par OCH₃, Cl, F, Br, I, OH, NH₂, CN, CF₃, CH₃, OC(O)CH₃ et NO₂ ;
R^{P} est choisi parmi le groupe constitué par R^{y}, -C₂₋₄alkylOR^{Y}, R^{Ar}, -C₁₋₂alkylCO₂R^{Y}, -C₁₋₂alkylCONR^{S}R^{S'}, indol-7-yle et -SO₂C₁₋₄alkyle ;
R^{Q} est choisi parmi le groupe constitué par fluoro, chloro, bromo, iodo, trifluorométhyle, trichlorométhyle, -CN, alkyle en C₁₋₄, -C₀₋₄alkylR^{Ar}, -C₀₋₄alkylR^{Ar'}, -C₀₋₄alkylOR^{Y}, -C₀₋₄alkylCO₂R^{Y}, -C₀₋₄alkylNR^{Y}R^{Z}, -C₀₋₄alkylNR^{Y}COR^{Y}, -C₀₋₄alkylNR^{Y} CONR^{Y}R^{Z} -C₀₋₄alkylNR^{Y}SO₂R^{Y}, et -C₀₋₄alkylSR^{Y} ;
R^{S} et R^{S'} sont indépendamment choisis parmi le groupe constitué par H, alkyle en C₁₋₄, et alkylphényle en C₀₋₄; de façon alternative, R^{S} et R^{S'} sont pris ensemble avec le chaînon azote auquel lesdits R^{S} et R^{S'} sont fixés pour former un hétérocycle à 4 à 7 chaînons ayant 0 ou 1 chaînon hétéroatome supplémentaire choisi parmi le groupe constitué par O, S et >NR^{Y}, à condition que ledit chaînon hétéroatome supplémentaire soit séparé par au moins deux chaînons carbone dudit chaînon azote auquel sont fixés R^{S} et R^{S'}, et à condition que lorsque R^{Y} est C₀₋₄alkylR^{Ar}, alors R^{Ar} n'est pas substitué avec R^{L} ;
R^{W} est choisi parmi le groupe constitué par R^{Y}, et cycloalkyle en C₃₋₇;
R^{X} est choisi parmi le groupe constitué par -OR^{Y}, -NR^{Y}R^{Z}, alkyle en C₁₋₄, et -C₀₋₄alkylR^{Ar} ;
R^{Y} est choisi parmi le groupe constitué par H, alkyle en C₁₋₄, -C₀₋₄alkylR^{Ar} et -C₀₋₄alkylR^{Ar'}, chacun étant éventuellement substitué avec 1, 2 ou 3 substituants R^{N} ; R^{Z} est choisi parmi le groupe constitué par R^{Y}, -C₂₋₄alkylOR^{Y}, -C₁₋₂alkylCOR^{Y}, -C₁₋₂alkylC (O) NR^{S}R^{S'}, et -C₂₋₄alkylNR^{S}R^{S'} ;
lorsque R^{Y} et R^{Z} sont fixés à un chaînon azote, R^{Y} et R^{Z} sont choisis tel que défini ci-dessus, ou R^{Y} et R^{Z} sont pris ensemble avec le chaînon azote fixé à R^{Y} et R^{Z} pour former un hétérocycle HetR^{d} à 4 à 7 chaînons ayant 0 ou 1 chaînon hétéroatome supplémentaire choisi parmi le groupe constitué par 0, S et >NR^{M}, ledit hétérocycle HetR^{d} à 4 à 7 chaînons ayant 0 ou 1 chaînon carbonyle, et ledit hétérocycle HetR^{d} à 4 à 7 chaînons ayant 0 ou 1 chaînon à valence permise substitué avec au moins un parmi R^{M}, -CO₂H et -C₀₋₁alkylOR^{Y} ;
R^{Ar} est un fragment avec un point de fixation de chaînon carbone, et ledit fragment est choisi parmi le groupe constitué par phényle, pyridyle, pyrimidyle et pyrazinyle, où chaque chaînon carbone à valence permise dans chacun desdits fragments est indépendamment substitué avec au moins un parmi 0, 1, 2 ou 3 R^{N}, et 0 ou 1 R^{L} ;
R^{Ar'} est un cycle à 3 à 8 chaînons, ayant 0, 1 ou 2 chaînons hétéroatomes choisis parmi le groupe constitué par O, S, N et >NR^{Y}, ayant 0, 1 ou 2 liaisons non saturées, ayant 0 ou 1 chaînon carbonyle, où chaque chaînon à valence permise dans chacun desdits cycles est indépendamment substitué avec 0, 1 ou 2 R^{K} ; et
R^{f} est un fragment hydrocarbure linéaire à 3 à 5 chaînons ayant 0 ou 1 liaison carbone-carbone non saturée et ayant 0 ou 1 chaînon carbonyle ;
à condition que
(a) lesdits R^{2'} et R^{3'} remplissent en outre les conditions suivantes :
(e1) lesdits R^{2'} et R^{3'} ne soient pas tous les deux H, lorsque Y est 0, et X est S ;
(e2) lorsque Y est CH₂, X est N, et lesdits R^{2'} et R^{3'} font partie d'un groupe amino primaire ou secondaire, alors lesdits R^{2'} et R^{3'} ne sont pas choisis parmi le groupe constitué par H et méthyle ;
(e3) lesdits R^{2'} et R^{3'} pris ensemble avec le chaînon azote auquel ils sont fixés ne forment pas un groupe pipérazine, lorsque X est O, et Y est l'un parmi O et CH₂ ;
(e4) lesdits R^{2'} et R^{3'} pris ensemble avec le chaînon azote auquel ils sont fixés ne forment pas un groupe pipéridine qui est mono-substitué avec un groupe cyclique saturé à 6 chaînons, lorsque X est 0, et Y est l'un parmi 0 et CH₂ ; et
(e5) lesdits R^{2'} et R^{3'} pris ensemble avec le chaînon azote auquel ils sont fixés ne forment ni un groupe pipéridine substitué ni un groupe pipérazine substitué, où ledit groupe pipéridine substitué ou ledit groupe pipérazine substitué est substitué en 4^{ème} position avec un substituant XG, ledit XG ayant la structure : 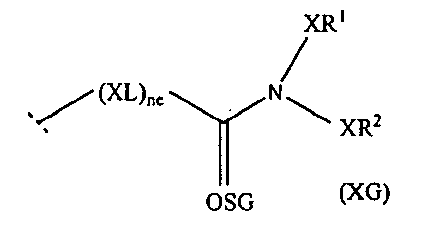 où n = 0, 1 et lorsque ne = 1, alors XL est un alkyle en C₁₋₆, OSG est O ou S, et XR¹ et XR² pris ensemble avec le chaînon azote auquel ils sont fixés forment l'un parmi un groupe pipéridine, un groupe pipérazine, un groupe morpholine, un groupe thiomorpholine, et un groupe pyrrolidine, ou chacun de XR¹ et XR² pris indépendamment sont l'un parmi H, alkyle en C₁₋₆, aryle, arylalkyle, cycloalkyle en C₃₋₈, C₃₋₈cycloalkyl-C₁₋₆alkyle, hétéroalkyle, hétéroaryl-C₁₋₆alkyle, hétérocycloalkyle et hétérocycloalkyl-C₁₋₆alkyle ; où l'aryle, l'arylalkyle, le cycloalkyle, l'hétéroaryle ou l'hétérocycloalkyle peut éventuellement être substitué avec un ou plusieurs substituants indépendamment choisis parmi l'halogène, l'hydroxy, l'alkyle en C₁₋₆, l'alcoxy en C₁₋₆, l'alkyle en C₁₋₆ hydrogéné, l'alcoxy en C₁₋₆ halogéné, le nitro, le cyano, l'amino, l'alkylamino en C₁₋₄, le di(C₁₋₉alkyl)amino, l'hétéroaryle ou l'hétérocycloalkyle ; et
(b) à condition, également, que lorsque X est S, et Y est 0, alors l'un parmi R^{2'} et R^{3'} n'est pas XCG lorsque l'autre est alkyle en C₁₋₆, où XCG est le groupe 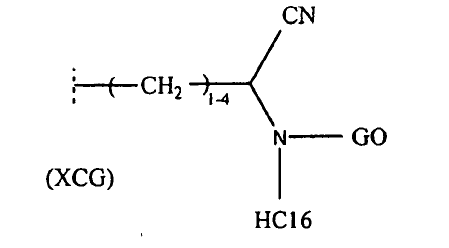 où HC16 est l'un parmi H, alkyle en Ci-6, haloC₁₋₆alkyle, allyle, et alcoxyméthyle en C₁₋₆, et GO est un groupe fixé à un chaînon carbone qui a un substituant =O formant un groupe amido avec le chaînon azote auquel ledit groupe GO est fixé.

2. Composé selon la revendication 1, dans lequel R⁴ est H.

3. Composé selon la revendication 1, dans lequel lesdits R² et R³ sont chacun indépendamment choisis parmi
(i) le groupe constitué par A), B), C), D), E), et I) ; ou
(ii) le groupe A) ; ou
(iii) le groupe B) ; ou
(iv) le groupe C) ; ou
(v) le groupe D) ; ou
(vi) le groupe E) ; ou
(vii) le groupe I),
tels que définis dans la revendication 1.

4. Composé selon la revendication 1, dans lequel lesdits R² et R³ sont pris ensemble avec l'azote auquel ils sont fixés pour former un hétérocycle qui contient au moins un chaînon hétéroatome qui est ledit azote de fixation, ledit hétérocycle étant choisi parmi l'un quelconque de
(a) le groupe constitué par i) et ii) ;
(b) le groupe i), et
(c) le groupe ii)
tels que définis dans la revendication 1.

5. Composé selon la revendication 1, dans lequel ledit composé est l'un parmi :
2-(4-pipéridin-1-ylméthyl-phénoxy)-benzooxazole ; et
2-(2-fluoro-4-pipéridin-1-ylméthyl-phénoxy)-benzooxazole.

6. Composé selon la revendication 1, dans lequel ledit composé est l'un parmi :
l'acide 1-[4-(benzothiazol-2-yloxy)-benzyl]-pipéridine-4-carboxylique ;
le 1-{1-[4-benzothiazol-2-yloxy)-benzyl]-pipéridine-4-yl}-pyrrolidin-2-one ;
le 2-(2-fluoro-4-pipéridine-1-ylméthyl-phénoxy)-benzothiazole ;
le 1-(2-{[4-benzothiazol-2-yloxy)-benzyl]-cyclopropyl-amino}-éthyl)-4-hydroxy-pyrrolidin-2-one ;
le N-{1-[4-benzothiazol-2-yloxy)-benzyl]-pipéridin-4-yl}-N-méthyl-méthanesulfonamide ;
le 2-{4-[4-(1H-tétrazol-5-yl)-pipéridin-1-ylméthyl]-phénoxy}-benzothiazole ;
le 1-{4-[4-(benzothiazol-2-yloxy)-benzyl]-pipérazin-1-yl}-2-hydroxy-éthanone ;
le N-{1-[4-(benzothiazol-2-yloxy)-benzyl]-pipéridin-4-ylméthyl}-méthanesulfonamide ;
le 3-{1-[4-(benzothiazol-2-yloxy)-benzyl}-pipéridin-4-yl}-oxazolidin-2-one ;
le 4-{1[4-(benzothiazol-2-yloxy)-benzyl]-pipéridin-4-yl}-morpholin-3-one ;
le 2-(4-pipéridin-1-ylméthyl-phénoxy)-benzothiazole ;
le 1-[4-(benzothiazol-2-yloxy)-benzyl]-4-phényl-pipéridin-4-ol ;
le 1-[4-(benzothiazol-2-yloxy)-benzyl]- pipéridin-4-ol ;
le {1-[4-(benzothiazol-2-yloxy)-benzyl]-pipéridin-4-yl}-méthanol ;
le N-{1-[4-(benzothiazol-2-yloxy)-benzyl]-pipéridin-4-yl}- méthanesulfonamide ;
le N-{1-[4-(benzothiazol-2-yloxy)-benzyl]-pipéridin-4-ylméthyl}-2-hydroxy-acétamide ;
l'ester méthylique d'acide {1-[4-(benzothiazol-2-yloxy)-benzyl]- pipéridin-4-yl}-carbamique ;
la {1-[4-(benzothiazol-2-yloxy)-benzyl]-pipéridin-4-ylméthyl}urée ;
le N-{1-[4-(benzothiazol-2-yloxy)-benzyl]-pipéridin-4-ylméthyl}-2,2,2-trifluoro-acétamide ;
l'acide {4-[4-(benzothiazol-2-yloxy)-benzyl]-pipérazin-1-yl}-acétique ;
le 2-[4-(4-méthanesulfonyl-pipérazin-1-ylméthyl)-phénoxy]-benzothiazole ;
le 1-{4-[4-(benzothiazol-2-yloxy)-benzyl]-pipérazin-1-yl}-2,2,2-trifluoro-éthanone ;
le 2-(4-morpholin-4-ylméthyl-phénoxy)-benzothiazole ;
l'ester phénylique d'acide {1-[4-(benzothiazol-2-yloxy)-benzyl}-pipéridin-4-yl}-carbamique ;
le N-{1-[4-(benzothiazol-2-yloxy)-benzyl]-pipéridin-4-yl}-benzènesulfonamide ;
l'ester éthylique d'acide 3-[4-(benzothiazol-2-yloxy)-benzylamino]-propionique ;
l'acide 3-[4-(benzothiazol-2-yloxy)-benzylamino]-propionique ;
le 1-{3-[4-(benzothiazol-2-yloxy)-benzylamino]-propyl}-pyrrolidin-2-one ;
le 1-(3-{[4-(benzothiazol-2-yloxy)-benzyl]-méthyl-amino}-propyl)-pyrrolidin-2-one ;
le 1-(3-{[4-(benzothiazol-2-yloxy)-benzyl]-isopropyl-amino}-propyl)-pyrrolidin-2-one ;
le 1-(3-{[4-(benzothiazol-2-yloxy)-benzyl]-éthyl-amino}-propyl)-pyrrolidin-2-one ;
la [4-(benzothiazol-2-yloxy)-benzyl]-cyclopropylamine ;
la N-1-[4-(benzothiazol-2-yloxy)-benzyl]-Nl-cyclopropyl-propane-1,3-diamine ;
le N-(3-{[4-(benzothiazol-2-yloxy)-benzyl]-cyclopropyl-amino}-propyl)-isobutyramide ;
la 1-(3-{[4-(benzothiazol-2-yloxy)-benzyl]-cyclopropyl-amino}-propyl)-3-isopropyl-urée ;
la 1-{1-[4-(benzothiazol-2-yloxy)-benzyl]-pipéridin-4-yl}-3-isopropyl-urée ;
l'ester méthylique d'acide N-{1-[4-(benzothiazol-2-yloxy)-benzyl]-pipéridin-4-yl}-oxalamique ;
le N-{1-[4-(benzothiazol-2-yloxy)-benzyl]-pipéridin-4-yl}-isobutyramide ;
le {1-[4-(benzothiazol-2-yloxy)-benzyl]-pipéridin-4-yl}amide d'acide tétrahydro-furan-2-carboxylique ;
le 1-{1-[4-benzothiazol-2-yloxy)-benzyl]-pipéridin-4-yl}-4-hydroxy-pyrrolidin-2-one ;
le 1-{1-[4-benzothiazol-2-yloxy)-benzyl]-pipéridin-4-yl}-4-hydroxy-pyrrolidin-2-one ;
la {1-[4-(benzothiazol-2-yloxy)-benzyl]-pipéridin-4-yl}-urée ;
l'acide N-{1-[4-(benzothiazol-2-yloxy)-benzyl]-pipéridin-4-yl}-oxalamique ;
le N-{1-[4-(benzothiazol-2-yloxy)-benzyl]-pipéridin-4-yl}-2-hydroxy-acétamide ;
le N-{1-[4-(benzothiazol-2-yloxy)-benzyl]-pipéridin-4-yl}-2,2,2-trifluoro-acétamide ;
le 2-[4-(1,1-dioxo-116-thiomorpholin-4-ylméthyl)-phénoxy]-benzothiazole ;
l'ester tert-butylique d'acide N-{1-[4-(benzothiazol-2-yloxy)-benzyl]-pipéridin-4-amino-sulfonyl}-carbamique ;
le N-{1-[4-(benzothiazol-2-yloxy)-benzyl]-pipéridin-4-yl}-acétamide ;
le N-{1-[4-(benzothiazol-2-yloxy)-benzyl]-pipéridin-4-yl}-N,N-diméthylsulfamide ;
la 1-{1-[4-benzothiazol-2-yloxy)-benzyl]-pipéridin-4-yl}-3-éthyl-urée ;
la 1-{1-[4-benzothiazol-2-yloxy)-benzyl]-pipéridin-4-yl}-3-éthyl-thiourée ;
le {1-[4-(benzothiazol-2-yloxy)-benzyl]-pipéridin-4-yl}-amide d'acide propane-1-sulfonique ;
le {1-[4-(benzothiazol-2-yloxy)-benzyl]-pipéridin-4-yl}-amide d'acide propane-2-sulfonique ;
le N-{1-[4-(benzothiazol-2-yloxy)-benzyl]-pipéridin-4-yl}-sulfamide ;
N-{1-[4-(benzothiazol-2-yloxy)-benzyl]-pipéridin-4-yl}-formamide ;
l'ester éthylique d'acide {1-[4-(benzothiazol-2-yloxy)-benzyl]- pipéridin-4-yl}-carbamique ;
le N-{1-[4-(benzothiazol-2-yloxy)-benzyl]-pipéridin-4-yl}-propionamide ;
N-{1-[4-(benzothiazol-2-yloxy)-benzyl]-pipéridin-4-yl}-butyramide ;
la 1-{1-[4-benzothiazol-2-yloxy)-benzyl]-pipéridin-4-yl}-3-propyl-urée ;
l'ester propylique d'acide {1-[4-(benzothiazol-2-yloxy)-benzyl]-pipéridin-4-yl}-carbamique ;
la 1-{1-[4-benzothiazol-2-yloxy)-benzyl]-pipéridin-4-yl}-3-méthyl-urée ;
la 1-{1-[4-benzothiazol-2-yloxy)-benzyl]-pipéridin-4-yl}-1,3-diméthyl-urée ;
la 1-{1-[4-benzothiazol-2-yloxy)-benzyl]-pipéridin-4-yl}-1-méthyl-urée ;
le N-{1-[4-(benzothiazol-2-yloxy)-benzyl]-pipéridin-4-yl}-N-méthyl-acétamide ;
l'ester méthylique d'acide {1-[4-(benzothiazol-2-yloxy)-benzyl]-pipéridin-4-yl}-méthyl-carbamique ;
l'ester méthylique d'acide N-{1-[4-(benzothiazol-2-yloxy)-benzyl]-pipéridin-4-yl}-N-méthyl-oxalamique ;
l'acide N-{1-[4-(benzothiazol-2-yloxy)-benzyl]-pipéridin-4-yl}-N-méthyl-oxalamique ;
la guanidine, N-{1-[4-(benzothiazol-2-yloxy)-benzyl]-pipéridin-4-yl}-N'-hydroxy ;
l'ester isopropylique d'acide {1-[4-(benzothiazol-2-yloxy)-benzyl]-pipéridin-4-yl}-carbamique ;
la 3-{1-[4-(benzothiazol-2-yloxy)-benzyl]-pipéridin-4-yl}-1,1-diméthyl-urée ;
l'ester méthylique d'acide {1-[4-(benzothiazol-2-yloxy)-benzyl]-pipéridin-4-ylcarbamoyl} ;
la {1-[4-(benzothiazol-2-yloxy)-benzyl]-pipéridin-4-yl}-thiourée ;
le 1'-[4-(benzothiazol-2-yloxy)-benzyl]-[1,4']bipipéridinyle ;
la {4-[4-(benzothiazol-2-yloxy)-benzyl]-pipérazin-1-yl}-(tétrahydro-furan-2-yl)-méthanone ;
l'ester tert-butylique d'acide {1-[4-(benzothiazol-2-yloxy)-benzyl]-pipéridin-4-yl}-carbamique ;
l'ester tert-butylique d'acide 4-[4-(benzothiazol-2-yloxy)-benzyl]-pipérazine-1-carboxylique ;
le 1-[4-(benzothiazol-2-yloxy)-benzyl]-pipéridin-4-ylamine ;
le 2-(4-pipérazin-1-ylméthyl-phénoxy)-benzothiazole ;
l'amide d'acide 4-[4-(benzothiazol-2-yloxy)-benzyl]-pipérazine-1-carboxylique ;
le 2-[4-(4-benzosulfonyl-pipérazin-1-ylméthyl)-phénoxy]-benzothiazole ;
le C-{1-[4-(benzothiazol-2-yloxy)-benzyl]-pipéridin-4-yl}-méthylamine ;
la 2-(2-{4-[4-(benzothiazol-2-yloxy)-benzyl]-pipérazine-1-yl}-2-oxo-éthyl)-cyclopentanone ;
l'ester éthylique d'acide {4-[4-(benzothiazol-2-yloxy)-benzyl]-pipérazin-1-yl}-acétique ;
l'ester 4-(benzothiazol-2-yloxy)-benzylique d'acide 4-{1-[4-(benzothiazol-2-yloxy)-benzyl]-pipéridin-4-yl}-butyrique ;
le 1-(3-{[4-(benzothiazol-2-yloxy)-benzyl]-cyclopropyl-amino}-propyl)-pyrrolidin-2-one ;
l'ester tert-butylique d'acide (3-{[4-(benzothiazol-2-yloxy)-benzyl]-cyclopropyl-amino}-propyl)-carbamique ;
le (3-{[4-(benzothiazol-2-yloxy)-benzyl]-cyclopropyl-amino}-propyl)-amide d'acide tétrahydro-furan-2-carboxylique ;
le 1-(3-{[4-(benzothiazol-2-yloxy)-benzyl]-cyclopropyl-amino}-propyl)-4-hydroxy-pyrrolidin-2-one ;
l'ester tert-butylique d'acide (2-{[4-(benzothiazol-2-yloxy)-benzyl]-cyclopropyl-amino}-éthyl)-carbamique ;
le N1-[4-(benzothiazol-2-yloxy)-benzyl]-N1-cyclopropyl-éthane-1,2-diamine ;
le {1-[4-benzothiazol-2-yloxy)-benzyl]-pipéridin-4-yl}-amide d'acide éthanesulfonique ;
le 1-{1-[4-benzothiazol-2-yloxy)-benzyl]-pipéridin-4-yl}-pyrrole-2,5-dione ;
l'ester tert-butylique d'acide {1-[4-benzothiazol-2-yloxy)-benzyl]-pipéridin-4-yl}-méthyl-carbamique ;
le {1-[4-benzothiazol-2-yloxy)-benzyl]-pipéridin-4-yl}-méthyl-amine ;
l'acide {1-[4-(benzothiazol-2-yloxy)-benzyl]-pipérazine-2-carboxylique ;
le [4-(benzothiazol-2-yloxy)-benzyl]-méthyl-amine ;
le benzothiazol-2-yl-{1-[4-(benzothiazol-2-yloxy)-benzyl]-pipéridin-4-yl}-amine ;
l'ester tert-butylique d'acide (3-{[4-benzothiazol-2-yloxy)-benzyl]-méthyl-amino}-propyl)-carbamique ;
le 4-({[4-(benzothiazol-2-yloxy)-benzyl]-méthyl-amino}-méthyl)-phénol ;
le N1-[4-(benzothiazol-2-yloxy)-benzyl]-N1-méthyl-propane-1,3-diamine ;
l'ester (3-{[4-benzothiazol-2-yloxy)-benzyl]-méthyl-amino}-propylcarbamoyl)-méthylique d'acide acétique ;
le N-(3-{[4-(benzothiazol-2-yloxy)-benzyl]-méthyl-amino}-propyl)-2-hydroxy-acétamide ;
le N-(3-{[4-(benzothiazol-2-yloxy)-benzyl]-méthyl-amino}-propyl)-méthanesulfonamide ;
le 6-chloro-2-(4-pipéridin-1-ylméthyl-phénoxy)-benzothiazole ;
le 6-méthoxy-2-(4-pipéridin-1-ylméthyl-phénoxy)-benzothiazole ;
le diméthylamide d'acide 4-[4-(benzothiazol-2-yloxy)-benzyl]-pipérazine-1-sulfonique ;
le 2-{4-[4-(benzothiazol-2-yloxy)-benzyl]-pipérazine-1-yl)-1-pyrrolidin-1-yl-éthanone ;
le 2-{4-[4-(benzothiazol-2-yloxy)-benzyl]-pipérazine-1-yl}-1-morpholin-4-yl-éthanone ;
le {4-[4-(benzothiazol-2-yloxy)-benzyl]-pipérazin-1-yl)-thiophèn-2-yl-méthanone ;
le 4-méthyl-2-(4-pipéridin-1-ylméthyl-phénoxy)-benzothiazole ;
le 4-chloro-2-(4-pipéridin-1-ylméthyl-phénoxy)-benzothiazole ;
le 2-[4-(4,4-difluoro-pipéridin-1-ylméthyl)-phénoxy]-benzothiazole ;
le {1-[4-(benzothiazol-2-yloxy)-benzyl]-pipéridin-4-ylméthyl}-amide d'acide 2-amino-cyclobutanecarboxylique ;
le [4-(benzothiazol-2-yloxy)-benzyl]-méthyl-(1-méthyl-pipéridin-4-yl)-amine ;
le 3-{[4-(benzothiazol-2-yloxy)-benzyl]-méthyl-amino}-propionitrile ;
le 4-[4-(benzothiazol-2-yloxy)-benzyl]-pipérazin-2-one ;
le 2-[4-(3-méthyl-pipéridin-1-ylméthyl)-phénoxy]-benzothiazole ;
l'ester ({1-[4-(benzothiazol-2-yloxy)-benzyl]-pipéridin-4-yl}-méthyl-carbamoyl)-méthylique d'acide acétique ;
le N-{1-[4-(benzothiazol-2-yloxy)-benzyl]-pipéridin-4-yl}-2-hydroxy-N-méthyl-acétamide ; et
l'ester éthylique d'acide 1-[4-(benzothiazol-2-yloxy)-benzyl]-pipéridine-4-carboxylique.

7. Composé selon la revendication 1, dans lequel ledit composé est l'un parmi :
2-(4-pipéridin-1-ylméthyl-phénoxy)-1H-benzoimidazole ; et
l'acide 1-[4-(1H-benzoimidazol-2-yloxy)-benzyl]-pipéridine-4-carboxylique.

8. Composition pharmaceutique comprenant au moins un composé selon l'une quelconque des revendications 1 à 7.

9. Utilisation d'une quantité efficace sur le plan thérapeutique d'au moins un modulateur de la LTA4H choisi parmi les composés selon la revendication 1, dans la fabrication d'un médicament pour le traitement, la prévention ou l'inhibition de l'inflammation chez un sujet.

10. Utilisation selon la revendication 9, dans laquelle ladite inflammation est due à au moins une pathologie parmi l'asthme, une maladie pulmonaire obstructive chronique, l'athérosclérose, la polyarthrite rhumatoïde, la sclérose en plaques, une maladie gastro-intestinale inflammatoire et le psoriasis.

11. Utilisation d'une quantité inhibitrice d'au moins un modulateur de la LTA4H choisi parmi les composés selon la revendication 1, dans la fabrication d'un médicament pour l'inhibition de l'activité de l'enzyme LTA4H.

12. Utilisation selon l'une quelconque des revendications 9 à 11, dans laquelle ledit R⁴ est H.

13. Utilisation selon l'une quelconque des revendications 9 à 11, dans laquelle lesdits R² et R³ sont chacun indépendamment choisis parmi
(i) le groupe constitué par A), B), C), D), E), et I) ; ou
(ii) le groupe A) ; ou
(iii) le groupe B) ; ou
(iv) le groupe C) ; ou
(v) le groupe D) ; ou
(vi) le groupe E) ; ou
(vii) le groupe I),
tels que définis dans la revendication 1.

14. Utilisation selon l'une quelconque des revendications 9 à 11, dans laquelle lesdits R² et R³ sont pris ensemble avec l'azote auquel ils sont fixés pour former un hétérocycle qui contient au moins un chaînon hétéroatome qui est ledit azote de fixation, ledit hétérocycle étant choisi parmi l'un quelconque des groupes suivants :
(d) le groupe constitué par i) et ii) ;
(e) le groupe i), et
(f) le groupe ii)
tels que définis dans la revendication 1.

15. Utilisation selon l'une quelconque des revendications 9 à 11, dans laquelle ledit au moins un modulateur de la LTA4H est choisi parmi les composés selon l'une quelconque des revendications 5 à 7.
